# EUROPEAN PATENT APPLICATION

(11) **EP 1 676 572 A1**
(43) Date of publication of application: **05.07.2006**
(21) Application number: 04788212.1
(22) Date of filing: 27.09.2004
(51) Int. Cl.: A61K 31/353

(54) **REMEDY FOR AUTOIMMUNE DISEASES**

(30) Priority: 26.09.2003 JP 2003336014; 27.07.2004 JP 2004219071
(71) Applicant: KIRIN BEER KABUSHIKI KAISHA, Tokyo 104-8288 (JP)
(72) Inventor: KOBAYASHI, M., c/o Kirin Beer K.K., Yokohama-shi, Kanagawa, 236-0004 (JP); ODAI, H., c/o Kirin Beer K.K., Yokohama-shi, Kanagawa 230-0052 (JP); FUJIWARA, D., c/o Kirin Beer K.K., Yokohama-shi, Kanagawa, 236-0004 (JP); SASAKI, K., c/o Kirin Beer K.K., Yokohama-shi, Kanagawa, 236-0004 (JP)
(74) Representative: HOFFMANN EITLE
(86) International application number: PCT/JP2004/014116
(87) International publication number: WO 2005/030200

(57) **Abstract**

In the present invention, there are disclosed a therapeutic agent for the treatment of a disease in which the suppression of Th1 cytokine production is therapeutically effective and/or a disease in which the suppression of autoantibody production is therapeutically effective, a therapeutic agent for the eradicative treatment of these diseases, and an agent for inhibiting the progress of these diseases, comprising (i) a proanthocyanidin with a polymerization degree of at least 4 or (ii) a plant material extract containing a proanthocyanidin as an active ingredient.

## Description

### BACKGROUND OF THE INVENTION

### Field of the Invention

The present invention relates to therapeutic agents for autoimmune diseases, and more specifically, to therapeutic agents for autoimmune diseases comprising proanthocyanidins as an active ingredient.

### Background Art

In recent years, autoimmune diseases represented by chronic rheumatoid arthritis have been increasing with the aging of society. The autoimmune diseases can be roughly classified into two groups by the difference in their immune response type: diseases caused by excessive acceleration of cellular immunity and diseases mediated by autoantibodies. Examples of the former include, but are not limited to, chronic rheumatoid arthritis (RA), multiple sclerosis (MS), autoimmune thyroiditis (AT), insulin dependent diabetes mellitus (IDDM) such as juvenile diabetes and type I diabetes, autoimmune uveal retinitis (AUR), and psoriasis. Examples of the latter include, but are not limited to, myasthenia gravis (MG), systemic lupus erythematodes (SLE), Good Pasture's syndrome, and autoimmune hemolytic anemia. A representative existing method for treating these autoimmune diseases is use of immunosuppressive agents that non-specifically suppress immune responses. Examples of the immunosuppressive agents include methotrexate, cyclophosphamide, cyclosporin A, tacrolimus, and various steroid compounds. These agents are not only highly toxic and cause serious side effects but also bring a risk of infections since immune reactions are suppressed during the period of treatment. Under these circumstances, biological formulations recently attract attentions particularly in the treatment of rheumatism. Aggravating factors in cellular immunity-dependent autoimmune diseases are inflammatory cytokines such as TNF-α, IL-1, and IL-6. Clinical tests have been performed with anti-TNF-α antibodies, soluble TNF-α receptors, anti-IL-6 receptor antibodies, IL-1 receptor antagonists, and the like. These biological formulations require continuous administration since they are symptomatic treatment therapy.

Of autoimmune diseases, cellular immunity accelerating autoimmune diseases are dominant in terms of the number of patients. In this type, Th1 cells and Tc1 cells play a central role in the onset mechanism. Th1 cells are a subgroup of helper T-cells and produce Th1 cytokines such as IFN-γ, TNF-α, and IL-2 selectively to antigen stimulation. For Th1 cell differentiation, a cytokine called IL-12 which is produced by an antigen presenting cell (e.g., dendritic cells, macrophages) is essential; in cellular immunity accelerating autoimmune diseases, production of IL-12 by antigen presenting cells increases, which promotes a Th1 immunity more easily. Th1 cells generate the Th1 cytokine environment and thus induce differentiation of Tc1 cells, namely T cells having a cytotoxic activity. Therefore, it is believed that the suppression of Th1 cytokine production is effective for the treatment and prevention of autoimmune diseases.

A number of traditional Chinese medicines and other alternative medicines are largely tried in this field and a certain kind of plant materials is empirically known to be effective for autoimmune diseases.

For example, tripterygium wilfordii has been reported to be effective for rheumatism (BMC Pharmacol. 2004, 4(1): 2), Ninjin-to (Ren-Shen-Tang) has been reported to be effective for type I diabetes (Microbiol. Immunol. 2000, 44 (4) : 299-305), and Kanran (Brassica oleracea) has been reported to be effective for multiple sclerosis (Arzneimittelforschung 1998, 48 (6) : 668-74) . Further, a fern species, Polypodium leucotomos, has been traditionally used for the treatment of psoriasis (Padilla HC., 1974, Int. J. Dermatol. 13, 276-282; Gonzales S., et al., 2000, Anticancer Res. 20, 1567-1576). Further, an extract of another fern species, samambaia (Polypodium lepidopteris), has been known to increase the number of CD8⁺ T cells without affecting the number of CD4⁺ T cells or B cells (Hostettmann K., et al. , 1995, Phytochemistry of Plants Used in Traditional Medicine, Proceedings of the Phytochemical Society of Europe. Oxford University Press: Oxford, NY). Although the effects obtained with these materials have not been scientifically elucidated, the materials are advantageously inexpensive and can be easily tried. Further, bark, sap, resin, and leaves of Jatoba (Hymenaea courbaril, also known as azucar huayo) have been used as a natural medicine for diarrhea, bladder inflammation, hepatitis, prostatitis, and coughing (Rutter, R.A., 1990, Catalogo de Plantas Utiles de la Amazonia Peruana. Instituto Linguistico de Verano. Yarinacocha, Peru. 349). Today, tea and liquid extract made from Jatoba bark are widely accepted as agents for appetite stimulation and recovery from fatigue, as nourishing tonic agents, and as nutrient supplements (Silva, 1930, Catalogo de Extractos Fluidos, Araija e Cia. Ltd., Rio de Janeiro, Brazil ; Cruz GL. , 1995, Dicionario das Plantas Uteis Do Brasil, 5th Ed. Rio de Janeiro: Bertrand Brazil) . Further, there has been reported that a flavonoid called astilibin contained in Jatoba bark exhibits antioxidant and liver protecting properties and there is a cause and effect relation between its antibacterial effect and terpene and phenol (Closa D. et al., 1997. Prostaglandins Leukot Essent Fatty Acids 56, 331-334 ; Pinheiro de SM. , et al., 1974. Molluscicidal activity of plants from Northeast Brazil., Rev Bras Fpesq Med Biol, 74: 389-394; Rouquayrol MZ., et al., 1980. Rev Brasil Fpesq Med Biol, 74 : 389-394; Rouquayrol MZ. , et al., 1980. Rev Brasil Pesq Med Biol 13, 135-143) . However, there has been no report on effects of these components on Th1 cytokine production or autoimmune diseases.

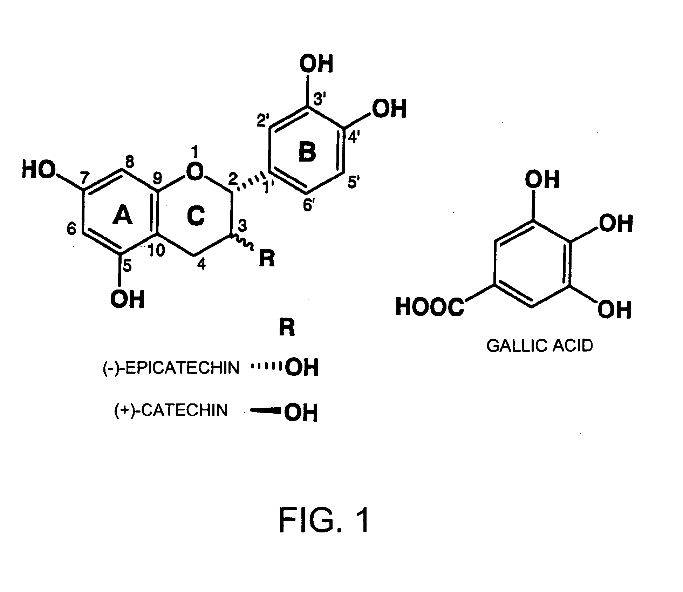

Here, proanthocyanidin is a condensed tannin widely found in various plants and gives anthocyanidin by acid treatment. As shown in Fig. 1, it is a polymerized polyphenol component mainly consisting of catechin, i.e., a flavan-3-ol as a basic unit. Namely, proanthocyanidin is a generic name for an extremely various compound group, in which monomers having hydroxyl groups in various sites are polymerized with a flavan backbone structure as a base. Of the constitutive units of the polymer, a terminal one is called a terminal unit, and others are called extension units and the polymer can be from a dimer, a trimer to a polymer of more than 100 units.

To date, use of proanthocyanidins as an anti-allergy agent based on the suppression of release of histamines and leukotrienes from basophiles has been reported (Japanese Patent Laid-Open Publication No.278792/2001).Further, there have been various reports on their anti-obesity activity (Japanese Patent Laid-Open Publication No. 291039/1997), matrix protease inhibition (Japanese Patent Laid-Open Publication No. 504402/2003), muscular atrophy suppressive activity (Japanese Patent Laid-Open Publication No. 338464/2002), blood sugar lowering activity (Japanese Patent Laid-Open Publication No. 253918/1992), and the like. However, the activity of proanthocyanidins in regard to the suppression of Th1 cytokine production as well as the treatment and prevention of autoimmune diseases has not been known.

### SUMMARY OF THE INVENTION

The present inventors have found that extracts derived from specific plants suppress Th1 cytokine production in Th1-biased mouse splenocytes, that an active ingredient conferring such an effect is proanthocyanidins, a kind of polyphenol, and that proanthocyanidins having a specific degree polymerization are particularly effective for the suppression of Th1 cytokine production, and thus completed the present invention.

The present inventors also have confirmed that tetramer or larger proanthocyanidins, especially pentamer or larger proanthocyanidins, exhibit a high therapeutic activity in an experimental autoimmune encephalitis (EAE) model.

Further, the present inventors have found that highly polymerized proanthocyanidins suppress B cell activation and that highly polymerized proanthocyanidins suppress autoantibody production in mice in which autoantibodies are induced, and thus completed the present invention.

An object of the present invention is to provide therapeutic agents for the treatment of diseases in which the suppression of Th1 cytokine production is therapeutically effective or diseases in which the suppression of autoantibody production is therapeutically effective, including chronic rheumatoid arthritis, multiple sclerosis, and insulin-dependent diabetes mellitus.

According to the present invention, there are provided a therapeutic agent for the treatment of diseases in which the suppression of Th1 cytokine production is therapeutically effective or diseases in which the suppression of autoantibody production is therapeutically effective, a therapeutic agent for the eradicative treatment of these diseases, and an agent for inhibiting the progress of these diseases (hereinafter referred to as "therapeutic agent according to the present invention"), which comprises (i) proanthocyanidins with a polymerization degree of 4 or more or (ii) a plant material extract containing proanthocyanidins, as an active ingredient.

According to another aspect of the present invention, there is provided use of (i) proanthocyanidins with a polymerization degree of 4 or more or (ii) a plant material extract containing proanthocyanidins, for manufacturing a therapeutic agent according to the present invention.

According to further another aspect of the present invention, there are provided a method of treating diseases in which the suppression of Th1 cytokine production is therapeutically effective or diseases in which the suppression of autoantibody production is therapeutically effective, a method for the eradicative treatment of these diseases, and a method of inhibiting the progress of these diseases, which comprises administering to a mammal a therapeutically effective amount of (i) proanthocyanidins with a polymerization degree of 4 or more or (ii) a plant material extract containing proanthocyanidins together with a pharmaceutically acceptable carrier, if necessary.

### BRIEF DESCRIPTION OF THE DRAWINGS

Fig. 1 shows formulae of representative constitutive flavan compounds that compose proanthocyanidin, i.e., epicatechin and catechin, and gallic acid which often makes ester addition with a hydroxyl group at position 3 of the flavan ring.
Fig. 2 shows the construction of a human multiple sclerosis model in mice and the schedule for intraperitoneal administration of Jatoba.
Fig. 3 shows the evaluation of anti-autoimmune disease activity of Jatoba, as measured by the change in clinical scores in a human multiple sclerosis model in mice.
Fig. 4A shows the activity of Jatoba in suppressing the production of IFN-γ, a Th1 cytokine produced by splenocytes. Fig. 4B shows the activity of Jatoba in suppressing the production of TNF-α, an inflammatory cytokine. "+ MOG" means that MOG peptide was added to the culture supernatant and "-MOG" means that no MOG peptide was added to the culture supernatant. The comparison of the two can confirm the antigen-dependent reaction.
Fig. 5 shows the effect of Jatoba in suppressing demyelination. Mice in the control group (control, score 3) (onset) and mice in Jatoba administration group (50 mg/kg of Jatoba, score 0) (no onset) were dissected on day 15 to observe the presence or absence of demyelination in the spinal cord.
Fig. 6 shows the activity of Jatoba in suppressing the production of IFN-γ, a Th1 cytokine produced by splenocytes, in the EAE model SJL/J mice, which were administered with PLP peptide for induction and dissected on day 12. "+ PLP" means that PLP peptide was added to the culture supernatant and "-PLP" means that no PLP peptide was added to the culture supernatant. The comparison of the two can confirm the antigen-dependent reaction.

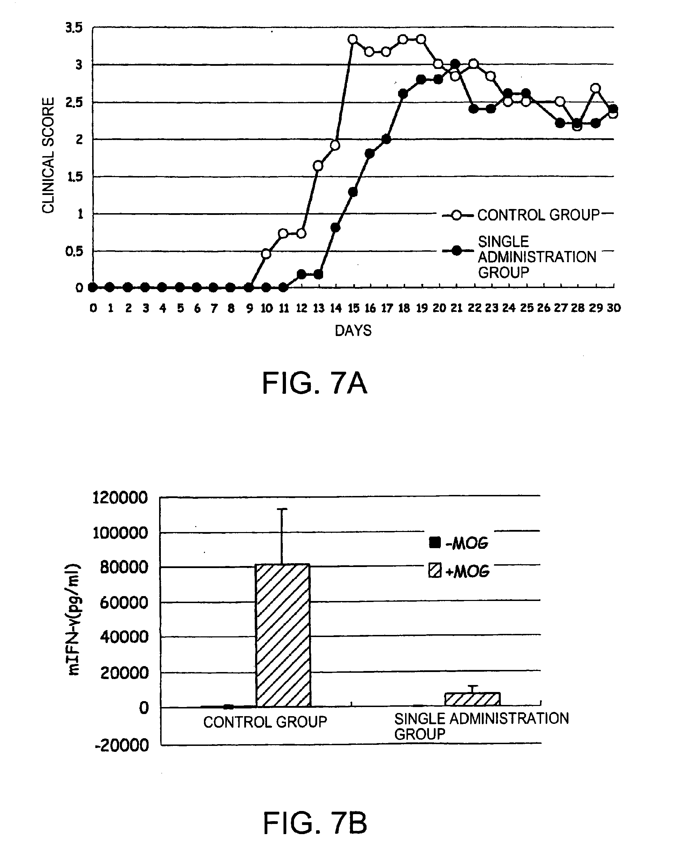

Fig. 7A shows the effect of a single prophylactic administration of Jatoba, as measured by the change in clinical scores; control: control group, and once: a single administration. Fig. 7B shows the activity of Jatoba in suppressing the production of IFN-γ, a Th1 cytokine produced by splenocytes, in mice which were administered with a single prophylactic dose of Jatoba and dissected on day 11. "+ MOG" means that MOG peptide was added to the culture supernatant and "- MOG" means that no MOG peptide was added to the culture supernatant. The comparison of the two can confirm the antigen-dependent reaction.
Fig. 8 shows the change in clinical scores when the PVPP-treated Jatoba extract was administered.
Fig. 9 shows the activity of the Jatoba extract and the PVPP-treated Jatoba extract in suppressing the production of IFN-γ, a Th1 cytokine produced by splenocytes, and TNF-α, an inflammatory cytokine. "+ MOG" means that MOG peptide was added to the culture supernatant and "-MOG" means that no MOG peptide was added to the culture supernatant. The comparison of the two can confirm the antigen-dependent reaction.
Fig. 10A shows that molecular ion peaks of procyanidin dimers, trimers, tetramers, pentamers, and hexamers were observed when the Jatoba ethanol extract was submitted to the electrospray ionization mass spectrometry in Example 7. Fig. 10B showed that molecular ion peaks of procyanidins with a polymerization degree of 20 or more were observed when the Jatoba ethanol extract was submitted to the matrix-assisted laser desorption/ionization time-of-flight massspectrometry in Example 7.
Fig. 11 is a schematic illustration showing the thiolytic cleavage in which proanthocyanidin is heated with toluene-α-thiol for reaction under acidic conditions.
Fig. 12 shows the chromatogram obtained by reverse phase column chromatography of the thiolytic reaction product of the Jatoba ethanol extract.
Fig. 13 shows that procyanidins in the Jatoba ethanol extract were fractionated by the polymerization degree by precipitation fractionation utilizing their difference in solubility in methanol and chloroform. *: Average polymerization degree of procyanidin/content (%).
Fig. 14 shows the activity of the Jatoba ethanol extract and individual fractions (Frs) fractionated in Example 9 in suppressing the production of IFN-γ cytokine, a Th1 cytokine produced by splenocytes. "+ MOG" means that MOG peptide was added to the culture supernatant and "- MOG" means that no MOG peptide was added to the culture supernatant. The comparison of the two can confirm the antigen-dependent reaction.
Fig. 15 is the proton (¹H) NMR spectrum (acetone-d₆, 20°C, 500 MHz) of epicatechin-benzylthiol ether (EC-BTE) to identify the structure.
Fig. 16 is the proton (¹H) NMR spectrum (acetone-d₆, 20°C, 500 MHz) of epicatechin-benzylthiol ether (EC-BTE) to verify that benzylthiol is added at position 4 of the flavan ring.
Fig. 17 shows the average polymerization degree of procyanidins and their content (%) obtained by analyzing HPLC data using EC-BTE as a standard and calculating individual components on the basis of the molar amount. *: Average polymerization degree of procyanidin/content (%).
Fig. 18 shows that procyanidins contained Applephenon (trade registered trade mark) was subjected to the reverse phase column chromatography for quantative analysis and then to the liquid chromatography-mass spectrometry to reveal the main molecular ion peakto be trimers.
Fig. 19 shows the proton (¹H) NMR (cd₃od, 20°C, 600 MHz) comparing the commercial standard epicatechin gallate (ECG) and the purified epicatechin gallate-benzylthiol ether (EC-BTE).
Fig. 20 shows the HMBC spectrum (cd₃od, 20°C, 600 MHz) of epicatechin gallate-benzylthiol ether (ECG-BTE) to verify that benzylthiol is added at position 4 of the flavan ring.
Fig. 21 shows the HSQC spectrum (cd₃od, 20°C, 600 MHz) of epicatechin gallate-benzylthiol ether (ECG-BTE) in which carbons at position 4 of the flavan ring and the SCH₂ site of benzylthiol are assigned.
Fig. 22 shows the proton (¹H) NMR (cd₃od, 20°C, 600 MHz) comparing the commercial standard catechin (CA) and the purified catechin-benzylthiol ether (CA-BTE).
Fig. 23 shows the HMBC spectrum (cd₃od, 20°C, 600 MHz) of catechin-benzylthiol ether (CA-BTE) to verify that benzylthiol is added at position 4 of the flavan ring.
Fig. 24 shows the HSQC spectrum (cd₃od, 20°C, 600 MHz) of catechin-benzylthiol ether (CA-BTE) in which carbons at position 4 of the flavan ring and the SCH₂ site of benzylthiol are assigned.
Fig. 25 is the LC-MS chromatogram showing that the procyanidins (Fr5 obtained in Example 9) were precisely isolated by the polymerization degree using the Discovery (registered trade mark) HS PEG column. The peaks in the solid-line frames were identified by monovalent ions, and the peaks in the broken-line frames were identified by divalent ions. Each number in a circle, 3, 4, 5, 6, 7, and 8 represents a polymerization degree of 3, 4, 5, 6, 7, and 8, respectively.
Fig. 26 is the LC-MS chromatogram showing that the procyanidins (Fr. 4 prepared as in Example 9) were precisely isolated by the polymerization degree using the Discovery (registered trade mark) HS PEG column. The peaks in the solid-line frames were identified by monovalent ions, and the peaks in the broken-line frames were identified by divalent ions. Each number in a circle, 3, 4, 5, 6, 7, 8, 9, 10, 11, and 12, represents a polymerization degree of 3, 4, 5, 6, 7, 8, 9, 10, 11, and 12, respectively.
Fig. 27 shows the analysis of the distribution of the polymerization degree of the procyanidins (Fr5 prepared as in Example 9) using the Discovery (registered trade mark) HS PEG column. Each number in a circle, 2, 3, 4, 5, 6, 7, 8, 9, and 10, represents a polymerization degree of 2, 3, 4, 5, 6, 7, 8, 9, and 10, respectively.
Fig. 28 shows the analysis of the distribution of the polymerization degree of the procyanidins (Fr. 4 prepared as in Example 9) using the Discovery (registered trade mark) HS PEG column. Each number in a circle, 2, 3, 4, 5, 6, 7, 8, 9, 10, 11, 12, and 13, represents a polymerization degree of 2, 3, 4, 5, 6, 7, 8, 9, 10, 11, 12, and 13, respectively.
Fig. 29 shows the analysis of the distribution of the polymerization degree of the procyanidins (Fr. 3 prepared as in Example 9) using the Discovery (registered trade mark) HS PEG column. Each number in a circle, 2, 3, 4, 5, 6, 7, 8, 9, ,10, 11, 12, and 13, represents a polymerization degree of 2, 3, 4, 5, 6, 7, 8, 9, 10, 11, 12, and 13, respectively.
Fig. 30 shows the effect of various procyanidin-containing samples on EAE clinical scores. The figures in parentheses are the average polymerization degree.
Fig. 31 shows the activity of various procyanidin containing samples in suppressing the production of IFN-γ, a Th1 cytokine produced by splenocytes. "+ MOG" means that MOG peptide was added to the culture supernatant and "- MOG" means that no MOG peptide was added to the culture supernatant. The comparison of the two can confirm the antigen-dependent reaction.
Fig. 32 shows the comparison of the activity of highly polymerized procyanidins of other materials in suppressing IFN-γ, a Th1 cytokine produced by splenocytes. "+ MOG" means that MOG peptide was added to the culture supernatant and "-MOG" means that no MOG peptide was added to the culture supernatant. The comparison of the two can confirm the antigen-dependent reaction.
Fig. 33 shows the construction of a chronic rheumatoid arthritis-induced model in mice and the schedule for intraperitoneal administration of Jatoba.
Fig. 34 shows the change in clinical scores in the Jatoba intraperitoneal administration group and the control group in the Type II collagen-induced arthritis model in mice.
Fig. 35 shows the construction of a type I diabetes-induced model in mice and the schedule for intraperitoneal administration of Jatoba.
Fig. 36 shows the change in the in vivo diabetes incidence rate in the Jatoba intraperitoneal administration group and the control group in the type I diabetes-induced arthritis model in mice.
Fig. 37 shows the activity of Jatoba in suppressing the production of IFN-γ, a Th1 cytokine produced by splenocytes, in mice dissected at 9 weeks of age.
Fig. 38 shows the experimental schedule for a spontaneous type I diabetes onset model in mice.
Fig. 39 shows antigen-specific reactivity of splenocytes, as measured by the insulin-dependent IFNγ productivity in the spontaneous type I diabetes onset model.
Fig. 40 shows the splenocyte population in the spontaneous type I diabetes onset model.
Fig. 41 shows the surface antigen expression level of macrophages in the spontaneous type I diabetes onset model.
Fig. 42 shows the suppression of the incidence rate by Jatoba administration in the spontaneous type I diabetes onset model.
Fig. 43 shows the change with-time in the proportion of individual immunocompetent cells in splenocytes in the EAE model.
Fig. 44 shows the amount of the expression of cell-surface antigens by macrophages in the splenocytes in the EAE model.
Fig. 45 shows the effect of oral administration of the Jatoba extract, Polyphenon, and the Jatoba extract Fr3, as measured by clinical scores in the rheumatoid model.
Fig. 46 shows effect of the oral administration of Jatoba in the rheumatoid model by the antigen-specific IFN-γ production by splenocytes. "+ collagen" means that type II collagen was added to the culture supernatant and "- collagen" means that no type II collagen was added to the culture supernatant. The comparison of the two can confirm the antigen-dependent reaction.
Fig. 47 shows the effect of Jatoba extract Fr3 in terms of dose dependency, as measured by clinical scores in the rheumatoid model.
Fig. 48 shows chronic rheumatoid arthritis scores by the starting time of the Jatoba administration.
Fig. 49 shows the suppression of serum level of antibody by the Jatoba extract Fr3 in chronic rheumatoid arthritis.
Fig. 50 shows the comparison of the activity of various oligomers in the production of IFN-γ, a Th1 cytokine produced by splenocytes.
Fig. 51 shows the comparison of the activity of various oligomers in the production of IFN-γ, a Th1 cytokine produced by splenocytes.
Fig. 52 shows the effect of single administration of the Jatoba ethanol extract and Polyphenon on the body weight and the amount of feed intake.

### DETAILED DESCRIPTION OF THE INVENTION

### Active ingredient

In the present invention, the active ingredient "proanthocyanidin" refers to a catechin, that is, a polyphenol which has a flavan backbone as a basic unit. Examples of such constitutive unit include flavan-3-ols such as catechin, epicatechin, gallocatechin, epigallocatechin, catechin gallate, epicatechin gallate, gallocatechin gallate, epigallocatechin gallate, epidistenin (having no hydroxyl group in the B ring), afzelechin, and epiafzelechin, and further flavan-4-ols leucoanthocyanin (namely flavan-3,4-diol), and anthocyanidin.

The hydroxyl group at position 3 may form an ester with gallic acid (gallate) or a hydroxyl group at any position other than position 3 may form glycoside or methyl ether (methoxy) (see Figure 1).

Examples of major linkage sites between the proanthocyanidin constitutive units include, but are not limited to, one site either between position 4 and position 6 or between position 4 and position 8 (B-type linkage) or two sites between position 4 and position 8 and between position 2 and position 7 oxygen (A-type linkage) ; the configuration of these linkages are also not particularly limited.

Proanthocyanidins in which certain specific monomers are polymerized have common names; representative proanthocyanidins include, but are not limited to, those defined and classified as depending on the number of hydroxyl groups in ring B (see Fig. 1), such as propelargonidin (4' -OH), procyanidin (3' -OH, 4' -OH), andprodelphinidin (3' -OH, 4' -OH, 5' -OH) . Other examples are polymers having a component lacking a hydroxyl group at position 5 of ring A as a constitutive unit, as proguibourtinidin, profisetinidin, and prorobinetinidin, and further, polymers which have common names depending on the presence or absence of hydroxyl groups on specified sites, such as proteracacidin, promelacacidin, proapigeninidin, and proluteolinidin.

In the present invention, "proanthocyanidins" are preferably procyanidin and prodelphinidin. Constitutive units of "procyanidin" can be the same or different and selected from catechin, epicatechin, catechin gallate, and epicatechin gallate. Constitutive units of "prodelphinidin" can be the same or different and selected from gallocatechin, epigallocatechin, gallocatechin gallate, and epigallocatechin gallate.

"Proanthocyanidins with a polymerization degree of at least 4" used in the present invention as an active ingredient can be preferably procyanidin and/or prodelphinidin with a polymerization degree of at least 5.

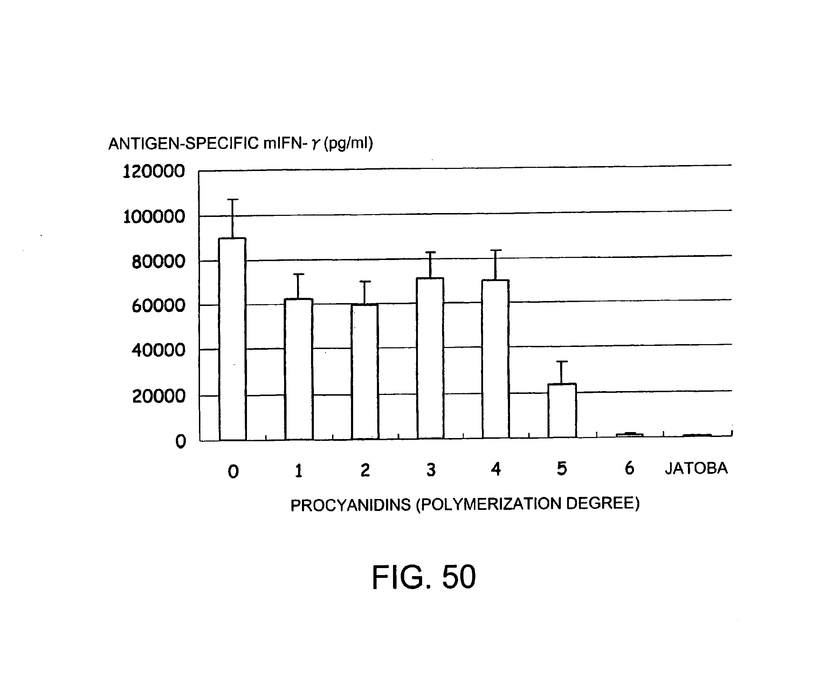

The proanthocyanidin used in the present invention showed a high therapeutic activity in an experimental autoimmune encephalitis (EAE) model when its polymerization degree was 5 or more (Example 31, Fig. 50). Therefore, the polymerization degree of the proanthocyanidin used in the present invention is preferably at least 5.

The upper limit of the polymerization degree of the proanthocyanidins used in the present invention is not particularly limited; however, proanthocyanidins derived from plants are confirmed to have a polymerization degree of approximately 20 to 30. When proanthocyanidins are organically synthesized, the upper limit of the polymerization degree can be set to be approximately 20 to 30 from viewpoints of economic efficiency and convenience in the synthetic process. Further, since proanthocyanidins with polymerization degrees of 5 or more are recognized to have a high therapeutic activity, effects according to the present invention can be expected even when the upper limit of the polymerization degree of proanthocyanidins is set to be about 10. Accordingly, in the present invention, the polymerization degree of the proanthocyanidins can be 4 to 30, 4 to 20, or 4 to 10, and preferably 5 to 30, 5 to 20, or 5 to 10. Further, in the present invention, the polymerization degree of the proanthocyanidins can be 5 to 6, 5 to 7, 5 to 8, and 5 to 9. In the present invention, the polymerization degree of the proanthocyanidins can be measured, for example, by the mass spectrometry method (Jan F. Stevens et al., J. Agric. Food Chem. 2002, 50, 3435-3443), more specifically in accordance with Examples 7, 16, 17, and 19.

In the present invention, proanthocyanidins can be represented by the following formula (I): 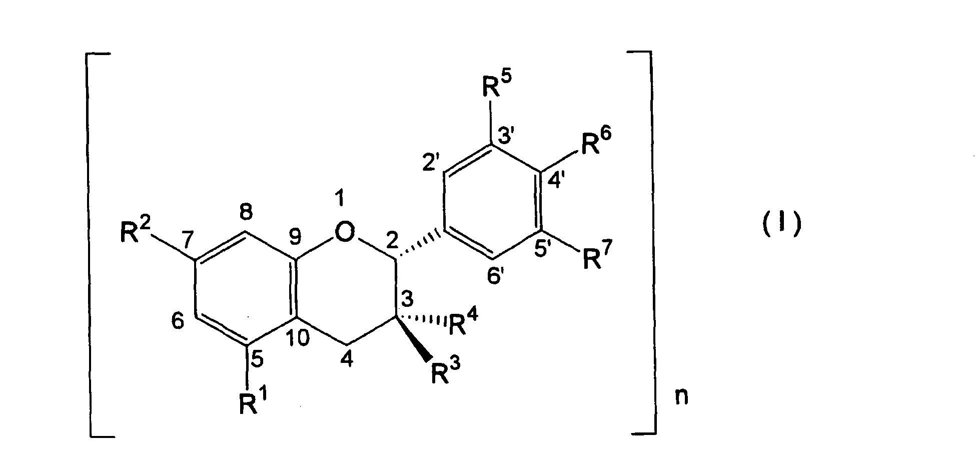 wherein R¹, R², R⁵, R⁶, and R⁷, whichmaybe the same or different, represent a hydrogen atom, a hydroxyl group, or -O-R¹¹ (R¹¹ is an alkyl group having 1 to 4 carbons or a sugar residue), and R³ and R⁴, which may be the same or different, represent a hydrogen atom, a hydroxyl group, or a group (II): 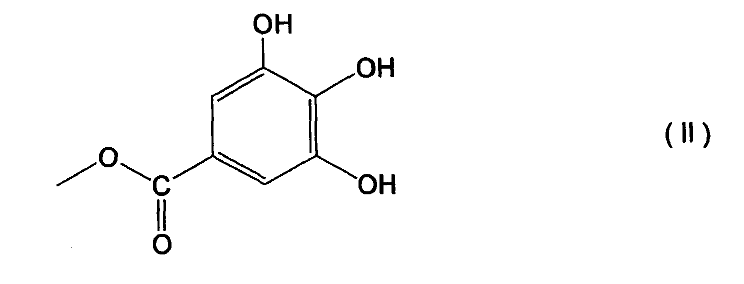 provided that R³ and R⁴ do not simultaneously represent a hydroxyl group; R³ and R⁴ do not simultaneously represent the group (II), n is an integer of 4 to 30; and individual constitutive units are linked each other on one site either between position 4 and position 6 or between position 4 and position 8 or on two sites between position 4 and position 8 and between position 2 and position 7.

In formula (I), "an alkyl group having 1 to 4 carbons" which may be represented by R¹¹ is preferably a methyl group.

In formula (I), "a sugar group" which maybe represented by R¹¹ means a sugar linked to the compound of formula (I) through an ether linkage between a hemiacetal or hemiketal hydroxyl group and a hydroxyl group of R³ or R⁴. Examples of the sugar include, but are not limited to, glucose, arose, altrose, mannose, grose, idose, galactose, talose, fructose, xylose, ribose, arabinose, lyxose, and rhamnose.

In formula (I), when constitutive units are linked each other through position 2, position 4, position 6, or position 8 of the constitutive unit, a single linkage is present at position 2, position 4, position 6, or position 8 in place of the hydrogen atom.

In formula (I), when the constitutive units are linked each other through position 7 of the constitutive unit, -O-is present at position 7 in place of R².

All substituents present in each constitutive unit in formula (I) maybe the same or different independently in every constitutive unit.

The hydroxyl groups in formula (II) may be the same or different and can be represented by R¹¹-O- (R¹¹ represents as defined above).

A compound having constitutive units of formula (I) wherein R¹ and R² represent a hydroxyl group or -O-R¹¹, R⁶ represents a hydroxyl group or -O-R¹¹, and R⁵ and R⁷ represent a hydrogen atom and a compound having constitutive units of formula (I) wherein R¹ and R² represent a hydroxyl group, R⁶ represents a hydroxyl group, and R⁵ and R⁷ represent a hydrogen atom corresponds to propelargonidin. In these cases, preferably, any one of R³ and R⁴ represents a hydroxyl group or group (II) and the other represents a hydrogen atom.

A compound having constitutive units of formula (I) wherein R¹ and R² represent a hydroxyl group or -O-R¹¹, R⁵ and R⁶ represent a hydroxyl group or -O-R¹¹, and R⁷ represents a hydrogen atom and a compound having constitutive units of formula (I) wherein R¹ and R² represent a hydroxyl group, R⁵ and R⁶ represent a hydroxyl group, and R⁷ represents a hydrogen atom correspond to procyanidin. In these cases, preferably, any one of R³ and R⁴ represents a hydroxyl group or group (II) and the other represents a hydrogen atom.

Of the constitutive units represented by formula (I),
- a constitutive unit of formula (I) wherein R¹, R², R⁵, and R⁶ represent a hydroxyl group or -O-R¹¹, R³ represents a hydroxyl group, and R⁴ and R⁷ represent a hydrogen atom corresponds to catechin;
- a constitutive unit of formula (I) wherein R¹, R², R⁵, and R⁶ represent a hydroxyl group or -O-R¹¹, R⁴ represents a hydroxyl group, and R³ and R⁷ represent a hydrogen atom corresponds to epicatechin;
- a constitutive unit of formula (I) wherein R¹, R², R⁵, and R⁶ represent a hydroxyl group or -O-R¹¹, R³ represents formula (II), and R⁴ and R⁷ represent a hydrogen atom corresponds to catechin gallate; and
- a constitutive unit of formula (I) wherein R¹, R², R⁵, and R⁶ represent a hydroxyl group or -O-R¹¹, R⁴ represents formula (II), and R³ and R⁷ represent a hydrogen atom corresponds to epicatechin gallate.

A compound having constructive units of formula (I) wherein R¹ and R² represent a hydroxyl group or -O-R¹¹ and R⁵, R⁶ and R⁷ represent a hydroxyl group or -O-R¹¹ and a compound wherein R¹ and R² represent a hydroxyl group and R⁵, R⁶ and R⁷ represent a hydroxyl group correspond to prodelphinidin. In these cases, preferably, any one of R³ and R⁴ represents a hydroxyl group or group (II) and the other represents a hydrogen atom.

Of the constitutive units represented by formula (I),
- a constitutive unit of formula (I) wherein R¹, R², R⁵, R⁶, and R⁷ represent a hydroxyl group or -O-R¹¹, R³ represents a hydroxyl group, and R⁴ represents a hydrogen atom corresponds to gallocatechin;
- a constitutive unit of formula (I) wherein R¹, R², R⁵, R⁶, and R⁷ represent a hydroxyl group or -O-R¹¹, R⁴ represents a hydroxyl group, and R³ represents a hydrogen atom corresponds to epigallocatechin;
- a constitutive unit of formula (I) wherein R¹, R², R⁵, R⁶, and R⁷ represent a hydroxyl group or -O-R¹¹, R³ represents formula (II), and R⁴ represents a hydrogen atom corresponds to gallocatechin gallate; and
- a constitutive unit of formula (I) wherein R¹, R², R⁵, R⁶, and R⁷ represent a hydroxyl group or -O-R¹¹, R⁴ represents formula (II), and R³ represents a hydrogen atom corresponds to epigallocatechin gallate.

In formula (I), n represents the polymerization degree (units) of proanthocyanidins. n represents preferably an integer of 5 to 30. n can also be an integer of 4 to 20, 4 to 10, 5 to 20, 5 to 10, 5 to 6, 5 to 7, 5 to 8, and 5 to 9.

Examples of the linkage mode of the individual constitutive units in formula (I) include A-type linkage and B-type linkage. The A-type linkage refers to a linkage mode in which the individual constitutive units are linked on two sites between position 4 and position 8 and between position 2 and position 7. The B-type linkage refers to a linkage mode in which the individual constitutive units are linked on one site either between position 4 and position 6 or between position 4 and position 8. The present invention includes not only the case where linkages between the constitutive units in formula (I) are altogether either A-type linkage or B-type linkage but also the case where A-type linkage and B-type linkage are intermixed. The linkage mode of the individual constitutive units may be the same or different in each constitutive unit.

Preferred examples of the linkage mode of the individual constitutive units in formula (I) are as follows: (catechin, gallocatechin, epicatechin, and epigallocatechin also include gallates thereof).
Epicatechin/epigallocatechin - (4β→8 linkage) - catechin/gallocatechin;
epicatechin/epigallocatechin - (4β→8 linkage) - epicatechin/epigallocatechin;
catechin/gallocatechin - (4α→8 linkage) - catechin/gallocatechin;
epicatechin/epigallocatechin - (4β→6 linkage) - epicatechin/epigallocatechin;
catechin/gallocatechin - (4α→6 linkage) - catechin/gallocatechin;
[epicatechin/epigallocatechin - (4β→8 linkage)]₂- epicatechin/epigallocatechin; and
epicatechin/epigallocatechin - (2β→7, 4β→8 linkages) - catechin/gallocatechin.

Of proanthocyanidins represented by formula (I), a preferred example is a compound of formula (I), wherein
the individual constitutive units may be the same or different,
- R¹, R², R⁵, and R⁶ represent a hydroxyl group or -O-R¹¹, R³ represents a hydroxyl group, and R⁴ and R⁷ represent a hydrogen atom (catechin),
- R¹, R², R⁵, and R⁶ represent a hydroxyl group or -O-R¹¹, R⁴ represents a hydroxyl group, and R³ and R⁷ represent a hydrogen atom (epicatechin),
- R¹, R², R⁵, and R⁶ represent a hydroxyl group or -O-R¹¹, R³ represents formula (II), and R⁴ and R⁷ represent a hydrogen atom (catechin gallate),
- R¹, R² , R⁵, and R⁶ represent a hydroxyl group or -O-R¹¹, R⁴ represents formula (II), and R³ and R⁷ represent a hydrogen atom (epicatechin gallate),
- R¹, R², R⁵, R⁶ and R⁷ represent a hydroxyl group or -O-R¹¹, R³ represents a hydroxyl group, and R⁴ represents a hydrogen atom (gallocatechin),
- R¹, R², R⁵, R⁶ and R⁷ represent a hydroxyl group or -O-R¹¹, R⁴ represents a hydroxyl group, and R³ represents a hydrogen atom (epigallocatechin),
- R¹, R², R⁵, R⁶ and R⁷ represent a hydroxyl group or -O-R¹¹, R³ represents formula (II), and R⁴ represents a hydrogen atom (gallocatechin gallate), or
- R¹, R², R⁵, R⁶ and R⁷ represent a hydroxyl group or -O-R¹¹, R⁴ represents formula (II), and R³ represents a hydrogen atom (epigallocatechin gallate);
   n is an integer of 5 to 30;
   the linkage modes of the individual constitutive units may be the same or different and are selected from
   epicatechin/epigallocatechin - (4β→8 linkage) - catechin/gallocatechin,
   epicatechin/epigallocatechin - (4β→8 linkage) - epicatechin/epigallocatechin,
   catechin/gallocatechin - (4α→8 linkage) - catechin/gallocatechin,
   epicatechin/epigallocatechin - (4β→6 linkage) - epicatechin/epigallocatechin;
   catechin/gallocatechin - (4α→6 linkage) - catechin/gallocatechin,
   [epicatechin/epigallocatechin - (4β→8)]₂ -epicatechin/epigallocatechin; and
   epicatechin/epigallocatechin - (2β→7, 4β→8 linkages) - catechin/gallocatechin,
   provided that catechin, gallocatechin, epicatechin, and epigallocatechin also include gallates thereof.

Particularly preferred examples of the linkage modes of the individual constitutive units in formula (I) are as follows.
Epicatechin - (4β→8 linkage) - catechin,
epicatechin - (4β→8 linkage) - epicatechin,
catechin - (4α→8 linkage) - catechin,
epicatechin - (4β→6 linkage) - epicatechin,
catechin - (4α→6 linkage) - catechin,
[epicatechin - (4β→8)]2 - epicatechin, and
epicatechin - (2β→7, 4β→8 linkages) - catechin.

Of proanthocyanidins represented by formula (I), a particularly preferred example is a compound of formula (I), wherein
the individual constitutive units may be the same or different, R¹, R², R³, R⁵, and R⁶ represent a hydroxyl group and R⁴ and R⁷ represent a hydrogen atom (catechin), or R¹, R², R⁴ , R⁵, and R⁶ represent a hydroxyl group and R³ and R⁷ represent a hydrogen atom (epicatechin);
n is an integer of 5 to 30;
the linkage modes of the individual constitutive units may be the same or different and are selected from
epicatechin - (4β→8 linkage) - catechin,
epicatechin - (4β→8 linkage) - epicatechin,
catechin - (4α→8 linkage) - catechin,
epicatechin - (4β→6 linkage) - epicatechin,
catechin - (4α→6 linkage) - catechin,
[epicatechin - (4β→8)]₂ - epicatechin, and
epicatechin - (2β→7, 4β→8 linkages) - catechin.

Proanthocyanidins are compounds found in a variety of plants and can be prepared by extracting plant materials. Preferred examples of raw plant materials to prepare proanthocyanidins include Jatoba, grape seeds, cranberry, cinnamon, and pine bark. Jatoba is more preferable because of its rich proanthocyanidin content. Examples of plant materials containing proanthocyanidins include Jatoba bark dried powder (Edison SRL, Japan Office), cranberry extract ("Cranberry Powder", Kikkoman Corporation), grape seed extract ("Gravinol SL", Kikkoman Corporation), pine bark extract (Functional Material Laboratory Co., Ltd.), and cacao extract ("Cacao Polyphenol", Meiji Seika Kaisha, Ltd.) ; they can be used as an active ingredient according to the present invention, as they are or after appropriate fractionation and purification to obtain materials having a polymerization degree as high as the desired level, as mentioned later.

Accordingly, in the present invention, "the extract of a plant material containing proanthocyanidins" can be used as an active ingredient. Examples of the "extract of a plant material containing proanthocyanidins" include a Jatoba extract, a grape seed extract, a cranberry extract, a cinnamon extract, a pine bark extract, and a cacao extract; however, the materials are not restricted as long as they contain proanthocyanidins (particularly procyanidin and/or prodelphinidin with a polymerization degree of at least 5).

In the present invention, the "plant material extract containing proanthocyanidins" used as an active ingredient can be preferably a plant material extract containing procyanidin and/or prodelphinidin with a polymerization degree of at least 5.

Examples of compositions of proanthocyanidins contained in plant materials are shown in Table 1.

**Table 1**

| | R³ | R⁴ | R⁷ | Name | Linkage mode |
|---|---|---|---|---|---|
| Jatoba | H | OH | H | EC | B |
| Gravinol | OH | H | H | CA | B |
| | H | OH | H | EC | B |
| | H | OG | H | ECG | B |
| Pycnogenol | OH | H | H | CA | B |
| | H | OH | H | EC | B |
| Cinnamon | H | OH | H | EC | B |
| | OH | H | H | CA | B |
| Cranberry highly polymerized fraction | OH | H | H | CA | A, B |
| | H | OH | H | EC | A, B |
| Cacao polyphenol highly polymerized fraction | OH | H | H | CA | B |
| | H | OH | H | EC | B |
| Rhodiola sacha. extract | OH | H | H | CA | B |
| | H | OH | H | EC | B |
| Peanut seeds | OH | H | H | CA | B |
| | H | OH | H | EC | B |
| Applephenon | H | OH | H | EC | B |
| | OH | H | H | CA | B |
| | H | OG | H | ECG | B |
| Polyphenon | OH | H | H | CA | None |
| | H | OH | H | EC | None |
| | H | OG | H | ECG | None |
| | H | OG | OH | EGCG | None |

| | | | | | |
|---|---|---|---|---|---|
| * R¹, R², R⁵ and R⁶ each represent OH. | | | | | |

In the Table, R¹ to R⁷ correspond to those in formula (I); EC represents epicatechin, CA represents catechin, ECG represents epicatechin gallate, EGCG represents epigallocatechin gallate, and G represents gallate.

An active ingredient according to the present invention, i. e. , proanthocyanidins and extract thereof, can be prepared by fractionating and purifying the abovementioned plant materials, more specifically, by subjecting the plant materials, as they are or after crushing, to an extraction process. Examples of the extraction method include a method in which plant materials, crushed products thereof, or the like are submersed in a cold or warmed solvent; a method in which extraction is carried out with heating and stirring and then the resulting extract is obtained by filtration; and a percolation method. After removing solids by filtration or centrifugation if necessary, the resulting extract can be used as it is or after removing the solvent by distillation and partially concentrating or drying, depending on the mode of use. Further, after concentrating or drying, the extract can be washed and purified with an insoluble solvent or further dissolved and suspended in an appropriate solvent for use. Further in the present invention, for example, the solvent extract obtained as described above can be dried using general means such as drying under reduced pressure and freeze drying to obtain a dried extract for use. Accordingly, "extract" in the present invention includes not only extract itself but also concentrated and dried products of the extract.

Examples of solvents to be used for the abovementioned extraction are polar media including water, lower alcohols having one to four carbon atoms, such as methanol, ethanol, propanol and butanol, lower alkyl esters such as ethyl acetate ester, glycols such as ethylene glycol, butylene glycol, propylene glycol, and glycerin, ethyl ethers, acetone, and acetic acid; non-polar media including hydrocarbons such as benzene and hexane, and ethers such as ethyl ethers and petroleum ethers; and other known organic solvents. These solvents can be used alone or in combination of two or more kinds. More preferably, an extraction method using hot water, ethanol, or acetone can be used. In particular, to fractionate a fraction of proanthocyanidins with a high average polymerization degree, the extraction with 70% acetone or ethanol is efficient. Further, to obtain a fraction with a specific average degree of polymerization, a 70% acetone or ethanol extract is extracted with ethyl acetate/water, after which the water layer fraction is redissolved in methanol and fractionated using chloroform according to the method by Saucier et al. (J. Agric. Food Chem. 49, 5732-5735 (2001)), as mentioned below. Namely, proanthocyanidins characteristically become less soluble in a lower concentration of chloroform as their polymerization degree increases, which makes it possible to obtain the fraction with a desired average degree of polymerization by appropriately changing the chloroform concentration. Accordingly the "extract" in the present invention can be preferably a water extract, an ethanol extract, or an acetone extract.

In addition to the abovementioned solvent extraction/precipitation procedure, a conventional fractionation and purification method may be applied. More specifically, an adsorbent which can adsorb and elute proanthocyanidins, such as stylene-divinyl benzene synthetic adsorptive resins, anion exchange resins, chemically octadecyl-bonded silica gel (ODS), and gel filtration resins (e.g., Sephadex LH20), can be used. The abovementioned clear liquid extract or clear fruit juice is applied on a column filled with such an adsorbent, thereby proanthocyanidins are adsorbed, after which the column is washed and eluted with an appropriate eluent to perform fractionation and purification of proanthocyanidins. Further, for example, the membrane separation method using a molecular sieve membrane can be used.

The various method for extraction and fractionation can also be used for the purpose of obtaining proanthocyanidins only with a specific polymerization degree or for the purpose of removing components or solvents which are undesirable in terms of safety, properties, flavor, and the like for use as a medicine or food.

According to the present invention, there is provided a method of manufacturing a fraction having an activity to suppress Th1 cytokine production, comprising the steps of
(a) subjecting a plant material containing proanthocyanidins to an extraction process, and
(b) obtaining a cake by subjecting the extracted fraction obtained in step (a) to an ultrafiltration process.

The extraction process and the plant material in step (a) are as described above. In the extraction process in step (a), a polar solvent, in particular, ethanol, water, or aqueous acetone, is preferably used. As the plant material, a plant containing proanthocyanidins is preferably used.

In step (a), an extract fraction is obtained by removing the solvent by distillation from the liquid extract and then this extract fraction is subjected to the extraction process using a nonpolar organic solvent (e.g. , ethyl acetate) /water to obtain a water-soluble fraction. Alternatively, the extract fraction obtained in step (a) may be subjected as it is to the ultrafiltration process of step (b).

In the ultrafiltration process of step (b), the cutoff value can be set so that a fraction having an activity to suppress Th1 cytokine production remains on the ultrafiltration membrane (namely, does not pass through the ultrafiltration membrane) . Since the relation between the activity to suppress Th1 cytokine production and the polymerization degree of proanthocyanidins is as mentioned above and the relation between the molecular weight of the substance passing through the ultrafiltration membrane is known to those skilled in the art, any one skilled in the art can appropriately set the cutoff value of the ultrafiltration membrane depending on the kind of proanthocyanidins to be manufactured. For example, the cutoff value can be set at 1400 since the molecular weight of proanthocyanidins with a polymerization degree of 5 is about 1442 and proanthocyanidins with a polymerization degree of 5 or more have a high activity to suppress Th1 cytokine production.

After step (b), the polymerization degree of the proanthocyanidins contained in the cake may be measured, if necessary. The polymerization degree can be measured by the mass spectrometry (Jan F. Stevens et al., J. Agric. Food Chem. 2002, 50, 3435-3443). Further, the average polymerization degree can be measured by the thiolysis analysis (Sylvain Guyot, Nathalie Marnet, and Jean-Francois Drilleau, J. Agric. Food Chem. 2001, 49, 14-20) . By measuring the polymerization degree of proanthocyanidins, the manufacture of the fraction having an activity to suppress Th1 cytokine production can be confirmed.

An active ingredient, i.e., proanthocyanidins, according to the present invention has a polymerization degree of 4 or more, preferably 5 or more. Accordingly, in preparing proanthocyanidins from plants, when the extract originally contains proanthocyanidin with a polymerization degree of 4 or more or 5 or more, it is used as it is as an active ingredient, or alternatively, the active ingredient is further fractionated and purified by using the activity measured by the measuring method described later in Examples or the polymerization degree, as an indicator. Further, when the extract contains proanthocyanidins with a polymerization degree of 3 or less, a product having quantities of the active ingredient with a polymerization degree of 4 or more can be obtained by fractionation and purification by using the activity measured by the measuring method described later in Examples or the polymerization degree, as an indicator. In a pharmaceutical composition or a food product which contains proanthocyanidins with various degrees of polymerization, as long as it contains an effective amount of proanthocyanidins with a polymerization degree of 4 or more, it is within a scope of the invention even when the average degree of polymerization is less than 4; however, proanthocyanidins with an average degree of polymerization of about 4 or more is preferably used.

Besides by the extraction from plants, proanthocyanidins can also be prepared by organochemical synthesis (see, for example, Swain et al., 1954. Chem. Ind. 1144; Eastmond, 1974. J. Inst. Brew. 80, 188; Kozikowski AP et al., 2003, J. Org. Chem. 68, 1641-1658; and Japanese Patent Publication 2002-542240).

### Use

Proanthocyanidins and extracts of specif ic plants, such as and Jatoba, used in the present invention suppress the production of Th1 cytokine (specifically, IFN-γ) in Th1-biased splenocytes and thus improve or suppress excessive cell-mediated immune conditions. Accordingly, a pharmaceutical composition according to the present invention is effective in treating a disease in which the suppression of Th1 cytokine production is therapeutically effective.

An example of the disease in which the suppression of Th1 cytokine production is therapeutically effective is a Th1 cytokine-dependent autoimmune disease. In the present invention, the term "Th1 cytokine-dependent autoimmune disease" is intended to include a cell-mediated immunity-dependent autoimmune disease.

Examples of the Th1-dependent autoimmune disease include multiple sclerosis (MS), chronic rheumatoid arthritis (RA), insulin-dependent diabetes mellitus (IDDM) such as juvenile diabetes and type I diabetes, autoimmune thyroiditis (AT), autoimmune uveoretinitis (AUR), Hashimoto' s disease, psoriasis, Crohn's disease, and alopecia areata.

In Th1-dependent autoimmune diseases, an extravasation of lymphocytes (Th1 cells) into an inflammatory site and an increase in the concentration of Th1 cytokine (e.g., IFN-γ, TNF-α) in blood have been observed (Skurkovich B & Skurkovich S, Curr. Opin. Mol. Ther. 2003 Feb 5 (1) :52-7; Fuss, I.J., et al., J. Immunol . 157:1261 (1996) ; Gherardo M. , etal., European Journal of Endocrinology (2003), 148, 383-388). Further, it has been reported that the symptoms of autoimmune diseases were actually improved using anti-IFN-γ antibodies or anti-TNF-α antibodies (Skurkovich B & Skurkovich S, Curr. Opin. Mol. Ther. 2003 Feb 5 (1) :52-7) . Accordingly, the suppression of a causative factor of these diseases, Th1 cytokine (e.g., IFN-γ, TNF-α), is effective for the treatment of thesediseases .

Highly polymerized proanthocyanidins used in the present invention suppress autoantibody production in mice in which autoantibodies are induced (Example 30) . An extract containing highly polymerized proanthocyanidins used in the present invention suppress activation of B cells which closely associates with the autoantibody production (Example 25). Accordingly, a pharmaceutical composition according to the present invention is effective for the treatment of the diseases in which the suppression of autoantibody production or B cell activation is therapeutically effective.

Examples of the diseases in which the suppression of autoantibody production or B cell activation is therapeutically effective include autoantibody-mediated autoimmune diseases (e.g.,systemic lupus erythematodes, Good Pasture's syndrome, autoimmune hemolytic anemia, Sjogren's syndrome, acute rheumatic fever, pemphigus vulgaris, autoimmune thrombopenic purpura, and mixed type essential cryoglobulinemia).

In the autoantibody-mediated autoimmune diseases, autoantibody production or acceleration of the production and B cell activation have been recognized (Clin. Exp. Immunol. 1985, 62, 639-46; N. Engl. J. Med. 338, 1359). Further, there have been reported that the suppression of B cell activation is effective for the treatment of autoimmune diseases caused byautoantibodyproduction (Int. Immunol. 2001, 13 (7), 921-31) and that inhibition of autoreactive B cell growth coincides with amelioration of systemic lupus erythematodes (J. Clin. Invest. 2000, 106 (1), 91-101). Accordingly, the suppression of production of autoantibodies which are causative agents of these diseases and the suppression of activation of B cells which are highly involved in the autoantibody production are therapeutically effective for these diseases. In the present invention, "suppression of autoantibody production" is intended to include the suppression of activation of B cells which are highly involved in the autoantibody production.

Proanthocyanidins used in the present invention can suppress Th1 cytokine production. Accordingly, the present invention is more advantageous than therapeutic agents used for symptomatic treatment therapy because it can eradicatively treat diseases in which Th1 cytokine production is an essential cause or it can inhibit the progress of these diseases. The present invention is considered to be advantageous particularly in the treatment of chronic rheumatoid arthritis because it can eradicatively treat the disease or it inhibits the progress of the disease unlike conventional therapeutic agents used in symptomatic treatment therapy which are efficacious, for example, in suppressing inflammation and alleviating pain. The eradicative treatment of chronic rheumatoid arthritis with proanthocyanidins is supported by Examples 28 and 29 in which clinical scores were improved in a chronic rheumatoid arthritis model in mice and Example 30 in which rheumatoid antibody (autoantibody) production was suppressed. Further, the inhibition of the progress of the symptoms of chronic rheumatoid arthritis with proanthocyanidins is supported by Examples 28 and 29 in which clinical scores were improved even after the establishment of the primary immunity in a chronic rheumatoid arthritis model in mice and Example 30 in which rheumatoid antibody (autoantibody) production was suppressed.

In chronic rheumatoid arthritis, it has been reported that in the early stage of the disease, symptoms start developing as T cells (specifically Th1 cells) are activated and in the middle or chronic stage of the disease and that autoantibody production of B cells is stimulated by the activated T cells and thus the abnormally produced autoantibodies make the symptoms chronic and severe (Clin. Exp. Immunol. 1998, 111, 521-526; Springer Seminlmmunopathol. 2003, Aug, 25 (1), 3-18; Immunol . Rev. 1990, Dec, 118, 193-232) . It has also been reported that rheumatism is suppressed in mice by lowering the IgG level (Ann. Rheum. Dis. 2003, 62, 707-14). As mentioned above,proanthocyanidinssuppressed Th1 cytokine, suppressed Th1 cell activation and at the same time actually suppressed the production of rheumatoid antibodies which are autoantibodies stimulated by the Th1 cell activation. Further, the active ingredient according to the present invention suppressed the activation of B cells which are highly involved in the autoantibody production. Accordingly, proanthocyanidins used in the present invention are advantageous also in respect that they can be used in treating not only conditions in early stage of chronic rheumatoid arthritis but also chronically and severely developed conditions.

Accordingly, according to the present invention, there are provided an agent for the eradicative treatment of chronic rheumatoid arthritis and an agent for inhibiting its progress, comprising (i) proanthocyanidins with a polymerization degree of 4 or more (preferably, procyanidins and/or prodelphinidins with a polymerization degree of 5 or more) or (ii) a plant material extract containing proanthocyanidins (preferably, procyanidins and/or prodelphinidins with a polymerization degree of 5 or more), as an active ingredient.

According to the present invention, there are also provided a method for the eradicative treatment of chronic rheumatoid arthritis and a method for inhibiting its progress, comprising administering to a mammal a therapeutically effective amount of (i) proanthocyanidins with a polymerization degree of 4 or more (preferably, procyanidins and/or prodelphinidin with a polymerization degree of 5 or more) or (ii) a plant material extract containing proanthocyanidins (preferably, procyanidins and/or prodelphinidins with a polymerization degree of 5 or more), if necessary, together with a pharmaceutically acceptable carrier.

According to the present invention, there is also provided use of (i) proanthocyanidins with a polymerization degree of 4 or more (preferably, procyanidin and/or prodelphinidin with a polymerization degree of 5 or more) or (ii) a plant material extract containing proanthocyanidins (preferably, procyanidin and/or prodelphinidin with a polymerization degree of 5 or more) for the manufacture of a therapeutic agent for the eradicative treatment of chronic rheumatoid arthritis and an agent for inhibiting its progress.

In the present invention, "treatment" is intended to include amelioration, control, delay, and prevention (particularly prevention of onset and recurrence) of diseases.

### Pharmaceutical composition and food composition

A therapeutic agent according to the present invention can be manufactured by using proanthocyanidins, the abovementioned active ingredient of the present invention, or a plant extract containing the same or a partially purified product from said extract, as an active ingredient, and by mixing them with physiologically acceptable carriers, excipients, binding agents, diluents, and the like. The therapeutic agent according to the present invention can be administered orally or non-orally. Examples of oral formulations include granules, dispersible powders, tablets (including sugar-coated tablets), pills, capsules, syrups, emulsions, and suspensions. Examples of non-oral formulations include injections (e.g., subcutaneous injections, intravenous injections, intramuscular injections, intraperitonealinjections),intravenous drips,preparations for external use (e.g., nasal formulations, percutaneous agents, ointments), and suppositories (e.g., rectal suppositories, vaginal suppositories) . These pharmaceutical preparations can be formulated by a method generally used in this field using pharmaceutically acceptable carriers (e.g., excipients, additives). Examples of pharmaceutically acceptable excipients and additives include carriers, binding agents, flavoring agents, buffers, thickening agents, coloring agents, stabilizers, emulsifying agents, dispersing agents, suspending agents, and preservatives. Examples of pharmaceutically acceptable carriers include magnesium carbonate, magnesium stearate, talc, sucrose, lactose, pectin, dextrin, starch, gelatin, tragacanth, methylcellulose, sodium carboxymethylcellulose, low-melting wax, and cacao butter.

Pharmaceutical preparations can be produced, for example, as follows.

Oral formulations can be manufactured by adding, for example, excipients (e.g., lactose, sucrose, starch, mannitol), disintegrating agents (e.g., calcium carbonate, calcium carboxymethylcellulose), binding agents (e.g., pregelatinized starch, gum arabic, carboxymethylcellulose, polyvinylpyrrolidone, hydroxypropylcellulose), or lubricating agents (e.g., talc, magnesium stearate, polyethylene glycol 6000) to an active ingredient, pressing the admixture into an appropriate form, and if necessary, coating for the purpose of taste masking, enteric film coating or durability using a known method. Examples of coating agents include ethylcellulose, hydroxymethylcellulose, polyoxyethylene glycol, cellulose acetate phthalate, hydroxypropylmethylcellulose phthalate, and Eudragit (methacrylic acid-acrylic acid copolymer; Roehm, Germany).

Formulations for injection can be manufactured by dissolving, suspending or emulsifying an active ingredient in an aqueous solvent (e.g., distilled water, physiological saline, Ringer's solution) or an oily medium (e.g., vegetable oils such as olive oil, sesame oil, cotton seed oil, and corn oil, or propylene glycol) together with dispersing agents (e.g., Tween 80 (Atlas Powder, USA), HCO 60 (Nikko Chemicals), polyethylene glycol, carboxymethylcellulose, sodium alginate), preservatives (e.g., methylparabene, propylparabene, benzylalcohol, chlorobutanol, phenol), osmosis equilibrating agents(e.g., sodium chloride , glycerin, sorbitol, glucose, invert sugar) and the like. If desired, additives such as solubilizing agents (e.g., sodium salicylate, sodium acetate), stabilizing agents (e.g., human serum albumin) and analgesic agents (e.g., benzalkonium chloride, procaine hydrochloride) may be added.

Pharmaceutical preparations for external use can be manufactured by formulating an active ingredient into a solid, semi-solid or liquid composition. For example, the above-mentioned solid composition can be manufactured by formulating an active ingredient into a powder as it is or by ad mixing it with excipients (e.g., lactose, mannitol, starch, microcrystal cellulose, sucrose), thickening agents (e.g., natural gums, cellulose derivatives, acrylic acid polymers)and thelike.The above-mentioned liquid composition can be manufactured in almost the same manner as described for injectable preparations. The semi-solid composition is preferably an aqueous or oleaginous gel or ointment. Further, any of these compositions can contain a pH controlling agent (e.g., carbonic acid, phosphoric acid, citric acid, hydrochloric acid, sodium hydroxide), a preservative (e.g., paraoxybenzoic acid esters, chlorobutanol, benzalkonium chloride), and the like. Suppositories can be manufactured by formulating an active ingredient into an oleaginous or aqueous solid, semi-solid, or liquid composition. Examples of the oleaginous base to be used for such compositions include higher fatty acid glycerides (e.g., cacao oil, Witepsols (Dynamite Nobel)), medium fatty acids (e.g., Miglyols (Dynamite Nobel)), and vegetable oils (e.g., sesame oil, soybean oil, cotton seed oil) . Examples of the aqueous base include polyethylene glycols and propylene glycols. Further, examples of the aqueous gel base include natural gums, cellulose derivatives, vinyl polymers, and acrylic acid polymers.

In formulation, in addition to an active ingredient according to the present invention, one or more medically effective active ingredients can be admixed. Further in the administration of an active ingredient according to the present invention, one or more medically effective active ingredients can be administered in combination. Examples of these other active ingredients include, but are not limited to, anti-inflammatory agents, anti-inflammatory analgesics, immunosuppressants, and other therapeutic agents for autoimmune diseases. Examples of the anti-inflammatory agents include steroidal anti-inflammatory agents and nonsteroidal anti-inflammatory agents. Examples of the steroidal anti-inflammatory agents include cortisone acetate, hydrocortisone, paramethasone acetate, prednizolon, methyl predonine, dexamethasone, triamcinolone, and betamethasone. Examples of the nonsteroidal anti-inflammatory agents include salicylic acid nonsteroidal anti-inflammatory agents such as aspirin, diflunisal, aspirin/ascorbic acid, and aspirin dialuminate; aryl acid nonsteroidal anti-inflammatory agents such as diclofenac sodium, sulindac, fenbufen, indomethacin, indomethacin farnesyl, acemethacin, proglumetacin maleate, amfenac sodium, nabumetone, mofezolac, and etodolac; fenamic acid nonsteroidal anti-inflammatory agents such as mefenamic acid, aluminium flufenamate, tolfenamic acid, and floctafenin; propionic acid nonsteroidal anti-inflammatory agents such as ibuprofen, flurubiprofen, ketoprofen, naproxen, pranoprofen, fenoprofen calcium, tiaprofen, oxaprozin, loxoprofen, sodium, alminoprofen, and zaltoprofen; oxicam nonsteroidal anti-inflammatory agents such as piroxicam, ampiroxicam, tenoxicam, lornoxicam, and meloxicam; basic nonsteroidal anti-inflammatory agents such as tiaramide hydrochloride, epilizole, and emorfazone. Examples of the immunosuppressants include cyclosporin and FK506. Examples of the other therapeutic agents for autoimmune diseases include therapeutic agents for multiple sclerosis such as interferon β.

By combining an active ingredient according to the present invention with other active ingredients such as anti-inflammatory agents, anti-inflammatory analgesics, and immunosuppressive agents, a multi-functional therapeutic agent having a further fortified therapeutic activity can be provided. In particular, a therapeutic agent which is expected to be effective in eradicative therapy and symptomatic therapy of autoimmune diseases can be provided by using the active ingredient according to the present invention together with pharmaceutical agents used in symptomatic therapy of autoimmune diseases, such as anti-inflammatory agents and anti-inflammatory analgesics. Further, by combining the active ingredient according to the present invention with pharmaceutical agents which are expected to be effective in eradicative therapy, such as immunosuppressants, a therapeutic agent having fortified effects for the eradicative treatment of autoimmune diseases can be provided. In treating chronic rheumatoid arthritis, the active ingredient according to the present invention may be administered in combination with a chondroitin or mucopolysaccharide mainly consisting of glucosamines.

A therapeutic agent according to the present invention is intended to be applicable not only to pharmaceutical products but also to food products. Accordingly, in applying the therapeutic agent according to the present invention, the following descriptions for food product can be referred to.

A food according to the present invention is a food containing an effective amount of an active ingredient according to the present invention, proanthocyanidins. The expression "containing an effective amount of an active ingredient" herein means that the active ingredient is contained in such an amount that it can be ingested in the range described below when a food or drink is taken in its usual amount. An active ingredient according to the present invention can be blended into a food according to the present invention as it is or in the form of the above-mentioned compositions. More specifically, a food according to the present invention can be one prepared as a food or drink by using the abovementioned active ingredient according to the present invention, proanthocyanidins, or a plant extract containing it or a partially purified product from said extract as it is; one further admixed with various proteins, sugars, fats, trace elements, vitamins, and the like; one formulated into a form of liquid, semi-liquid, solid, or paste; or one added to a general food or drink. In the present invention, "food" is not a medicine and its form is notparticularly limited as long as it is ingestible for mammals.

The term "food" as used in the present invention is intended to include health foods (e.g., foods for specified health use, nutritional functional foods, nutrient supplement foods), functional foods, and foods for patients.

Further, the form of the "food" is not particularly limited and can be, for example, a drink form.

According to the present invention, there is provided a health food containing proanthocyanidins with a polymerization degree of at least 4 so that the amount of its daily ingestion for an adult ranges from 50 to 2000 mg (preferably from 400 to 2000 mg).

According to the present invention, there is also provided a health food containing proanthocyanidins with a polymerization degree of at least 5 so that the amount of its daily ingestion for an adult ranges from 50 to 2000 mg (preferably from 400 to 2000 mg) .

The proanthocyanidins contained in the abovementioned health food is an extract selected from the group consisting of a Jatoba extract, a grape seed extract, a cranberry extract, a cinnamon extract, a pine bark extract, and a cacao extract and can be provided in the form of an extract containing proanthocyanidins with a polymerization degree of at least 4 or at least 5.

The abovementioned health food can be in the form of ordinary foods or in the form of nutrient supplement foods (for example, supplements).

An active ingredient according to the present invention suppresses the production of Th1 cytokine (specifically, IFN-γ) in Th1-biased splenocytes and has an activity to ameliorate or suppress a hyperactive cell-mediated immune state. The active ingredient according to the present invention also suppresses autoantibody production in mice in which autoantibodies are induced. Accordingly, it is possible to provide a food product which functions versatilely in suppressing Th1 cytokine production, suppressing autoantibody production, and ameliorating or alleviating conditions associated with the enhancement of Th1 cytokine production and conditions associated with the autoantibody production, in particular in treating autoimmune diseases, by admixing an active ingredient or an extract, such as a Jatoba extract, of the present invention with daily foods, health foods and functional foods taken as supplements.

Accordingly, according to the present invention, there is provided a food which comprises (i) proanthocyanidins with a polymerization degree of at least 4 (preferably, procyanidins and/or prodelphinidins with a polymerization degree of at least 5) or (ii) a plant material extract containing proanthocyanidins (preferably, a plant material extract containing procyanidins and/or prodelphinidins with a polymerization degree of at least 5), as an active ingredient, and has an indication for a function of suppressing Th1 cytokine production, a function of suppressing autoantibody production, or a function of ameliorating or alleviating conditions associated with enhancement of Th1 cytokine production and conditions associated with autoantibody production (for example, systemic stiffness or strained feeling in joints characteristic to Th1 cytokine-dependent autoimmune diseases and autoantibody-mediated autoimmune diseases). Such an indication is placed on food itself, a container, a package, an instruction, an attached document, or an advertisement.

According to the present invention, there is provided a food comprising an effective amount of (i) proanthocyanidins with a polymerization degree of at least 4 (preferably, procyanidins and/or prodelphinidins with a polymerization degree of at least 5) or (ii) a plant material extract containing proanthocyanidins (preferably, a plant material extract containing procyanidins and/or prodelphinidins with a polymerization degree of at least 5), which is used for the treatment of diseases in which the suppression of Th1 cytokine production is therapeutically effective and/or diseases in which the suppression of autoantibody production is therapeutically effective, the eradicative treatment of these diseases, and/or the inhibition of the progress of these diseases.

According to the present invention, there is also provided a food comprising an effective amount of (i) proanthocyanidins with a polymerization degree of at least 4 (preferably, procyanidins and/or prodelphinidins with a polymerization degree of at least 5) or (ii) a plant material extract containing proanthocyanidins (preferably, a plant material extract containing procyanidins and/or prodelphinidins with a polymerization degree of at least 5), which has a function of suppressing Th1 cytokine production, a function of ameliorating or alleviating conditions associated with enhancement of Th1 cytokine production, a function of suppressing autoantibody production, or a function of ameliorating or alleviating conditions associated with autoantibody production.

According to the present invention, there is further provided a food comprising an extract which is (ii) a plant material extract containing proanthocyanidins which has a function of suppressing Th1 cytokine production, a function of ameliorating or alleviating conditions associated with enhancement of Th1 cytokine production, a function of suppressing autoantibody production, or a function of ameliorating or alleviating conditions associated with autoantibody production.

A food according to the present invention can be provided as a food appropriate for consumers who expect functions such as a function of suppressing Th1 cytokine production, a function of suppressing autoantibody production, or a function of ameliorating or alleviating conditions associated with enhancement of Th1 cytokine production or conditions associated with autoantibody production, namely as a food for specified health use. The food for specified health use herein means a food which is regulated by a certain law in individual countries from the viewpoint of public health when the food is, for example, manufactured or sold for the purpose of, for example, suppressing Th1 cytokine production, suppressing autoantibody production, or ameliorating or alleviating conditions associated with enhancement of Th1 cytokine production or conditions associated with autoantibody production.

Examples of the daily food to which an active ingredient or an extract such as a Jatoba extract according to the present invention is admixed include, but are not particularly limited to, foods and drinks containing carbohydrate, such as rice products, noodles, breads, and pastas; various confectionaries including western sweets such as cookies and cakes, Japanese sweets such as buns with a filling and steamed adzuki-bean pastes, candies, chewing gums, and chilled sweets such as yogurt, puddings and jelly; various drinks such as juice, softdrinks, and milk drinks; processed foods with eggs; andprocessedfoods (including delicacies) using seafood (e.g., squid, octopus, shellfish, eel) or meat (including entrails such as liver).

According to a preferred embodiment of the present invention, examples of the food for admixing are drinks (e.g. , tea drinks, milk drinks) and yogurt.

"Tea drinks" as used herein means drinks which are made by infusing leaves of the tea plant, i.e., an evergreen shrub of the Theacae family (tea leaves), leaves of plants other than tea plant, or grains and includes fermented tea, semi-fermented tea and non-fermented tea. Specific examples of tea drinks include Japanese tea (e.g., green tea, roasted barley tea), black tea, herbal tea (e.g., jasmine tea), Chinese tea (e.g., Chinese green tea, oolong tea) and roasted tea.

"Milk drinks" as used herein means drinks which use raw milk, cow's milk or the like or food products which are manufactured using them as a raw material, as a major raw material, and include those which use cow's milk or the like as a raw material and those which use processed milk products, such as nutrient fortified milk, flavored milk and sweetened hydrolyzed milk, as a raw material.

Further, "yogurt" as used herein includes hard yogurt, soft yogurt, and drink-type yogurt as well as processed yogurt products which use yogurt as a raw material.

An example of the daily food to which an active ingredient or an extract such as a Jatoba extract according to the present invention is admixed is a food which originally contains proanthocyanidins. A function of suppressing Th1 cytokine production, a function of suppressing autoantibody production, or a function of ameliorating or alleviating conditions associated with enhancement of Th1 cytokine production or conditions associated with autoantibody production can be fortified by admixing an active ingredient or an extract such as a Jatoba extract according to the present invention into the food which originally contains proanthocyanidins. "Fortification" as used herein means admixing an active ingredient or an extract according to the present invention so that the amount of the active ingredient originally contained in the food exceeds the amount necessary to exhibit the expected functions. Further, examples of the food originally containing proanthocyanidins include, but are not limited to, cranberry products such as cranberry juice and cranberry jam; cacao products such as chocolates and cocoa; cinnamon products such as cinnamon sticks, cinnamon sugar, cinnamon gum, and cinnamon tea.

Examples of the form of health foods or functional foods ingested as a supplement to which an active ingredient or an extract such as Jatoba extract is admixed include drinks such as juice and tea, jelly, capsules, granules, pills, and paste. A health food such as a supplement and a functional food have a function of suppressing Th1 cytokine production, a function of suppressing autoantibody production, or a function of ameliorating or alleviating conditions associated with enhancement of Th1 cytokine production or conditions associated with autoantibody production can be provided by processing an active ingredient or an extract such as a Jatoba extract of the present invention alone or in combination with other components (for example, plant materials) into the form of drinks, jelly, capsules, granules, pills, paste and the like. In particular, a function of ameliorating or alleviating conditions associated with enhancement of Th1 cytokine production or conditions associated with autoantibody production can be further fortified by combining with other components known to have anti-inflammatory activity, anti-inflammatory analgesic activity or immunosuppressive activity, in addition to an active ingredient according to the present invention. Other components known to have anti-inflammatory activity, anti-inflammatory analgesic activity or immunosuppressive activity can be selected from generally known components.

Examples of components having anti-inflammatory activity include allicin, capsicin, bromelain, and allyl sulfide. These components are contained in plant materials; exemplary plant materials containing allicin are onions, Chinese chive, garlic, garlic chive and green onions; an exemplary plant material containing capsicin is red pepper; an exemplary plant materialcontaining bromelain is pineapple; and exemplary plant materials containing allyl sulfide are asatsuki (babyscallion, Alliumschoenoprasum L. var. foliosum Regel), garlic, shallot, Welshonion, greenonion, garlicchive, and rakkyo onion. Further, an exemplary component having immunosuppressive activity is glycyrrhitinic acid and an exemplary plant material containing glycyrrhitinic acid is kanzo (Glycyrrhizae radix). Further, plant materials, such as Tripterygium wilfordii Hook known to be effective for rheumatism, Ninjin-to (Ren-Shen-Tang) known to be effective for type I diabetes, kanran (Brassica oleracea) known to be effective to multiple sclerosis, and Polypodium leucotomos known to be effective for psoriasis, can be combined. Further, a multi-functional food can be provided by the combination with other components which exhibit functions other than those according to the present invention or with other functional foods.

In manufacturing drinks provided in the present invention (including drink-type health foods and functional foods), sugar, flavors, juice, food additives and the like which are used in formulation of ordinary drink products can be appropriately added. Further, in manufacturing drinks, manufacturing technologies known in the art, for example those in "New Edition Soft Drinks" (Korin Publishing Co., Ltd.) can be referred to.

According to a preferred embodiment of the present invention, proanthocyanidins with a polymerization degree of at least 5 (preferably, procyanidins and/or prodelphinidins with a polymerization degree of at least 5) can be admixed with a food which originally contains proanthocyanidins, for the purpose of, for example, fortifying a function of suppressing Th1 cytokine production, a function of suppressing autoantibody production, or a function of ameliorating or alleviating conditions associated with enhancement of Th1 cytokine production or conditions associated with autoantibody production. When an active ingredient or an extract according to the present invention is used as a food as it is or by admixing with a raw material for a general food, it is desirable to prevent the food frombeing affected by bitterness of the active ingredient by limiting the amount of use or manipulatively masking the bitterness. For example, the bitterness can be masked by capsulating the active ingredient or the extract or coating it with an appropriate coating agent. Exemplary capsule forms are gelatin capsules and pullulan capsules, and an exemplary coated form is a sugar coated tablet.

A food according to the present invention can be made into various forms and can be manufactured in accordance with known technology for manufacturing pharmaceutical products. In such cases, it can be manufactured using carriers or additives for manufacturing pharmaceutical products as mentioned above in the section for manufacturing therapeutic agents according to the present invention, more specifically, in the section for oral agents.

Since proanthocyanidins, the active ingredient according to the present invention, are contained largely in cacao or grapes that have been ingested by humans for many years as foods, they are low in toxicity and can be used safely for mammals (e.g. , humans, mice, rats, rabbits, canines, cats, cattle, horses, pigs, monkeys).

When a therapeutic agent and a food according to the present invention are administered or taken, the amount of administration or intake for an active ingredient according to the present invention can be determined depending on the recipient, recipient's age, body weight, and symptoms, the time of administration, the type of dosage form, the route of administration, the combination with other medicines, and the like. For example, an active ingredient according to the present invention can be administered as a medicine to an adult orally at a daily dose ranging from 50 to 5000 mg, preferably 50 to 2000 mg, and non-orally at a daily dose ranging from 5 to 500 mg, preferably 5 to 200 mg, as an amount of proanthocyanidins. Appropriate dosages of medicines having other functions to be used in combination with an active ingredient according to the present invention can be determined based on their individual dosage for clinical use. Further, when taken as a food, an active ingredient according to the present invention can be admixed in the food so that the amount of its daily ingestion for an adult ranges from 50 to 5000 mg, preferably from 50 to 2000 mg.

According to a preferred embodiment of a therapeutic agent according to the present invention, there are provided a therapeutic agent for the treatment of diseases in which the suppression of Th1 cytokine production is therapeutically effective (particularly Th1 cytokine-dependent autoimmune diseases), a therapeutic agent for the eradicative treatment of these diseases, and an agent for inhibiting the progress of these diseases, comprising (i) procyanidins and/or prodelphinidins with a polymerization degree of at least 5 or (ii) aplantmaterialextractcontainingprocyanidinsand/or prodelphinidins with a polymerization degree of at least 5 as an active ingredient.

According to a preferred embodiment of the present invention, there is provided use of (i) procyanidins and/or prodelphinidins with a polymerization degree of at least 5 or (ii) aplantmaterialextractcontainingprocyanidinsand/or prodelphinidins with a polymerization degree of at least 5 for the manufacture of a therapeutic agent for the treatment of diseases in which the suppression of Th1 cytokine production is therapeutically effective (particularly Th1 cytokine-dependent autoimmune diseases), a therapeutic agent for the eradicative treatment of these diseases, and an agent for inhibiting the progress of these diseases.

According to a preferred embodiment of the present invention, there are provided a method for treating diseases in which the suppression of Th1 cytokine production is therapeutically effective (particularly Th1 cytokine-dependent autoimmune diseases), a therapeutic method for the eradicative treatment of these diseases, and a therapeutic method for inhibiting the progress of these diseases, comprising administering to a mammal a therapeutically effective amount of (i) procyanidins and/or prodelphinidins with a polymerization degree of at least 5 or (ii) aplantmaterialextractcontainingprocyanidinsand/or prodelphinidins with a polymerization degree of at least 5, if necessary, together with pharmaceutically acceptable carriers.

### Agents for suppressing Th1 cytokine production and agents for suppressing autoantibody production

According to the present invention, there is provided an agent for suppressing Th1 cytokine production, comprising (i) proanthocyanidins with a polymerization degree of at least 4 (preferably, procyanidins and/or prodelphinidins with a polymerization degree of at least 5) or (ii) a plant material extract containing proanthocyanidins (preferably, a plant material extract containing procyanidins and/or prodelphinidins with a polymerization degree of at least 5) as an active ingredient.

According to the present invention, there is provided use of (i) proanthocyanidins with a polymerization degree of at least 4 (preferably, procyanidins and/or prodelphinidins with a polymerization degree of at least 5) or (ii) a plant material extract containing proanthocyanidins (preferably, a plant material extract containing procyanidins and/or prodelphinidins with a polymerization degree of at least 5) for the manufacture of an agent for suppressing Th1 cytokine production.

According to the present invention, there is provided a method for suppressing Th1 cytokine production, comprising administering to a mammal an effective amount of (i) proanthocyanidins with a polymerization degree of at least 4 (preferably, procyanidins and/or prodelphinidins with a polymerization degree of at least 5) or (ii) a plant material extract containing proanthocyanidins (preferably, a plant material extract containing procyanidins and/or prodelphinidins with a polymerization degree of at least 5) .

The Th1 cytokine is preferably IFN-γ.

The agent for suppressing Th1 cytokine production is intended not only to be applied to pharmaceutical products for the purpose of suppressing Th1 cytokine production but also to be applied to foods for the purpose of suppressing Th1 cytokine production. Accordingly, in the embodiment of the agent for suppressing Th1 cytokine production according to the present invention, the abovementioned descriptions for therapeutic agents and foods can be referred to.

According to the present invention, there is provided an agent for suppressing autoantibody production, comprising (i) proanthocyanidins with a polymerization degree of at least 4 (preferably procyanidins and/or prodelphinidins with a polymerization degree of at least 5) or (ii) a plant material extract containing proanthocyanidins (preferably, a plant material extract containing procyanidins and/or prodelphinidins with a polymerization degree of at least 5) as an active ingredient.

According to the present invention, there is also provided use of (i) proanthocyanidins with a polymerization degree of at least 4 (preferably procyanidins and/or prodelphinidins with a polymerization degree of at least 5) or (ii) a plant material extract containing proanthocyanidins (preferably, a plantmaterial extract containing procyanidins and/or prodelphinidins with a polymerization degree of at least 5) for the manufacture of an agent for suppressing autoantibody production.

According to the present invention, there is also provided a method of suppressing autoantibody production, comprising administering to a mammal (i) proanthocyanidins (preferably procyanidins and/or prodelphinidins with a polymerization degree of at least 5) or (ii) a plant material extract containing proanthocyanidins (preferably, a plant material extract containing procyanidins and/or prodelphinidins with a polymerization degree of at least 5) .

The agent for suppressing autoantibody production is intended not only to be applied to pharmaceutical products for the purpose of suppressing autoantibody production but also to be applied to foods for the purpose of suppressing autoantibody production. Accordingly, in the embodiment of the agent for suppressing autoantibody production according to the present invention, the abovementioned descriptions for therapeutic agents and foods can be referred to.

### Test method

According to the present invention, there is provided a method for testing an activity for suppressing Th1 cytokine production, comprising the step of measuring the polymerization degree of proanthocyanidins in a sample, characterized in that the detection of proanthocyanidins with a polymerization degree of 4 or more demonstrates the activity for suppressing Th1 cytokine production.

Samples to be tested are, for example, extracts and dried powders derived from plant materials. The plant materials include not only plant materials containing proanthocyanidins but also plant materials in which the presence of proanthocyanidins is unknown. Exemplary extracts are those extracted with water or organic solvents.

Other examples of the samples to be tested are synthesized proanthocyanidins and samples for which the presence of proanthocyanidins is unknown.

The polymerization degree of proanthocyanidins can be measured, for example, by the electrospray ionization mass spectrometry (ESI-MS) or the matrix-assisted laser desorption/ionization time-of-flight mass spectrometry (MALDI-TOF-MS).

In the test method according to the present invention, whether proanthocyanidins with a polymerization degree of 5 or more are contained in a sample can be an index. As shown in Example 31 and Fig. 50, proanthocyanidins with a polymerization degree of 5 or more exhibit a high therapeutic activity in EAE model mice. Accordingly, whether a sample has a high activity for suppressing Th1 cytokine production can be tested by applying such an index.

Also, the test method according to the present invention can be used for testing a therapeutic activity for diseases in which the suppression of Th1 cytokine production is therapeutically effective.

### EXAMPLES

The present invention is further illustrated by the following examples that are not intended as a limitation of the invention.

### Example 1: study on the ability of various Amazon herb extracts to suppress IFN-γ production using Th1-biased mouse splenocytes

A series of Amazon herbs were studied on their ability to suppress IFN-γ production in vitro using Th1-biased mouse splenocytes.

### 1. Experimental methods

### (1) Samples

To commercial dried powders (10 g each) of Samambaia (Polypodium lepidopteris), Jatoba (Hymenaea courbaril), Cat's Claw (Uncaria tomentosa), Psidium (Psidium guajava), and Jergon Sacha (Dracontium loretense Krause) (Edison SRL, Japan Office) was added 200 ml each of ethanol, and the admixtures were stirred overnight. After filtration, the solvent was removed by distillation to obtain ethanol extracts (Table 2). The extracts were each dissolved in 1% ethanol to prepare samples for administration.

**Table 2: In Example 1 of the present invention, the amounts of ethanol extracts of Samambaia, Jatoba, Cat' s Claw, Psidium, and Jergon Sacha are shown.**

| | Amount of ethanol extract |
|---|---|
| Samambaia | 0.46 g |
| Jatoba | 1.84 g |
| Cat's Claw | 0.89 g |
| Psidium | 1.08 g |
| Jergon Sacha | 0.15 g |

### (2) Experimental animals

To 6-8 week-old male C57BL/6 mice (Charles River Laboratories), an emulsion of 1 mg of ovalbumin (OVA; Seikagaku Kogyo) emulsified with complete Freund's adjuvant (Sigma) supplemented with 0.8 mg of Mycobacterium tuberculosis H37Ra (Difco) (referred to as CFA hereinafter) was administered intraperitoneally. On day 14, the animals were dissected and splenocytes were prepared according to an ordinary method.

### (3) Measurement of splenocyte IFN-γ

IFN-γ was measured using an OptEIA ELISA Set (Becton Dickinson).

### (4) Experimental conditions

The splenocytes prepared as in the experimental animal section were suspended in an RPMI1640 (Sigma) + 10% FCS (Roche) + 1 mg OVA medium at 5 × 10⁶ cells/ml and the suspension was dispensed into a 96-well plate at 200 µl/well. Further, each herb extract to be tested was added at 100 or 500 µg/ml. After one week, the culture supernatant was recovered and IFN-γ was measured for Th1 cytokine.

### 2. Experimental results

The results are shown in Table 3. Samambaia showed no ability to suppress IFN-γ production and rather increased the production. A similar increase in IFN-γ production was observed with other herb extracts. A marked suppression of IFN-γ production was observed only with Jatoba.

**Table 3: Suppressive or enhancing activity of various Amazon herbs on IFN-γ, a Th1 cytokineproduced specifically to antigens, in Th1-biased mouse splenocytes is shown.**

| | mIFN-γ (pg/ml) | SD |
|---|---|---|
| Control | 309 | 110.8 |
| Jatoba | 64 | 68.5 |
| Psidium | 1431 | 395.9 |
| Samambaia | 2940 | 120.2 |
| Cat's Claw | 1722 | 655.5 |
| Jergon Sacha | 2917 | 166.6 |

### Example 2: Study on the concentration dependence of IFN-γ suppressing activity of Jatoba extract in vitro

The concentration dependence of the Jatoba extract which exhibited IFN-γ suppressing activity in vitro in Experiment 1 was studied.

### 1. Experimental method

### (1) Samples for administration

To 100 g of Jatoba driedpowder was added 500 ml of ethanol, and the admixture was stirred at room temperature overnight. After filtration, the residue was extracted again with an equal volume of ethanol. The filtrate was concentrated to dryness to obtain 27.2 g of ethanol extract. The extract was dissolved in 1% ethanol to prepare a sample for administration.

### (2) Experimental animals

In the same manner as described in Example 1, C57BL/ 6 mice were immunized twice with the OVA emulsified with CFA to obtain Th1-biased mice.

### (3) Measurement of splenocyte IFN-γ

IFN-γ was measured using an OptEIA ELISA Set (Becton Dickinson).

### (4) Experimental conditions

The splenocytes were cultured in an RPMI1640 (Sigma) + 10% FCS (Roche) + 100 µg/ml OVAmedium at 37°C in an atmosphere of 5% CO₂ for one week and then the culture supernatant was recovered to measure the IFN-γ concentration. Concentrations of the Jatoba extract added were 0 (control), 15.5, 31, 62.5, 125, 250, and 500 µg/ml.

### 2. Experimental results

The IFN-γ concentrations in the supernatants after 7 days of culture are shown in Table 4. High IFN-γ production by the addition of OVA was observed in the control (no Jatoba extract added) . The IFN-γ production was suppressed depending on the Jatoba concentration and hardly observed at the concentration of 125 µg/ml or more. These results showed that Jatoba had an activity to suppress Th1 cytokine in vitro. It shows the possible effectiveness in the prevention and treatment of autoimmune diseases caused by excessive cell-mediated immunity.

**Table 4: Concentration dependence of Jatoba activity in suppressing IFN-γ, a Th1 cytokine produced specifically to antigens, in Th1-biased mouse splenocytes is shown.**

| Jatoba (µg/ml) | mIFN-γ (pg/ml) | SD |
|---|---|---|
| 0 | >1500 | - |
| 15.5 | >1500 | - |
| 31 | >1500 | - |
| 62.5 | 1008.9 | 153.5 |
| 125 | 125.3 | 28.9 |
| 250 | 28.9 | 9.3 |
| 500 | 13.6 | 6.4 |

### Example 3: Effect of Jatoba extract in vivo in experimental autoimmune encephalomyelitis (EAE) model - I

In order to elucidate in vivo effects of Jatoba extract, an experimental autoimmune encephalomyelitis (EAE) model in mice were used. The EAE model mice have been used as a model for human multiple sclerosis (Antel JP. , 1999. J. Neuroimmunol . 100, 181-189).

### 1. Experimental methods

### (1) Samples for administration

The samples prepared in Example 2 were used.

### (2) Experimental animals

To 6-8 week-old male C57BL/6 mice, an emulsion of 200 µg of myelin oligodendrocyte glycoprotein peptide (referred to as "MOG peptide" hereinafter, Qiagen) emulsified with 800 µg of CFA was subcutaneously administered, and then on day 1 and on day 3, 200 ng of pertussis toxin (PTX, Calbiochem) was administered intraperitoneally, thereby experimental autoimmune encephalomyelitis was induced. During the experimental period, mice were fed standard solid feed CE-2 (standard composition by US National Institute for Nutrition) . Tap water was used as drinking water.

### (3) Experimental conditions

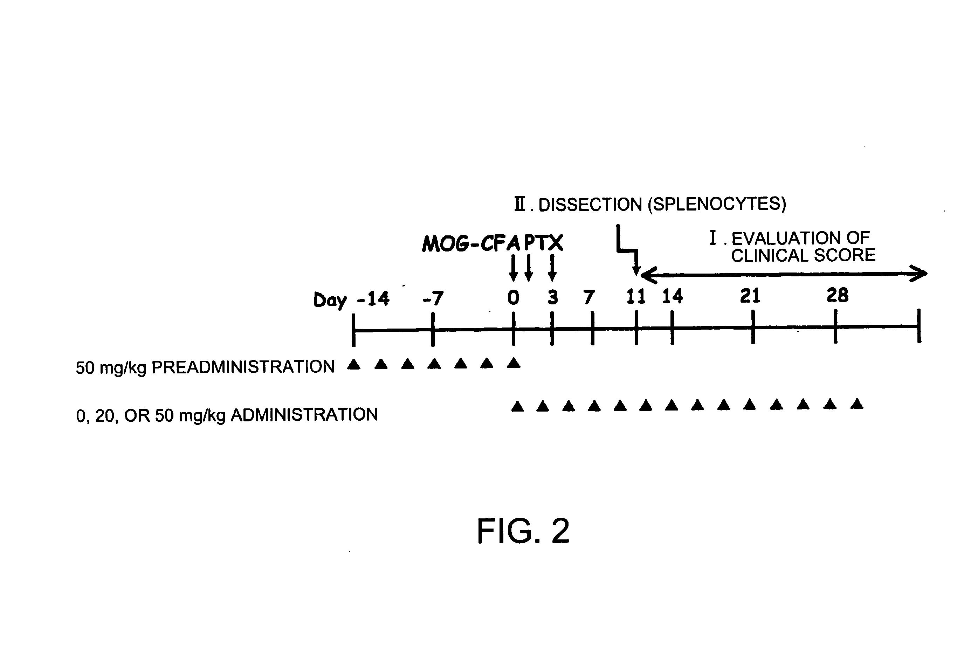

The 6-8 week-old male C57BL/6 mice were divided into groups of 10 animals, i.e., a control group, a 20 mg/kg administration group, a 50 mg/kg administration group, and a 50 mg/kg preadministration group. In the control group, after the administration of MOG antigen on day 0, 1% ethanol was administered intraperitoneally 3 times a week up to day 30. In the 20 mg/kg administration group and the 50 mg/kg administration group, after the administration of MOG antigen on day 0, the specified amounts of Jatoba were administered 3 times a week up to day 30. In a 50 mg/kg preadministration group, Jatoba was administered at 50 mg/kg 7 times for 2 weeks before the administration of MOG antigen and was replaced by 1% ethanol after the antigen sensitization. The experimental schedule is shown in Fig. 2.

### (4) How to record clinical scores

Clinical scores were recorded daily for individual animals up to 55 days as follows. Score 0: normal; score 1: tail paralysis; Score 2: impaired righting; score 3: single hind limb paralysis; score 4: dual hind limb paralysis; score 5: fore and hind limb paralysis; and score 6: death.

### (5) Measurement of ex vivo IFN-γ and TNF-α productivity

On day 12, 5 animals in each group were dissected to prepare splenocytes. The splenocytes were cultured in an RPMI1640 (Sigma) + 10% FCS (Roche) ± 2 µM MOG medium. The IFN-γ concentration in the supernatant was measured after one week of culture and the TNF-α concentration in the supernatant was measured after 10 days of culture. The IFN-γ and TNF-α concentrations were measured using an OptEIAELISA Set (Becton Dickson).

### (6) Preparation of pathological section of spinal cord and observation of demyelination

To study whether demyelination of the spinal cord is suppressed by Jatoba, tissue sections were prepared and observed. On day 15, the spinal cord was sectioned from mice in the control group and the Jatoba administration group and fixed in a 2% formalin solution, after which it was embedded in paraffin to prepare a 5 µm section using the Microtome RM2145 (Leica). Then each section was deparaffined, subjected to myelin sheath staining with the Luxol fast blue solution (Muto Kagaku Yakuhin) and washed, after which the cytoplasm was stained with cold-Schiff's reagent. Finally the nuclei were stained with hematoxylin and microscopic observation was performed.

### 2. Experimental results

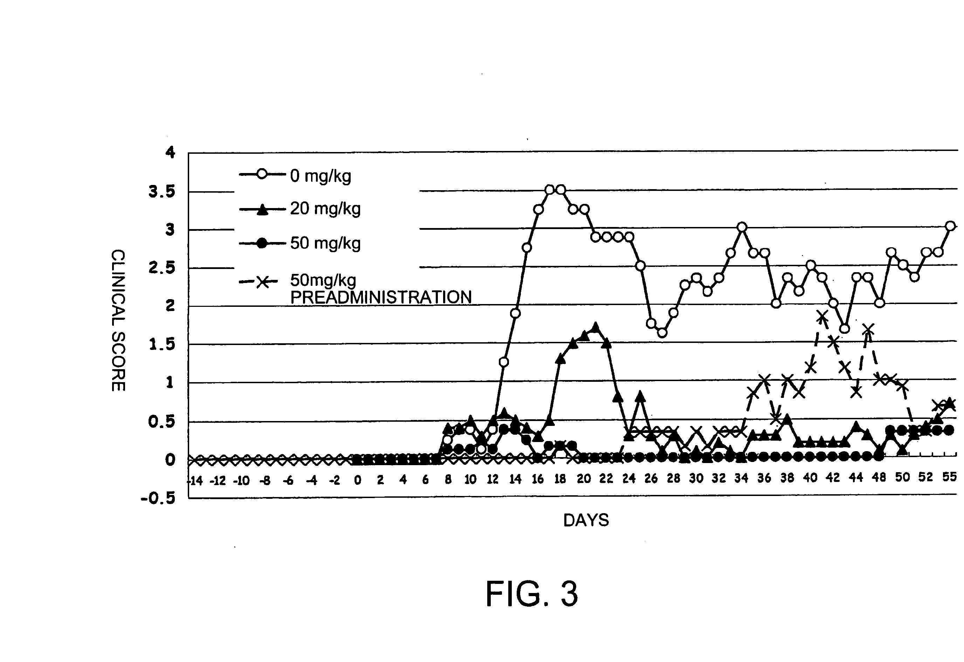

Fig. 3 is a graph showing the change in average clinical scores with time. In the control group, the onset started on day 9 and the average score exceeded 3.5 on day 16. In the 20 mg/kg administration group, the onset started on day 15 or so and the average score reached the peak, 1.5, on day 21 or so but the symptoms were recovered thereafter. In the 50 mg/kg administration group, there was no onset. In the 50 mg/kg preadministration group, the onset started on day 30 or so and the average score reached the peak, about 2, on day 41 but the symptoms were recovered thereafter. These results revealed that Jatoba exhibited a distinct effect in suppressing EAE onset. Further, its preventive effect was also revealed in delaying the onset for 3 weeks when administered before the EAE induction.

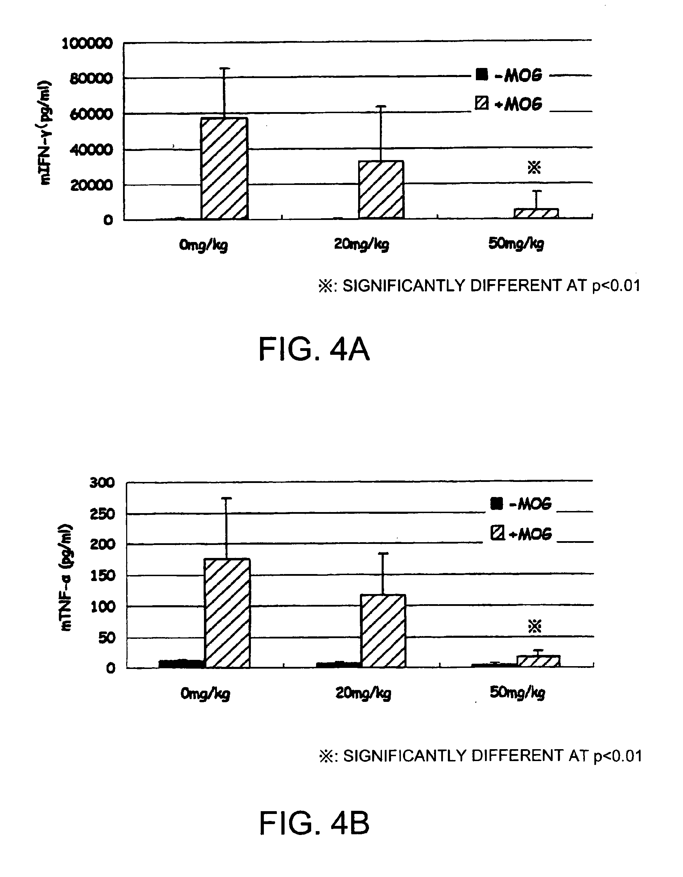

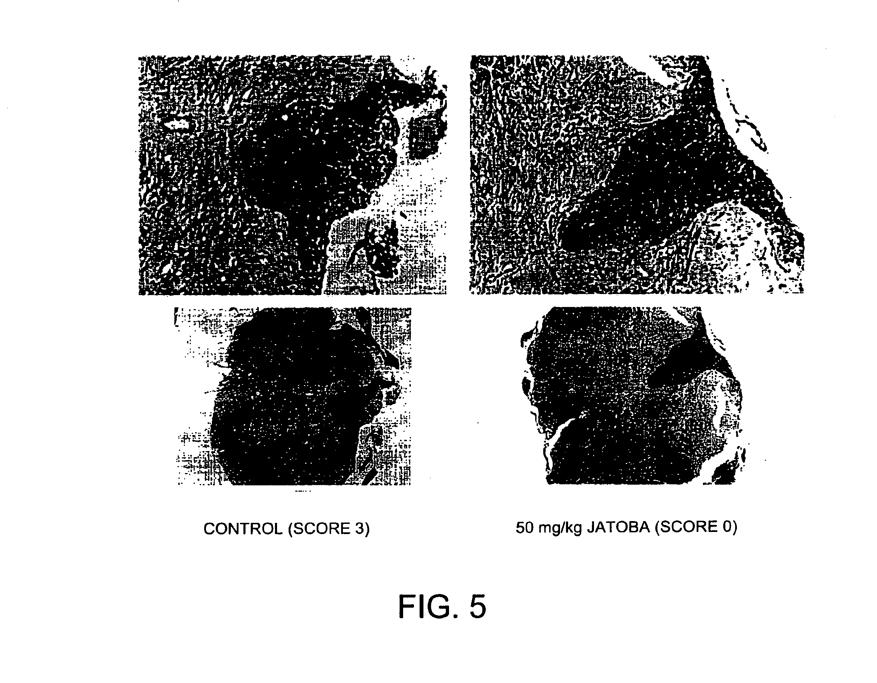

The amount of ex vivo cytokine production is shown in Fig. 4. As to IFN-γ, the production was as high as 60,000 pg/ml in the control group, whereas it was reduced by half to about 30,000 pg/ml in the 20 mg/kg administration group and was drastically reduced to about 5000 pg/ml in the 50 mg/kg administration group. Also as to TNF-α, production was about 180 pg/ml in the control group, whereas it was reduced to about 110 pg/ml in the 20 mg/kg administration group and was drastically reduced to about 20 pg/ml in the 50 mg/kg administration group. Further, the result of the observation of the spinal cord sections with Luxol fast blue staining is shown in Fig. 5. Photographs of spinal cord sections revealed that a spot without blue stain due to demyelination (on the right side of the tissue in the photograph of the section) was observed in the control group, whereas such demyelination was distinctly suppressed in the Jatoba administration groups. These results showed that Jatoba strongly suppressed the Th1 cytokine production and suppressed the symptoms of EAE.

### Example 4: In vivo effect of Jatoba extract in EAE model - II

In order to show that the effect in Example 3 did not depend on the species of mice, a study was carried out using an experimental autoimmune encephalomyelitis (EAE) model in mice which are different in species from those used in Example 3.

### 1. Experimental methods

### (1) Samples for administration

The samples prepared in Example 2 were used.

### (2) Experimental animals

To 8-10 week-old male SJL/Jmice (purchased from Charles River), an emulsion of 100 µg of proteolipid protein peptide (referred to as "PLP peptide" hereinafter; Qiagen) emulsified with 800 µg of CFA was subcutaneously administered, and on day 1 and on day 3, 200 ng of PTX was administered intraperitoneally, thereby experimental autoimmune encephalomyelitis was induced. Feeds and drinking water used were the same as in Example 3.

### (3) Experimental conditions

The 8-10 week-old male SJL/J mice were divided into groups of 10 animals, i.e., a control group and a 50 mg/kg administration group. Theme thod and time of the administration of the antigen and Jatoba were the same as described in Example 3.

### (4) Measurement of ex vivo IFN-γ and TNF-α productivity

The measurement was performed in the same manner as described in Example 3, except that the antigen added to the culture medium was 2 µM MPLP peptide.

### 2. Experimental results

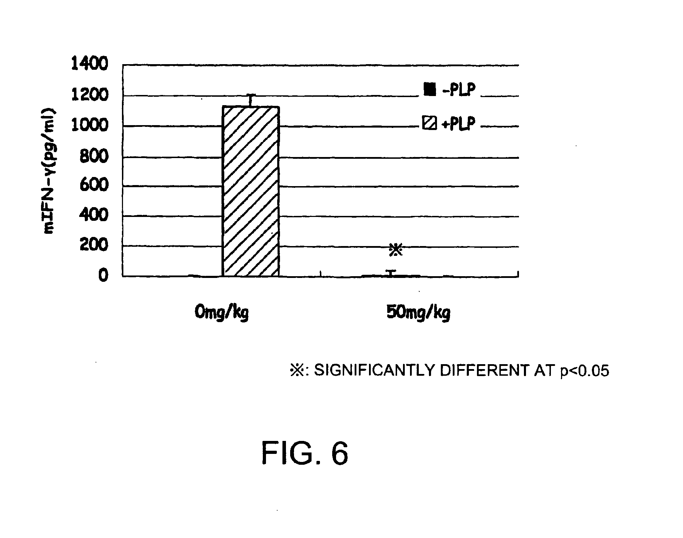

In SJL/J mice, the species different from those in Example 3, the ex vivo IFN-γ production was almost entirely suppressed by Jatoba administration as shown in Fig. 6, which revealed that the EAE suppressing effect of Jatoba was independent on the species of the mice.

### Example 5: In vivo effect of single preadministration of Jatoba extract in EAE model

The study was performed on prevention of EAE onset by single preadministration of Jatoba.

### 1. Experimental methods

### (1) Samples for administration

The Jatoba ethanol extract of Example 2 was used.

### (2) Experimental animals

EAE was induced by immunizing C57BL-6 mice with MOG antigen as described in Example 3. Each group consisted of 10 mice and the half of the animals were dissected on day 12.

### (3) Experimental conditions

On day -1 that was the day before the administration of MOG antigen on day 0, a single dose of 50 mg/kg of Jatoba was administered intraperitoneally.

### (4) Measurement of ex vivo IFN-γ productivity

On day 12, 5 animals were dissected to prepare splenocytes. The splenocytes were cultured in an RPMI1640 (Sigma) + 10% FCS (Roche) ± 2 µM MOG medium. The IFN-γ concentration in the supernatant was measured after one week of culture. The IFN-γ concentration was measured using an OptEIA ELISA Set (Becton Dickson).

### 2. Experimental results

The change with time of average EAE clinical scores showed that the onset was delayed for 2 days in the single preadministration group, whereas it started on day 9 in the control group (Fig. 7A) . This result showed that even the single administration was effective although it was not as effective as the multiple administrations as described in Example 3. The results of the present Example and the abovementioned Example 3 suggested that the preventive effect increased with an increase in the number of doses. The IFN-γ productivity of splenocytes was shown to markedly decrease even by a single prophylactic administration (Fig. 7B).

### Example 6: Study on the composition of Jatoba extract (I)

In order to elucidate the characteristics of the active ingredient of Jatoba extract, the Jatoba extract was treated with polyvinylpyrrolidone (PVPP) for adsorption and the activityof the resultant unadsorbed fraction was studied using an experimental autoimmune encephalomyelitis (EAE) model.

### 1. Experimental methods

### (1) Samples for administration

The Jatoba ethanol extract (3.49 g) of Example 2 was dissolved in 500 ml of water and 50 mg of polyvinylpyrrolidone (PVPP; Sigma) was added. The admixture was stirred overnight and then subjected to aspiration-filtration and lyophilization to obtain 420.3 mg of a PVPP-treated product, i.e., a PVPP-unadsorbed faction.

### (2) Experimental animals

EAE was induced by immunizing C57BL-6 mice with MOG antigen as described in Example 3. Each group consisted of 10 mice and the half of the animals were dissected on day 12.

### (3) Experimental conditions

In the control group, 1% ethanol was intraperitoneally injected 3 times a week up to day 30. The Jatoba ethanol extract was administered in the Jatoba administration group and the PVPP-treated Jatoba ethanol extract was administered in the PVPP-treated Jatoba administration group, 3 times a week at 50 mg/kg, individually.

### (4)Measurement of ex vivo IFN-γ and TNF-α productivity

On day 12, 5 animals of each group were dissected to prepare splenocytes. The splenocytes were cultured in an RPMI1640 (Sigma) + 10% FCS (Roche) ± 2 µM MOG medium. The supernatant after one week of culture was measured for the IFN-γ concentration and the supernatant after 10 days of culture was measured for the TNF-α, concentration. IFN-γ and TNF-α concentrations were measured using an OptEIA ELISA Set (Becton Dickson).

### 2. Experimental results

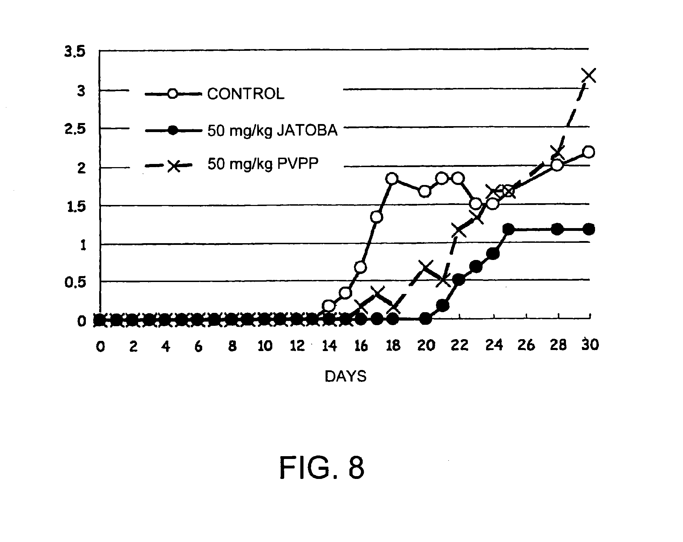

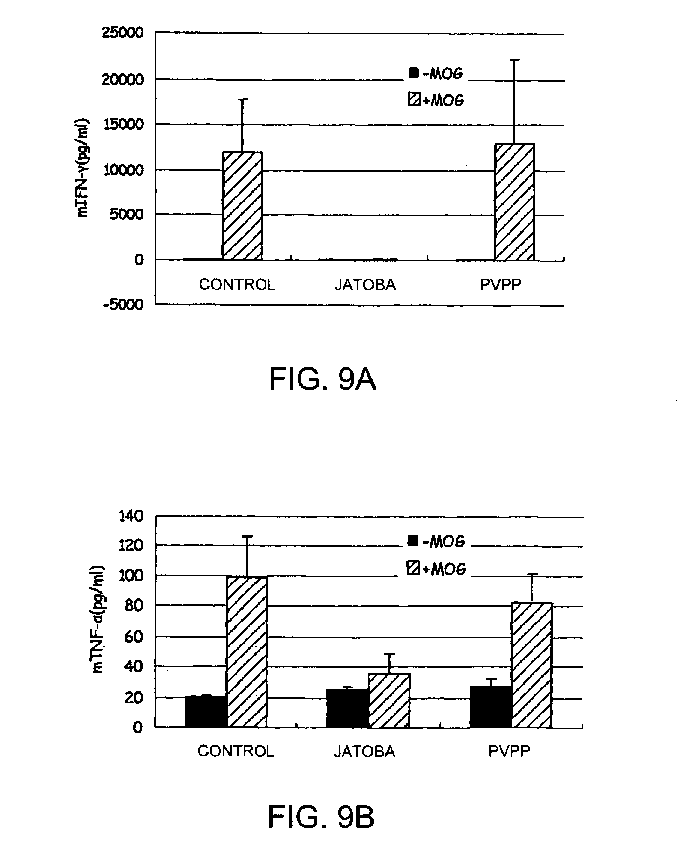

The results are shown in Fig. 8. The onset started on day 13 in the control group, whereas it did not start before day 21 in the Jatoba administration group. The onset was observed on day 15 in the PVPP-treated Jatoba administration group. The IFN-γ and TNF-α, productivity of splenocytes was markedly suppressed in the Jatoba administration group but it was almost the same in the PVPP-treated Jatoba administration group as in the control group (Fig. 9). These results revealed that the activity of Jatoba was markedly weakened by the PVPP treatment and thus the active ingredient of Jatoba was adsorbed to PVPP. The PVPP has a property of strongly adsorbing polyphenols and has been known to be a polyphenol removing agent (Loomis, W.D., Battaile, J. 1966. Phytochem. 5, 423-438; Loomis, W.D., 1969. In: Lowenstein, J.M., (Ed.), Methods in Enzymology Vol. 13. Academic Press, New York, pp. 555-563; Loomis, W.D., 1974. In" Fleischer, S. , Packer, L., (Eds .), Methods in Enzymology Vol. 31. Academic Press, New York, pp. 528-544) . Since 88% of the Jatoba ethanol extract was adsorbed to PVPP, it was suggested that possibly 88% of the Jatoba extract is polyphenols and its active component is also polyphenols.

### Example 7: Study on composition of Jatoba extract (II)

Example 6 suggested that the active component of Jatoba may possibly be polyphenols. In order to further study the active components, the following experiment was carried out with the Jatoba ethanol extract.

### 1. Experimental methods

### (1) Butanol-HCl Method

The Jatoba ethanol extract was subjected to the butanol-HCl method according to the method in the literature (J. Agric. Food Chem. 1998, 46, 1698-1705) . Namely, a 4 mg/ml solution of the Jatoba ethanol extract in methanol was prepared and to a 250 µl portion of this solution were added 1.5 ml of 1-butanol containing 5% HCl and 50 µl of 2N HCl containing 2% ammonium sulfate, after which the admixture was reacted at 95°C for 50 minutes. The reaction solution was then cooled to room temperature and measured for the absorbance at 550 nm. A 4 mg/ml Jatoba ethanol extract solution (methanol solution) was used as a control for the measurement.

### (2) Normal bonded-phase column chromatography (NP-HPLC)

The Jatoba ethanol extract was subjected to NP-HPLC according to the method in the literature (Journal of Chromatography A. 1993. 255-260). The pump and control apparatus used was Shimadzu LC-10Advp, and the UV detector used was Shimadzu SPD-10A. The chromatographic conditions were as follows.
Column: Finepac SIL-5 φ4.6 × 250 mm (Nippon Bunko)
Mobile Phase:
(A) MeOH:CH₂Cl₂:HCOOH:H₂O = 7:41:1:13
(B) MeOH:CH₂Cl₂:HCOOH:H₂O = 43:7:1:1

**Conditions for mobile phase gradient:**

| Time (min) | Solvent A (%) | Solvent B (%) |
|---|---|---|
| 0 | 95 | 5 |
| 10 | 85 | 15 |
| 35 | 80 | 20 |
| 37 | 77 | 23 |
| 40 | 77 | 23 |
| 42 | 70 | 30 |
| 45 | 50 | 50 |
| 46 | 0 | 100 |
| 50 | 0 | 100 |

Mobile phase flow rate: 1 ml/min
Detection wavelength: 280 nm

### (3)Electrospray ionization mass spectrometry (ESI-MS)

The Jatoba ethanol extract was subjected to ESI-MS. The apparatus used was LCQ by Thermoquest. The measurement was by the infusion method in the negative ion mode, and the conditions were flow rate: 6 µl/min; sheath gas flow rate: 60 arb; sprayvoltage: 4.5 kV; and capillary temperature: 200°C.

### (4) Matrix-assisted laser desorption/ionization time-of-flight mass spectrometry (MALDI-TOF-MS)

The Jatoba ethanol extract was subjected to MALDI-TOF-MS. The apparatus used was PerSeptive Biosystems Voyager and 2,5-dihydroxybenzoic acid (DHB) was used as a matrix.

### 2. Experimental results

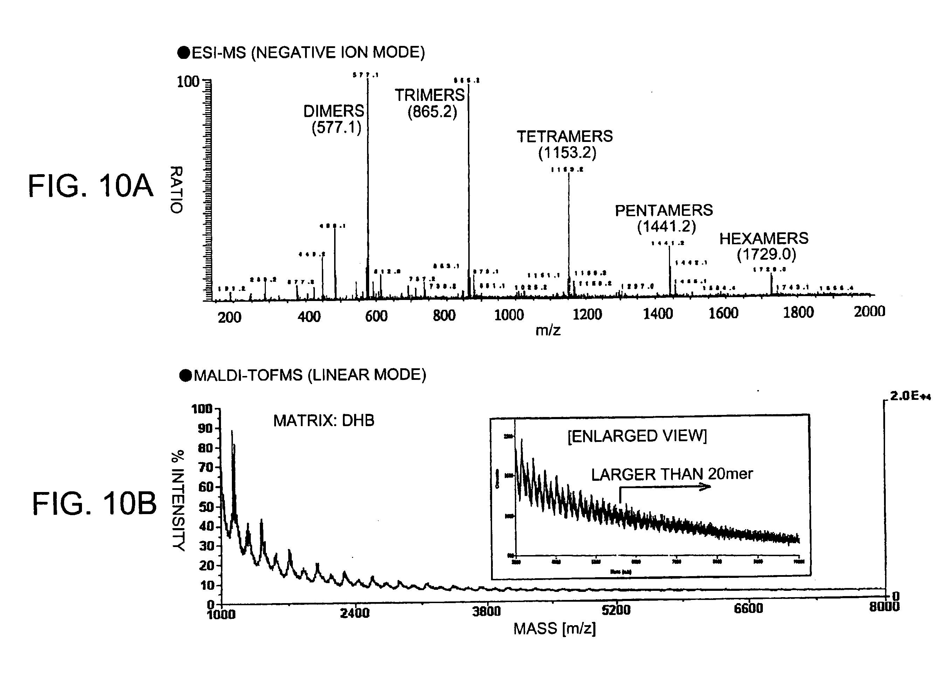

(1) When the interflavanoid linkages are cleaved by heat treatment under acidic conditions, proanthocyanidins yield carbenium ions derived from extension units and catechins derived from terminal units. The former is further oxidized into red anthocyanidin. Colorimetry utilizing this principle is the Butanol-HCl method which sensitively detects proanthocyanidins in a sample by detecting an increase in the absorbance at 550 nm. Using this method, the Jatoba ethanol extract was reddened and its absorbance at 550 nm was 2.72. Thus, it is revealed that the polyphenols contained in Jatoba are proanthocyanidins.
(2) NP-HPLC can detect oligomer components of proanthocyanidins as elution peaks. However, since components with a higher polymerization degree are strongly adsorbed to the column, a characteristic broad shape chromatogram appears at the late stage of the elution. The chromatogram of the Jatoba ethanol extract showed eight elution peaks followed by the characteristic broad shape, suggesting the presence of components with a higher degree of polymerization as well as proanthocyanidin oligomers.
(3) In ESI-MS, five molecular ion peaks with the molecular weight increased by 288 were observed as shown in Fig. 10, although components with a molecular weight of only up to 2000 were observable because of the measurement limit of the apparatus. Namely, the proanthocyanidins of Jatoba have a repetitive structure with a molecular weight of 288. This molecular weight corresponds to thatof catechin or epicatechin and shows there is no gallate adduct. These results reveal that a major component of the Jatoba ethanol extract is one of proanthocyanidins, procyanidin.
(4) MALDI-TOP-MS confirmed the presence of procyanidins with a high degree of polymerization which belong to higher polymer region and are not observable in ESI-MS. Namely, as shown in Fig. 10B, the molecular ion peaks for 20-mer or greater procyanidins were observed.

### Example 8: Study on composition of Jatoba extract (III)

Example 7 showed that the major component of the Jatoba ethanol extract is procyanidins. In order to precisely perform identification of constitutive units and mode of linkages, calculation of the polymerization degree, and quantitative analysis, the thiolysis analysis was carried out according to the method of Guyot et al. (Sylvain Guyot, Nathalie Marnet, and Jean-Francois Drilleau, J. Agric. Food Chem. 2001, 49, 14-20).

### 1. Experiment methods

### (1) Thiolysis and reverse phase liquid chromatography (RP-HPLC) analysis

A 4 mg/ml solution of the Jatoba ethanol extract in methanol was prepared and to a 25 µl portion of the solution were added 25 µl of methanol containing 3.3% HCl and 50 µl of 5% toluene-α-thiol (Sigma), after which the admixture was reacted at 40°C for 30 minutes. The reaction solution was then subjected to RP-HPLC analysis as it was. The pump used was Hitachi L-7100, and the UV detector used was Shimadzu SPD-10A. The chromatographic conditions were as follows.
Column: Discovery C18 φ4.6 × 250 mm (Sperco)
Mobile Phase: (A) 2.5% acetic acid/water (v/v), (B) acetonitrile

**Conditions for mobile phase gradient:**

| Time (min) | Solvent A (%) | Solvent B (%) |
|---|---|---|
| 0 | 97 | 3 |
| 5 | 91 | 9 |
| 15 | 84 | 16 |
| 45 | 50 | 50 |
| 55 | 0 | 100 |

Mobile phase flow rate: 0.6 ml/min
Detection wavelength: 280 nm

### (2) Identification of reaction products by liquid chromatography/mass spectrometry (LC-MS)

Since toluene-α-thiol remains in the thiolysis reaction solution, the reaction solution cannot be submitted to LC-MS as it is. Therefore, individual peaks were first fractionated by RP-HPLC in (1) and then submitted to LC-MS. The pump in the LC part used was Waters 2690 alliance and the detection was carried out using a photo-diode array detector (Waters 996). The chromatographic conditions were as follows.
Column: YMC Pack C18 φ4.6 × 250 mm
Mobile Phase: (A) 1% HCOOH/water (v/v), (B) MeOH

**Conditions for mobile phase gradient:**

| Time (min) | Solvent A (%) | Solvent B (%) |
|---|---|---|
| 0 | 90 | 10 |
| 5 | 84 | 16 |
| 45 | 50 | 50 |
| 50 | 0 | 100 |

Mobile phase flow rate: 1 ml/min
Detection wavelength: 200-400 nm

The ionization mode of MS was ESI and the apparatus used was Waters Micromass ZQ2000.

### (3) Correction of molar absorbance coefficient (ε) associated with toluene-α-thiol addition

The change in ε values at 280 nm associated with addition of toluene-α-thiol to epicatechin was calculated using procyanidin B2 which is an epicatechin dimer. Namely, procyanidin B2 was subjected to thiolysis and then to RP-HPLC analysis as in (1).

### (4) Calculation of average degree of polymerization and quantitative analysis

Based on the results in (1), (2), and (3), the average degree of polymerization of proanthocyanidins and their content in the Jatoba ethanol extract were calculated.

### 2. Experiment results

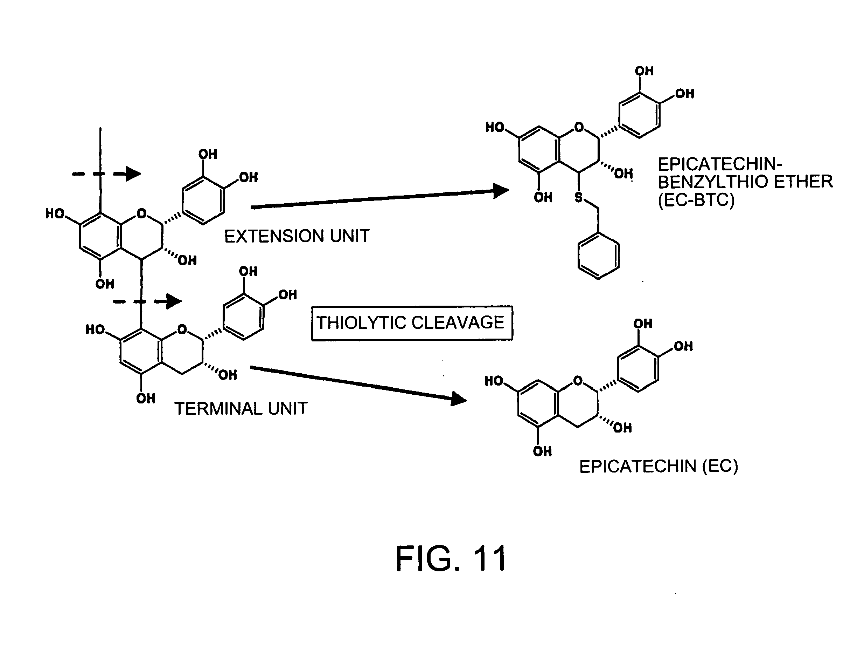

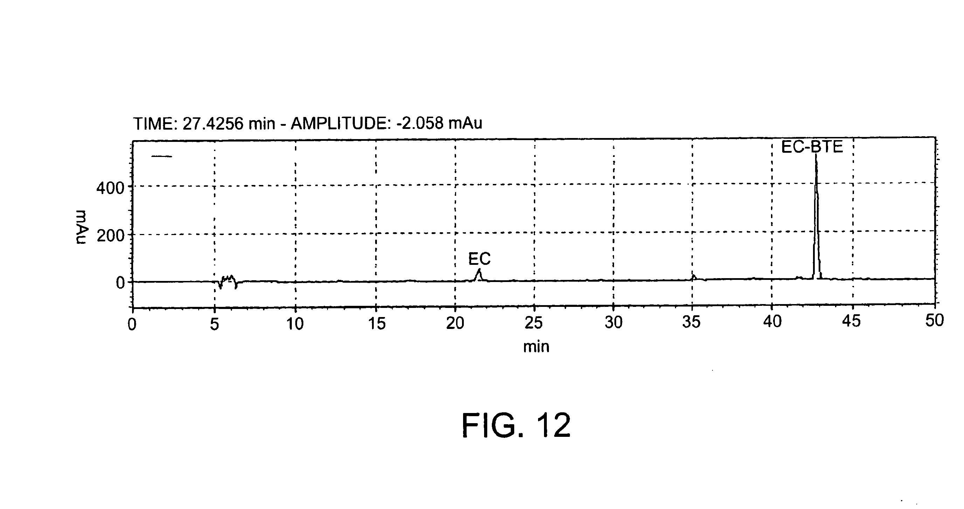

(1) Since proanthocyanidins yield a monomer component derived from terminal units and a benzyl thioether adduct derived from extension units by thiolysis as shown in Fig. 11, theseproducts can be isolated, identified and quantified by RP-HPLC. As a result of thiolysis of the Jatoba ethanol extract, two peaks were recognized at 21.6 min and 42.8 min as shown in Fig. 12. The former was eluted at the position comparable to that of epicatechin (EC). No peaks other than these two appeared.
   A-type linkage cannotbe cleaved by thiolysis and remains intact. Accordingly, when A-type linkages are present, benzyl thioether adduct dimers linked by A-type linkage are produced by thiolysis and detected by RP-HPLC. The result of thiolysis analysis revealed that proanthocyanidins of Jatoba have no A-type linkage and are comprised of B-type linkages only.
(2) The peaks detected in (1) wereidentifiedbyLC-MS. Namely, the LC elution positions and the molecular weight of these two peaks agreed with those of EC, which is a thiolysis product of procyanidin B2, and EC-benzyl thioether adduct (EC-BTE), respectively. The results of (1) and (2) revealed that EC is the only constitutive units of Jatoba procyanidin.
(3) When procyanidin B2 is subjected to thiolysis, EC derived from terminal units and EC-BTE derived from extension units are produced in an equimolar ratio. The absorbance at 280 nm or peak areas of the two components detected by RP-HPLC were compared to calculate the change in ε values at 280 nm associated with addition of toluene-α-thiol to EC. The result showed ε for EC:ε for EC-BTE = 1:1.2.
(4) Based on the result of (3), the average degree of polymerization of procyanidins contained in the Jatoba ethanol extract was calculated as follows.
   Average degree of polymerization = (T+E/1.2)/T
   T = peak area of terminal unit
   E = peak area of extension unit, toluene-α-thiol adduct

As a result, the average degree of polymerization of procyanidins contained in the Jatoba ethanol extract was calculated to be 6.8.

The procyanidin content of the Jatoba ethanol extract can be obtained by quantitatively analyzing the thiolysis reactionproducts, EC andEC-BTE . The thiolysisyieldnecessary for the quantitative analysis was obtained by quantifying the produced EC using procyanidin B2 as an external standard. The reaction yield was generally 83%. As a result, the procyanidin content of the Jatoba ethanol extract was calculated to be 76.0% (w/w).

### Example 9: Fractionation and analysis of Jatoba procyanidins Experimental methods and results

### (1) Extraction and fractionation

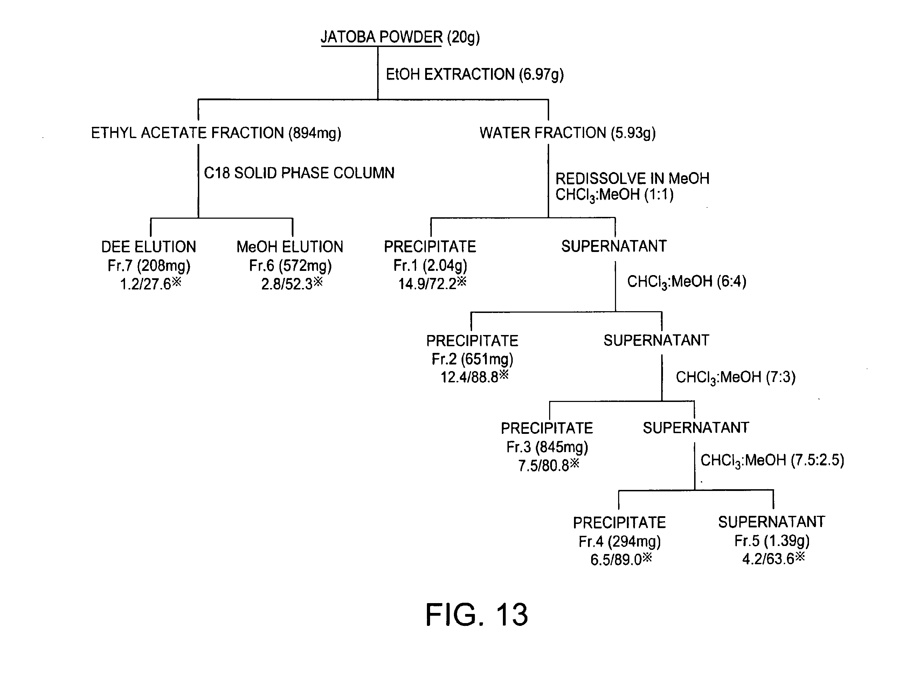

To 20 g of commercial Jatoba dried powder was added 500 ml ethanol (EtOH), and the admixture was stirred at room temperature overnight. After filtration, the resulting residue was re-extracted with an equal volume of EtOH. The filtrate was concentrated to dryness to obtain 6.97 g of extract. This extract was redissolved in 100 ml of water and extracted 3 times with an equal volume of ethyl acetate. The solvent was removed by evaporation to obtain 5.93 g of water fraction and 894 mg of ethyl acetate fraction. The water fraction was redissolvedinmethanol (MeOH) and chloroform (CHCl₃) wasadded successively based on the method of Saucier et al. (J. Agric. Food Chem. 2001. 49, 5732-5735) to fractionate the resulting precipitates. By this method, procyanidins can be fractionated by different degrees of polymerization. The ethyl acetate fraction was subjected to solid-phase extraction by a C18 column and further fractionated into two fractions. Namely, Fr. 7 was first obtained by the elution with diethyl ether (DEE), then Fr. 6 was obtained by the elution with MeOH. The weights of the individual fractions were shown in Fig. 13.

### (2) Analysis of Jatoba extract and individual fractions

The average degree of polymerization of procyanidins and their content for each of the fractions obtained were calculated by thiolysis analysis (see Example 8 for the method). As shown in Fig. 13, the procyanidin fractions with different average degrees of polymerization could be easily prepared by this method.

### Example 10: IFN-γ suppressing activity of fraction samples derived from Jatoba using EAE model

Each of the samples of the fractions obtained in Example 9 was intraperitoneally administered to EAE model mice to study IFN-γ suppressing activity of splenocytes.

### 1. Experimental methods

### (1) Samples for administration

The Jatoba ethanol extract of Example 2 and Fr1 to Fr7 fractionated in Example 9 were used.

### (2) Experimental conditions

Male C57BL/6 mice (6-week-old) were divided into groups of 3 animals, i.e., a control group, a Jatoba (unfractionated) administration group, and Fr1 to Fr7 administration groups. In the control group, after the administration of MOG antigen on day 0, 1% ethanol was administered intraperitoneally altogether 5 times, on days 0, 2, 4, 7, and 9. In the Jatoba administration group, 50 mg/kg of the Jatoba extract was administered in the same schedule. Each fractions were administered at 50 mg/kg in the same schedule. The animals were dissected on day 11 and the resulting splenocytes were co-cultured with MOG and then the amount of IFN-γ produced in the culture supernatant was measured by ELISA.

### 2. Experimental results

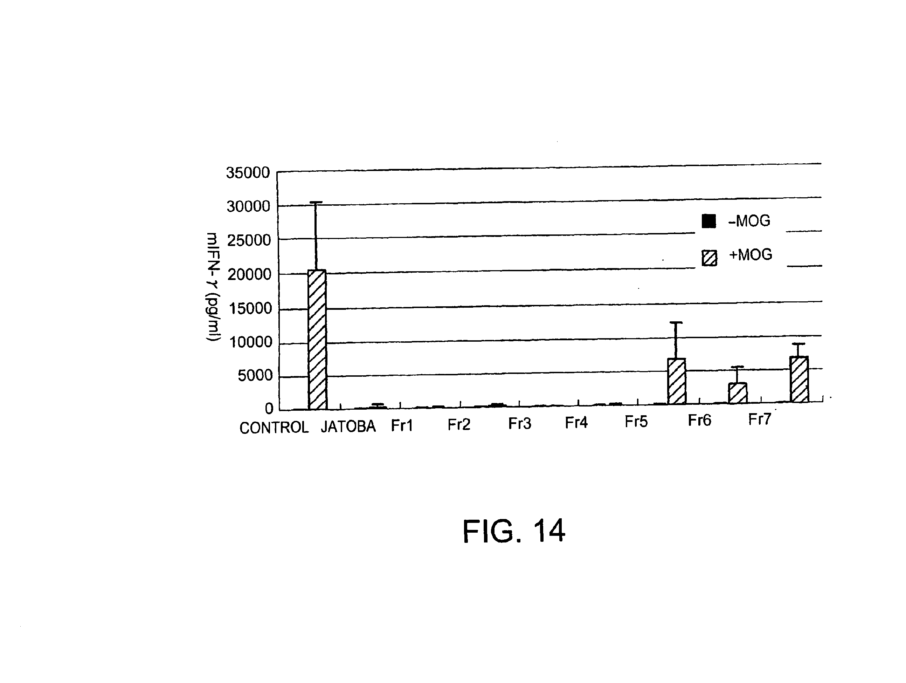

The results are shown in Fig. 14. In the control group, about 20000 pg/ml of IFN-γ production was observed but in the Jatoba administration group, the IFN-γ production was hardly observed. In the Fr5 to Fr7 administration groups, some IFN-γ production was observed as compared to that in the control group. In the Fr1 to Fr4 administration groups, the IFN-γ production was hardly observed. These results suggested that the activity to suppress IFN-γ production that is a malignant factor of autoimmune diseases was markedly high when the average degree of polymerization was 6.5 or more and increased with an increase in the polymerization degree.

### Example 11: Study on composition of Jatoba ethanol extract (IV)

In Example 8, the thiolysis analysis was carried out in order to more precisely perform identification of constitutive units, determination of the mode of linkage, calculation of the polymerization degree, and quantitative analysis for procyanidins contained in Jatoba. In order to more precisely perform analysis of the average degree of polymerization and quantification of procyanidins, a toluene-α-thiol adduct derivative of epicatechin (EC)(EC-BTE) was purified, isolated and subjected to high resolution mass spectrometry (HR-MS) and nuclear magnetic resonance spectroscopy (NMR) for structure identification, after which HPLC analysis was carried out using the resulting product as a standard. Use of the purified and isolatedproduct as a standard made it possible to precisely perform identification of constitutive units, determination of the mode of linkage, calculation of the polymerization degree, and quantitative analysis of procyanidins.

### 1. Experimental methods

### (1) Isolation and purification of EC-BTE

The Fr1 prepared in Example 9 was subjected to thiolysis as described in Example 8 and then the reaction product was submitted to HP-LC to fractionate an EC-BTE peak. The chromatographic conditions were as follows.
Column: Cadenza CD-C18 C18 φ10 x 250 mm (Intact)
Mobile phase: methanol:water:formic acid (50:49.8:0.2, v/v/v)
Mobile phase flow rate: 2 ml/min
Detection wavelength: 280 nm

### (2) Identification of the structure of EC-BTE

EC-BTE obtained in the section above was first subjected to HR-MS to obtain a precise molecular weight. The apparatus used was JEOL JMS-700 (Nippon Denshi) and the ionization mode was fast atom bombardment (FAB) with an ionization voltage of 70 eV. Further, proton NMR spectra were measured to compare the signals with literature data. The apparatus used was UNITY INOVA-500 (500 MHz; Varian).

### (3) HPLC analysis of procyanidins

HPLC analysis was carried out as described in Example 8 using BC-BTE thus obtained as a standard to calculate the average degree of polymerization of procyanidins and their content. The average degree of polymerization was calculated as follows.
Average degree of polymerization = (T+E)/T
T = mole of terminal unit (EC)
E = mole of extension unit (EC-BTE)

The content is percentage by weight of the amount of procyanidins in the sample on the basis of an amount of EC.

### 2. Experimental results

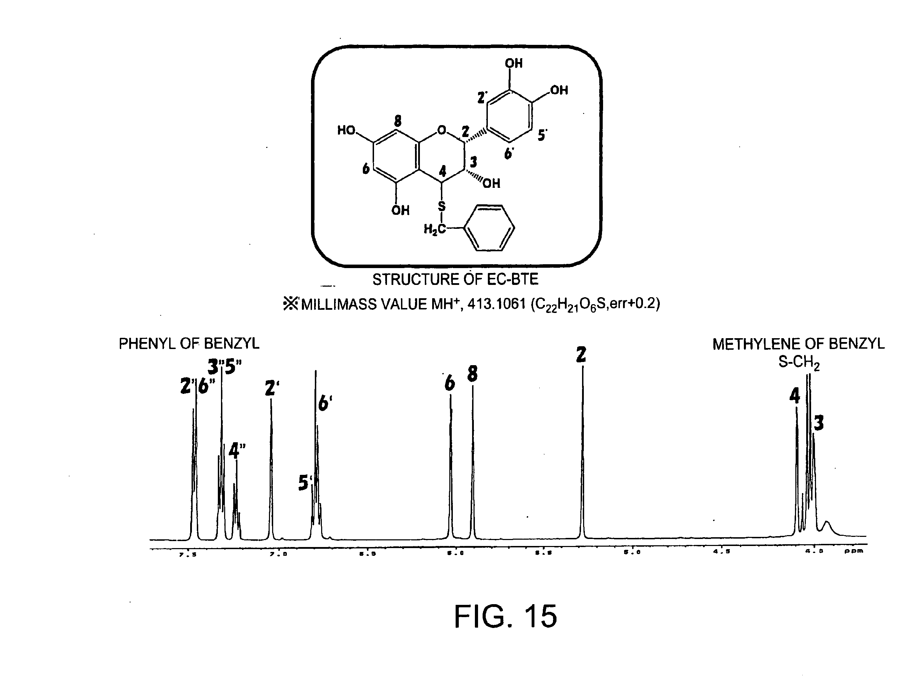

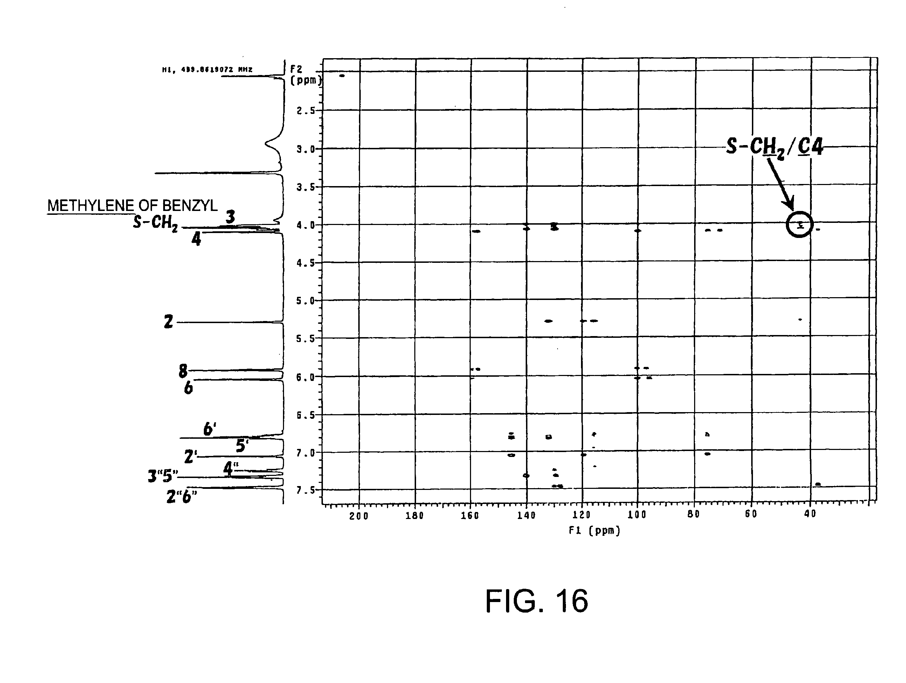

(1) From 66 mg of Fr1, 10.8 mg of EC-BTE was obtained.
(2) As a result of HR-MS, an ion peak of 413.1061 as MH⁺ was observed and its molecular formula was identified as C₂₂H₂₁O₆S, which corresponds to EC-BTE. Further, the proton NMR spectrum (Fig. 15) agreed with literature data (Chem. Pharm. Bull., 40, 889-898, 1992). Further, since a long range correlation signal from a proton on the SCH₂ site of benzylthiol to a carbon at position 4 on the flavan ring was observed in ¹H-detected multiple-bond heteronuclear multiple quantum coherence (HMBC) spectrum (Fig. 16), it was verified that benzylthiol was added at position 4 on the flavan ring. From the results above, the EC-BTE structure was completely identified.

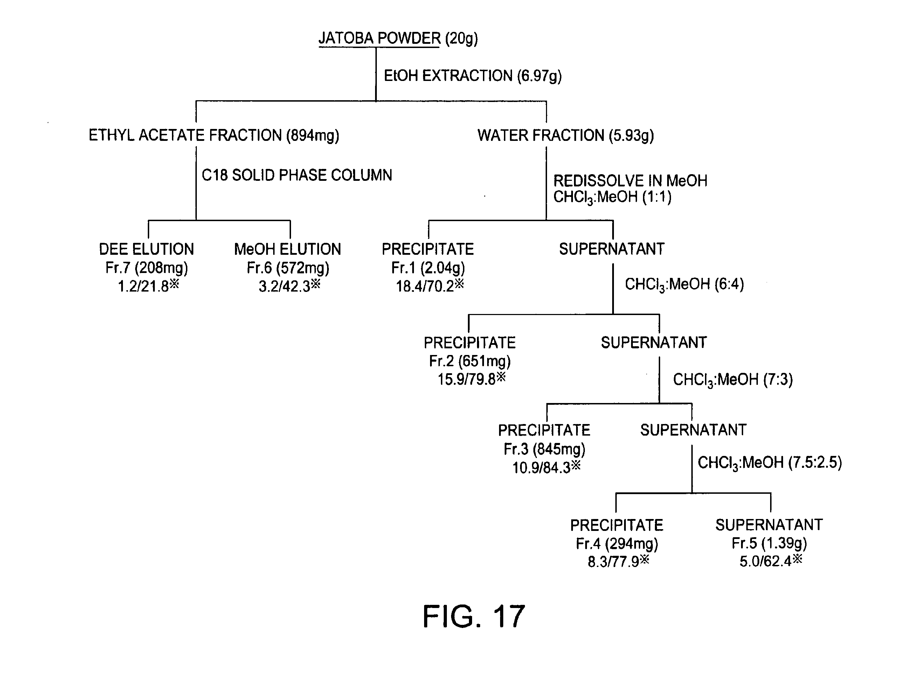

(3) Fig. 17 shows the average degree of polymerization of procyanidins and their content obtained as a result of HPLC analysis using EC-BTE as a standard.

### Example 12: Preparation and analysis of procyanidin-containing samples (I)

Since Example 10 revealed that the substantial activity of Jatoba is attributed to procyanidins with a polymerization degree higher than a certain degree, other materials containing procyanidins were studied.

### 1. Samples

Samples used were commercial materials including Gravinol SL (Kikkoman Corporation), Polyphenon (Tokyo Techno Food) Applephenon (Nikka Whisky), Cranberry Powder (Kikkoman Corporation), Cacao Polyphenol (Meiji Seika), Pine Bark Extract (Material Function Laboratories), and Cinnamon Powder (SB Food).

### 2. Experimental methods

### (1) Preparation of cinnamon sample

Cinnamon powder (30.5 g) was extracted with 300 ml of ethanol and the resulting ethanol extract was concentrated to dryness, after which it was redissolved in 50% methanol/water and extracted 4 times with an equal amount of chloroform. After removing the solvent by distillation, the aqueous methanol fraction was extracted with 1% ethanol/water .

### (2) Preparation of cranberry sample

A sample containing procyanidins with a high degree of polymerization was prepared from Cranberry Powder. Namely, 9 g of Cranberry Powder was dissolved in 600 ml of water, and the solution was applied on a column (φ2.6 cm x 72 cm) filled with Sephadex LH-20 resin. After eluting with 1 L each of water and methanol at a flow rate of 5 ml/minute, the elution was completed with 1 L of 70% acetone/water. This column chromatography was repeated 3 times.

### (3) Procyanidin analysis

Procyanidins contained in individual samples, the cinnamon sample prepared in (1) and the cranberry sample prepared in (2) were quantified and their average polymerization degree, constitutive units and linkage mode were determined, by thiolysis analysis as in Example 8. Further, since some samples have gallate addition to the hydroxyl group on position 3 of the flavan ring, the caused change in the molar absorbance coefficient (ε) at 280 nm was calculated using epicatechin (EC) and epicatechin gallate (ECG).

### 3. Experimental results

(1) 3.91 g of cinnamon ethanol extract was obtained. From 1.4 g of an aqueous methanol fraction after partitioning, 635 mg of a 1% ethanol/water soluble fraction was obtained, which was used as a cinnamon sample.
(2) 20.8 g of a water fraction, 303 mg of a methanol fraction, and 217 mg of an aqueous acetone fraction were obtained from 27 g of Cranberry Powder. By thiolysis analysis, the aqueous acetone elution fraction was used as a highly polymerized procyanidin fraction.
(3) Results of analysis ofprocyanidins contained in individual samples are shown in Table 5. See Example 8 for Jatoba. Peaks for gallate adducts and their modified derivatives were identified by liquid chromatography-mass spectrometry (LC-MS) in the same.manner as in 1.(2) of Example 8. Since the ε value ratio of EC to ECG at 280 nm was 1:3.1, the peak area was corrected by dividing by 3.1 in quantifying gallate adducts.

Table 5: Procyanidins contained in Jatoba, Gravinol SL, Polyphenon, Applephenon, purified highly polymerized fraction of cranberry, Cacao Polyphenol, pine bark extract, and cinnamon crude extract were analyzed for their average degree of polymerization, content and constitutive units.

| No. | Sample | Average degree of polymerization | content (%) | Constitutive units |
|---|---|---|---|---|
| 1 | Jatoba | 6.8 | 76.0 | EC |
| 2 | Gravinol-SL | 3.2 | 58.8 | CA, EC, ECG |
| 3 | Polyphenon | 1.0 | 44.3 | CA, EGCG, EC, ECG |
| 4 | Applephenon | 2.7 | 67.4 | CA, EC, ECG |
| 5 | Cranberry highly polymerized fraction | 6.2 | 68.3 | CA, EC, A-type linkage |
| 6 | Cacao polyphenol | 2.4 | 63.5 | CA, EC |
| 7 | Pine bark extract | 4.5 | 48.1 | CA, EC |
| 8 | Cinnamon | 5.5 | 32.2 | CA, EC |

| | | | | |
|---|---|---|---|---|
| EC: epicatechin; CA: catechin; ECG: epicatechin gallate; EGCG: epigallocatechin gallate | | | | |

Polyphenon, which is a polyphenol crude product derived from green tea, yielded no procyanidin oligomer and consisted mainly of epigallocatechin gallate (EGCG), EC, ECG, and catechin (CA) monomers only. Gravinol-SL derived from grape seeds and Applephenon (registered trademark) derived from apples are procyanidin crude products and their constitutive units were CA, EC, and ECG. Procyanidins of pine bark extract, cacao polyphenol, and cinnamon consisted of CA and EC. The average degree of polymerization of the samples ranged from 1 to 6.8 and Jatoba had the highest average degree of polymerization.

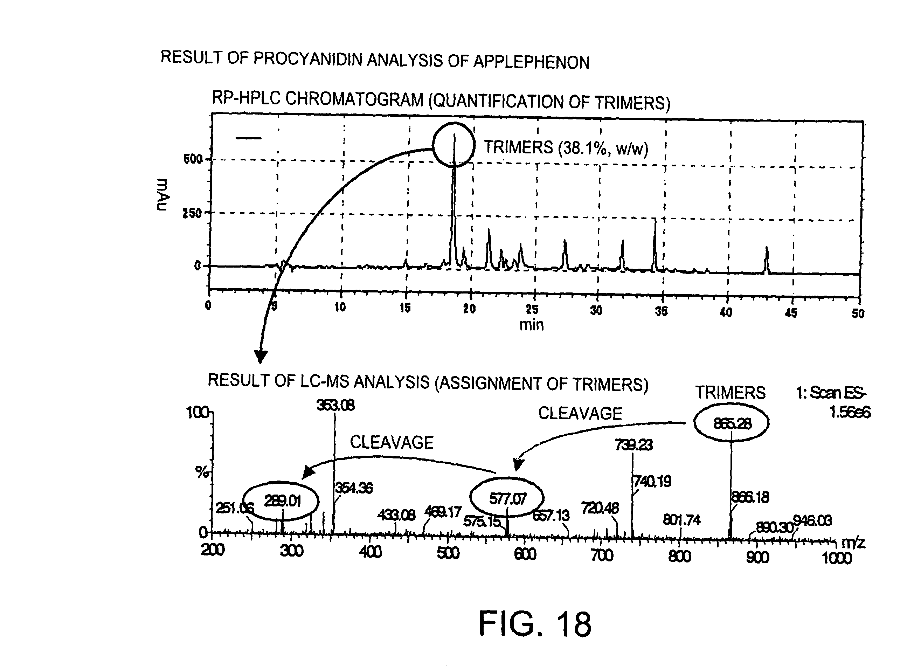

The average degree of polymerization of Applephenon was 2.7, while molecular ion peaks of procyanidin dimer, trimer, and tetramer only were observed when its sample without thiolytic cleavage was subjected to LC-MS as described in 1. (2) of Example 8. Further, quantitative analysis by the reversed liquid-phase chromatography (RP-HPLC) was performed as described in 1. (1) in Example 8, which showed that major component was trimers (38.1%, w/w) (Fig. 18).

The highly polymerized procyanidin fraction of cranberry was subjected to thiolysis and then to LC-MS as described in 1. (2) in Example 8, which confirmed the presence of A-type linkages. NoA-type linkage was recognized in samples other than the cranberry sample.

### Example 13: Preparation and analysis of procyanidin-containing samples (II)

### 1. Experimental methods

In order to more closely study the findings obtained in Example 12, commercialmaterials, Cinnamon (Okada Yakkyoku), Pycnogenol (Valentine), and Gravinol (Kikkoman Corporation) were subjected to the fractional precipitation method with chloroform as described in Example 9.

### (1) Cinnamon

To 200 g of cinnamon powder was added 2 L of ethanol, and the extraction was carried out at room temperature overnight. A cinnamon extract was obtained after filtration and then the solvent was removed by evaporation to obtain 22.58g of a crude extract. This extract was dissolved in 400 ml of methanol: water (50:50, v/v) and the concentration was adjusted to 40 g/L. This solution was washed 3 times with an equal volume of chloroform to remove impurities. The aqueous methanol fraction was distilled under reduced pressure to remove the solvent and obtain 11.97 g of a sample. This sample was redissolved in 300 ml of methanol and an equal volume of chloroform was added to make a chloroform:methanol (50:50, v/v) solution. The resulting precipitate (Fr. 1) was filtered using a glass funnel (pore size: P16) for fractionation, after which 150 ml of chloroform was added to the filtrate to make a chloroform:methanol (60:40, v/v) solution. The resulting precipitate (Fr. 2) was similarly fractionated and 250 ml of chloroform was added to the filtrate to make a chloroform:methanol (70:30, v/v) solution. The resulting precipitate (Fr. 3) was fractionated and 200 ml of chloroform was added to the filtrate to make a chloroform:methanol (75:25, v/v) solution. The resulting precipitate (Fr. 4) was fractionated and the filtrate was evaporated to remove the solvent and obtain Fr. 5.

### (2) Pycnogenol

10 g of Pycnogenol was dissolved in 200 ml of water and partitioned 4 times with an equal volume of ethyl acetate, and then the water phase was dried to solid to obtain 6.54 g of a sample. This sample was dissolved in 164ml of methanol and the concentration was adjusted to 40 g/L. To this solution, chloroform was successively added as described in (1) for fractional precipitation to obtain Fr. 1 to Fr. 5.

### (3) Gravinol

10 g of Gravinol was dissolved in 200 ml of water, and Fr. 1 to Fr. 5 were prepared in the same manner as described in (2).

### 2. Experimental results

### (1) Results of preparation of individual fractions

Procyanidins of cinnamon, Pycnogenol, and Gravinol were fractionated by the fractional precipitation method with chloroform. The weight and weight ratio of the resulting fractions are shown in Table 6.

**Table 6: The weights of the fractions obtained in (1) to (3) are shown.**

| | Cinnamon | | Pycnogenol | | Gravinol | |
|---|---|---|---|---|---|---|
| | Weight (g) | Weight ratio | Weight (g) | Weight ratio | Weight (g) | Weight ratio |
| Fr. 1 | 3.10 | 25.9% | 0.843 | 12.5% | 3.66 | 45.3% |
| Fr. 2 | 1.83 | 15.3% | 0.807 | 11.9% | 1.04 | 12.9% |
| Fr. 3 | 2.23 | 18.6% | 1.53 | 22.6% | 1.41 | 17.4% |
| Fr. 4 | 0.975 | 8.15% | 0.502 | 7.43% | 0.490 | 6.06% |
| Fr. 5 | 3.83 | 32.0% | 3.08 | 45.5% | 1.49 | 18.4% |
| Total | 12.0 | 100% | 6.75 | 100% | 8.08 | 100% |

### Example 14: Preparation and analysis of procyanidin-containing samples (III)

In order to precisely perform analysis for the average degree of polymerization and quantification, the individual preparations obtained in Example 12 were subjected to the thiolytic reaction to calculate in the same manner as in Example 11. Namely, atoluene-α-thioladductderivative of epicatechin gallate (ECG) (ECG-BTE) and a toluene-α-thiol adduct derivative of catechin (CA) (CA-BTE) were purified and isolated and subjected to high resolution mass spectrometry (HR-MS) andnuclearmagneticresonance (NMR) to identify the structures, after which HPLC analysis was performed using the products as standards.

### 1. Experimental methods

### (1) Isolation and purification of ECG-BTE

Gravinol was subjected to thiolysis in the same manner as in Example 8 and the reaction product was submitted to HPLC to fractionate the ECG-BTE peak. The chromatographic conditions were as follows.
Column: XTerra MS C18 φ19 x 100 mm (Waters)
Mobile Phase: 0.2% (v/v) aqueous formic acid:methanol (47:53)
Mobile phase flow rate: 3 ml/min
Detection wavelength: 280 nm

### (2) Identification of ECG-BTE structure

In order to verify the benzylthiol addition to position 4 of the flavan ring of ECG in the ECG-BTE obtained in the section above, the NMR spectrum was measured. The apparatus used was the UNITY INOVA-600 (600 MHz) by Varian. For signal comparison, proton (¹H) NMR of a commercial standard ECG (Funakoshi) was measured.

### (3) Isolation and purification of CA-BTE

Enzogenol (Valentine), a pine bark extract from the New Zealandpine tree, was subjected to thiolysis in the same manner as in Example 8 and the reaction product was submitted to HPLC to fractionate the CA-BTE peak. The chromatographic conditions were as follows. Further, for the purposed of identifying the CA-BTE peak, procyanidin B3 (CA dimer) was subjected to thiolysis.
Column: Discovery (registered trademark) C18 φ10 x 250 mm
(Sigma-Aldrich, Sperco section)
Mobile phase: 0.2% (v/v) aqueous formicacid:methanol (50:50) Mobile phase flow rate: 2 ml/min
Detection wavelength: 280 nm

### (4) Identification of CA-BTE structure

In order to verify the benzylthiol addition to position 4 of the flavan ring of CA, the NMR spectrum of the CA-BTE obtained in the section above was measured. The apparatus used was the UNITY INOVA-600 (600 MHz) by Varian. For signal comparison, proton (¹H) NMR of a commercial standard CA (Funakoshi) was measured.

### (5) HPLC analysis of procyanidins

Using epicatechin-benzyl thioether (EC-BTE) obtained in Example 11 and the ECG-BTE and CA-BTE obtained in this example as individual standards, HPLC analysis was performed in the same manner as in Example 8 to calculate the average degree of polymerization of procyanidins and their content. The average degree of polymerization was calculated as follows. Average degree of polymerization = (T+E)/T
T = mole of terminal units (EC, ECG, CA)
E = mole of extension units (EC-BTE, ECG-BTE, CA-BTE)

### 2. Experimental results

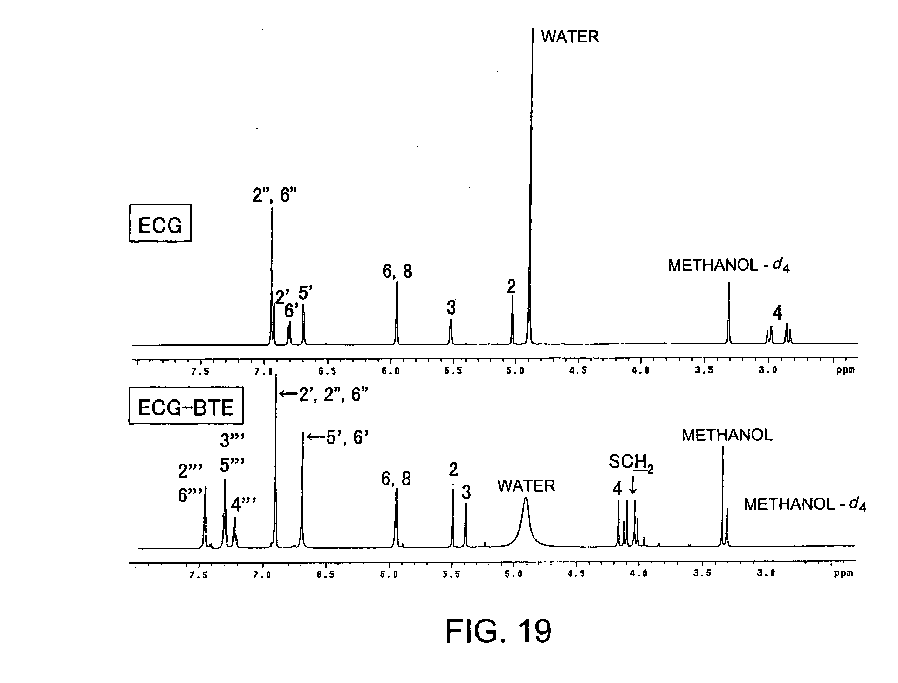

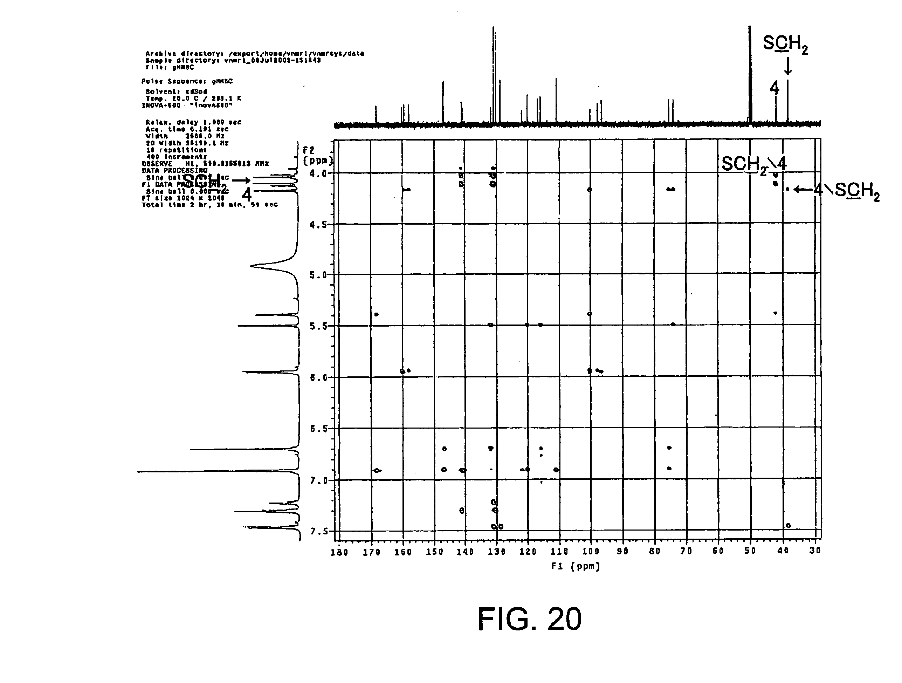

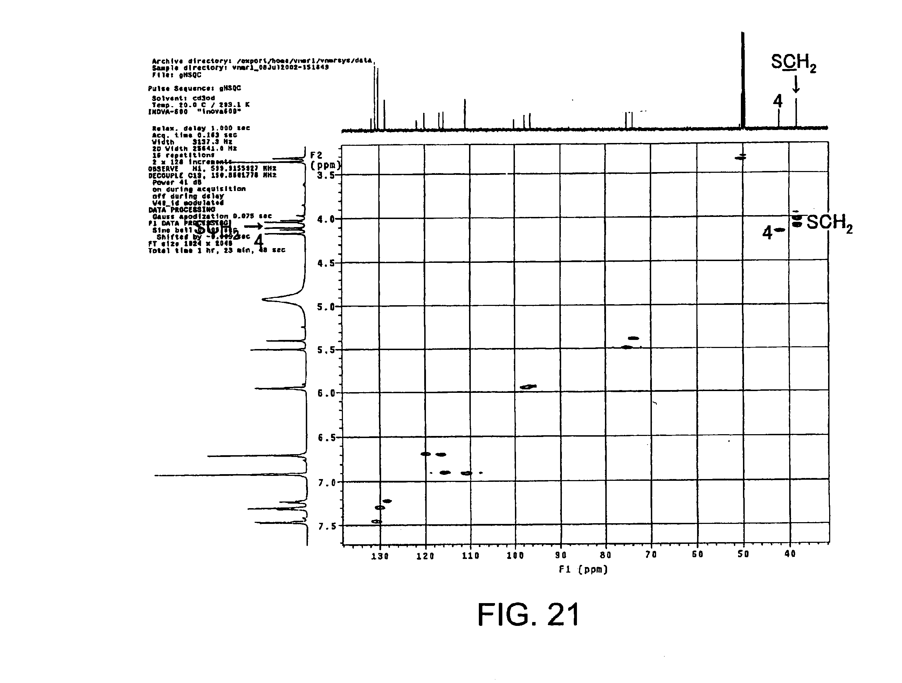

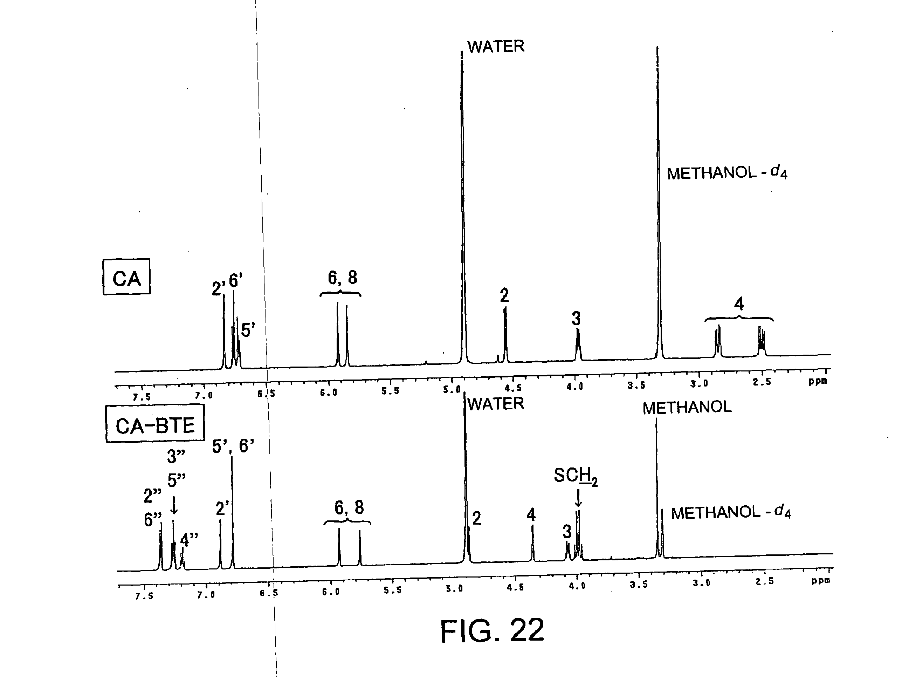

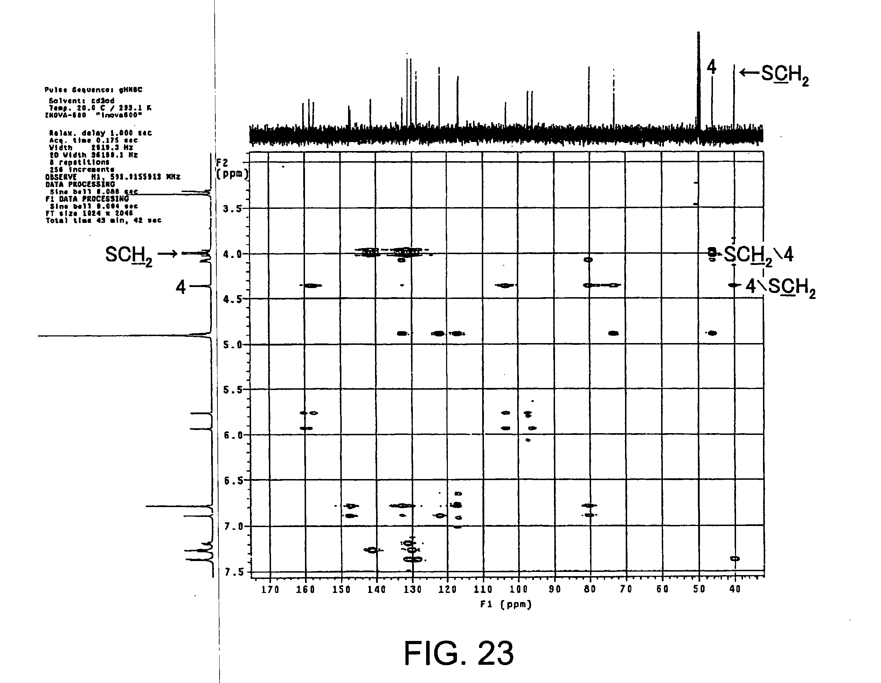

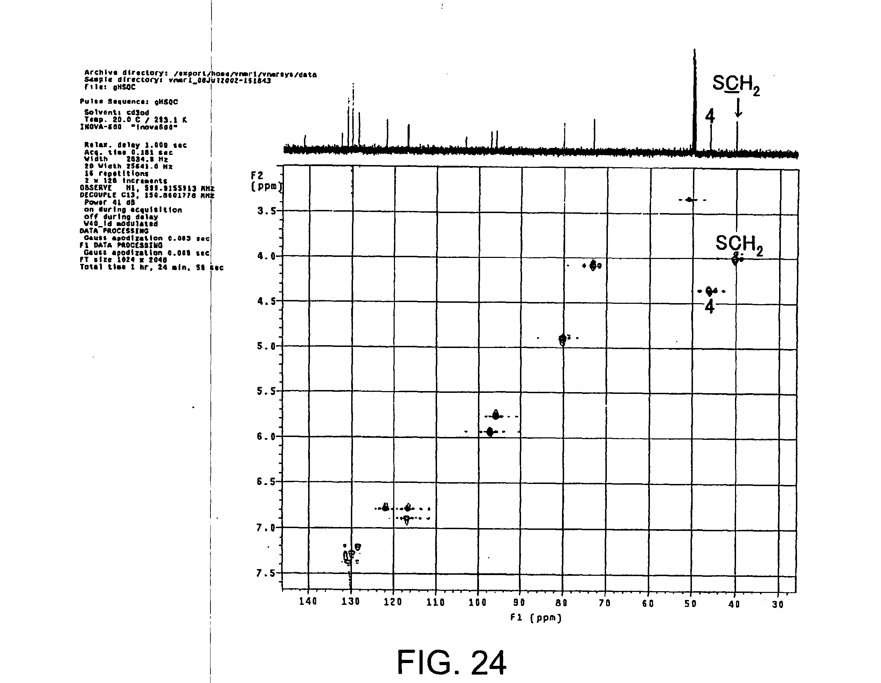

(1) From 326 mg of Gravinol, 32.0 mg of ECG-BTE was obtained.
(2) The result of proton NMR comparison between ECG-BTE and ECG (Fig. 19) showed that methylene signals at position 4 of the flavan ring, which were observed for ECG at 2.85 ppm and 3.00 ppm, was absent in ECG-BTE, and alternatively a methine signal at position 4 shifted to a lower magnetic field at 4.17 ppm and aromatic proton signals of benzylthiol at the 7.2 ppm to 7.5 ppm region were observed, associated with benzylthiol addition. Further, since a long range correlation signal from a proton at the SCH₂ site of benzylthiol to a carbon at position 4 and a remote correlation signal from a proton at position 4 to a carbon at the SCH₂ site were observed in ¹H-detected multiple-bond heteronuclear multiple quantum coherence (HMBC) spectrum (Fig. 20), it was verified that benzylthiol was added at position 4 of the flavan ring. Here signals of carbon at position 4 and carbon at the SCH₂ site were assigned by heteronuclearsingle quantum coherence (HSQC)spectrum (Fig. 21). From the results above, the ECG-BTE structure was identified.
(3) The column elution position of CA-BTE was determined to be 18.6 minutes by the thiolytic reaction product of procyanidin B3. From 90 mg of Enzogenol, 11.3 mg of CA-BTE was obtained.
(4) The result of proton NMR comparison between CA-BTE and CA (Fig. 22) showed that methylene signals at position 4 of the flavan ring, which were observed in CA at 2.50 ppm and 2.85 ppm, were absent in CA-BTE, and alternatively a methine signal at position 4 shifted to a lower magnetic field at 4.36 ppm and aromatic proton signals of benzylthiol at the 7.2 ppm to 7.4 ppm region were observed, associated with benzylthiol addition. Further, since a long range correlation signal from a proton at the SCH₂ site of benzylthiol to a carbon at position 4 and a long range correlation signal from a proton at position 4 to a carbon at the SCH₂ site were observed in ¹H-detected multiple-bond heteronuclear multiple quantum coherence (HMBC) spectrum (Fig. 23), it was verified that benzylthiol was added at position 4 of the flavan ring. Here signals of carbon at position 4 and carbon at the SCH₂ site were assigned by heteronuclear single quantum coherence (HSQC) spectrum (Fig. 24). From all the results above, the CA-BTE structure was identified.
(5) HPLC analysis was performed to obtain the average degree of polymerization of procyanidins and their content (%, w/w) of various preparations using EC, CA, ECG, EC-BTE, CA-BTE and ECG-BTE produced by thiolytic cleavage as standards. The results are shown in Table 7.

Table 7: Procyanidins contained in Jatoba, Gravinol SL, Polyphenon, Applephenon, purified highly polymerized fraction of cranberry, cacao polyphenol, pine bark extract, and cinnamon crude extract were analyzed for their average degree of polymerization, content and constitutive units.

| No. | Sample | Average degree of polymerization | Content (%) | Constitutive units |
|---|---|---|---|---|
| 1 | Jatoba | 8.2 | 70.4 | EC |
| 2 | Gravinol-SL | 3.7 | 59.1 | CA, EC, ECG |
| 3 | Polyphenon | 1.0 | 44.3 | EGCG, EC, ECG |
| 4 | Applephenon | 3.3 | 76.7 | CA, EC, ECG |
| 5 | Cranberry highly polymerized fraction | 8.8 | 65.9 | CA, EC, ECG, A-type linkage |
| 6 | Cacao polyphenol | 2.6 | 79.5 | CA, EC |
| 7 | Pine bark extract | 5.2 | 44.4 | CA, EC |
| 8 | Cinnamon | 7.0 | 32.5 | CA, EC |

| | | | | |
|---|---|---|---|---|
| EC: epicatechin; CA: catechin; ECG: epicatechingallate; EGCG: epigallocatechin gallate | | | | |

### Example 15: Preparation and analysis of procyanidin-containing samples (IV)

The fractions derived from cinnamon, Pycnogenol, and Gravinol obtained in Example 13 were cleaved by thiolysis as inExample8, after which identification of constitutive units, calculation of the polymerization degree and quantitative analysis for procyanidins were performed in the same manner as described in Example 14.

### 1. Experimental methods

Using epicatechin-benzyl thiolether (EC-BTE) obtained in Example 11 and ECG-BTE and CA-BTE obtained in Example 14 as individual standards, HPLC analysis was performed in the same manner as in Example 8 to calculate the average degree of polymerization of procyanidins and their content. The average degree of polymerization was calculated as follows. Average degree of polymerization = (T+E)/T
T = mole of terminal units (EC, ECG, CA)
E = mole of extension units (EC-BTE, ECG-BTE, CA-BTE)

### 2. Experimental results

HPLC analysis was performed using EC, CA, ECG, EC-BTE, CA-BTE andECG-BTE obtained by thiolytic cleavage as individual standards. The resulting average degree of polymerization of procyanidins and their content (%, w/w) of the individual fractions obtained are shown in Table 8.

Table 8: The average degree of polymerization of procyanidins (PCs) and their content (%, w/w) of the individual fractions obtained by fractionating cinnamon, Pycnogenol, and Gravinol by the fractional precipitation with chloroform are shown. The values for Jatoba are given for reference.

| | Fr. 1 | | Fr. 2 | | Fr. 3 | | Fr. 4 | | Fr5 | |
|---|---|---|---|---|---|---|---|---|---|---|
| | DPn | PC (%) | DPn | PC (%) | DPn | PC (%) | DPn | PC (%) | DPn | PC (%) |
| Cinnamon | 9.8 | 43.8 | 9.1 | 39.9 | 8.2 | 51.3 | 6.5 | 58.1 | 4.4 | 45.7 |
| Pycnogenol | 9.9 | 38.1 | 11.1 | 43.6 | 8.5 | 46.5 | 6.1 | 56.1 | 2.1 | 26.4 |
| Gravinol | 10.0 | 53.3 | 10.0 | 53.7 | 7.6 | 69.9 | 6.5 | 64.8 | 3.6 | 69.4 |
| Jatoba (reference) | 18.4 | 70.2 | 15.9 | 79.8 | 10.9 | 84.3 | 8.3 | 77.9 | 5.0 | 62.4 |

| | | | | | | | | | | |
|---|---|---|---|---|---|---|---|---|---|---|
| DPn: Average degree of polymerization | | | | | | | | | | |
| PC: Procyanidin content | | | | | | | | | | |

### Example 16: Purification of Jatoba procyanidin trimers to hexamers

### 1. Experimental methods

Procyanidins from trimers to hexamers were purified from Fr. 5 prepared in Example 9, using column chromatography. First, the elution positions for individual oligomers were determined by LC-MS and then a large scale purification was carried out using HPLC by Hitachi. Conditions used were as follows.
(1) LC-MS
   Column: Discovery (registered trademark), HS PEG φ10 x 250 mm, 5 µm (Sigma-Aldrich, Sperco section)
   Pump: Alliance (trademark) 2690 by Waters
   Mobile phase: 0.1% (v/v) formic acid-methanol:0.1% (v/v) aqueous formic acid (60:40, v/v)
   Mobile phase flow rate: 2 ml/min
   Detection wavelength: 280 nm (Waters 2996 photo-diode array detector)
   MS apparatus: Waters Micromass ZQ2000
   To a 10 µl portion of a 100 µg/µl Fr5 solution in methanol, 60 µl of methanol and then water were added to make a total volume of 120 µl. A 100-µl portion of the thus diluted solution was injected. The exit channel of the UV detector was split into 1/6 to introduce to MS. The ionization mode used was the ESI negative ion mode and procyanidins with a molecular weight of more than 2000 were detected with divalent ions. The cone voltage was set to 40 V.
(2) Large scale purification
   The column, mobile phase, mobile phase flow rate, detection wavelength were the same as in (1) and the Hitachi L-7100 pump and the Hitachi L-7455 detector (photo-diode array detector) were used. The sample loop volume was 1 ml and a sample of 150 mg of Fr5 dissolved in 1 ml of methanol:water (50:50, v/v) solvent was used for a single injection.

### 2. Experimental results

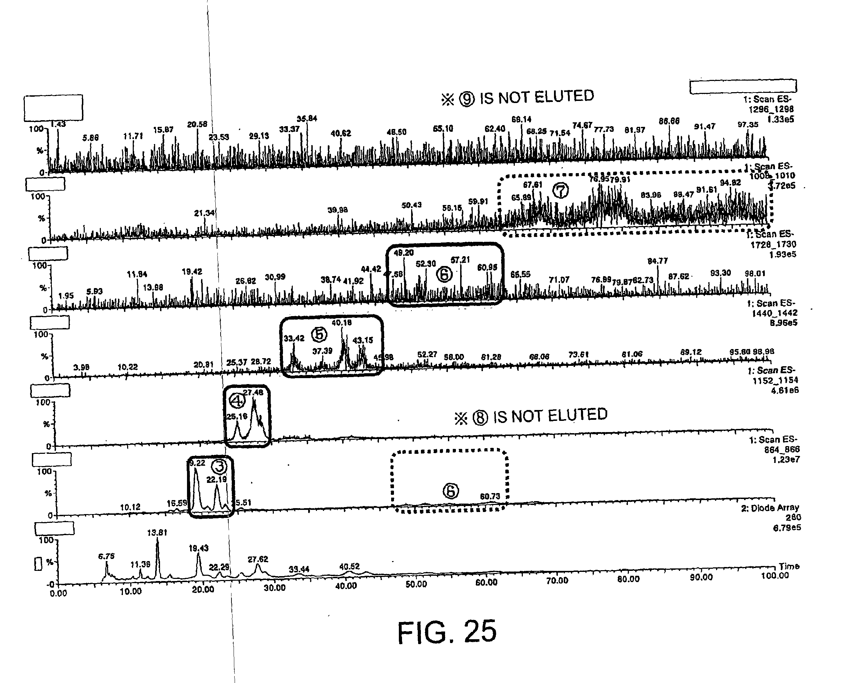

(1) LC-MS
   A chromatogrammonitored at 280 nm and an MS chromatogram are shown in Fig. 25. Plural peaks were detected for individual oligomers probably because isomers constructed with 4→8 linkage and 4→6 linkage were present. From Fr5, oligomers up to heptamers were detected but octamer and nonamer peaks were not detected. Here, since the upper detection limit of the MS apparatus is 2000, oligomers up to hexamers with a molecular weight of 1730 were detected with monovalent ions and heptamers were detected with divalent ions (1009).
   The PEG column revealed that individual oligomers were eluted consecutively by the polymerization degree.
(2) Large scale purification
   Purification of the trimers to hexamers was carried out based on the elution peak pattern assigned in (1). Theheptamers were not fractionated since their content in Fr. 5 was small. From 2.31 g of Fr. 5, 132.1 mg of trimers, 173.1 mg of tetramers, 187.0 mg of pentamers, and 111. 0 mg of hexamers were obtained. A portion of each of the purified products was subjected to HPLC as above to examine their purity. The result showed that the amount of cross-contamination between adjacent oligomers was very small and the individual purity was 95.7% for trimers, 92.1% for tetramers, 97.4% for pentamers, and 95.9% for hexamers.

### Example 17: Purification of Jatoba procyanidin hexamers to nonamers

### 1. Experimental methods

Procyanidin hexamers to nonamers were purified from Fr. 4 prepared in the same manner as in Example 9, using column chromatography. First, the elution positions for individual oligomers were determined by LC-MS and then a large scale purification was carried out using HPLC by Hitachi. Conditions used were as follows.
(1) LC-MS
   Column: Discovery (registered trademark), HS PEG φ10 x 250 mm, 5 µm (Sigma-Aldrich, Sperco section)
   Pump: Alliance (trademark) 2690 by Waters
   Mobile phase: 0.1% (v/v) formic acid-methanol:0.1% (v/v) aqueous formic acid (80:20, v/v)
   Mobile phase flow rate: 2 ml/min
   Detection wavelength: 280 nm (Waters 2996 photo-diode array detector)
   MS apparatus: Waters Micromass ZQ2000
   Fr. 4 was made into a 100 µg/µl solution in methanol and an 80 µl portion of the solution was diluted to make a total volume of 120 µl with water. A 100-µl portion of the thus diluted solution was injected. The exit channel of the UV detector was split into 1/6 to introduce toMS. The ionization mode used was the ESI negative ion mode and procyanidins with a molecular weight of more than 2000 were detected wi th divalent ions. The cone voltage was set to 40 V.
(2) Large scale purification
   The column, mobile phase flow rate, and detection wavelength were the same as in (1); the mobile phase of 0.1% (v/v) formic acid-methanol:0.1% (v/v) aqueous formic acid (80:20, v/v), the Hitachi L-7100 pump and the Hitachi L-7455 detector (photo-diode array detector) were used. The sample loop volume was 1 ml and a sample of 150 mg of Fr. 4 dissolved in 900 µl of methanol : water (70:30, v/v) solvent was used for a single injection.

### 2. Experimental results

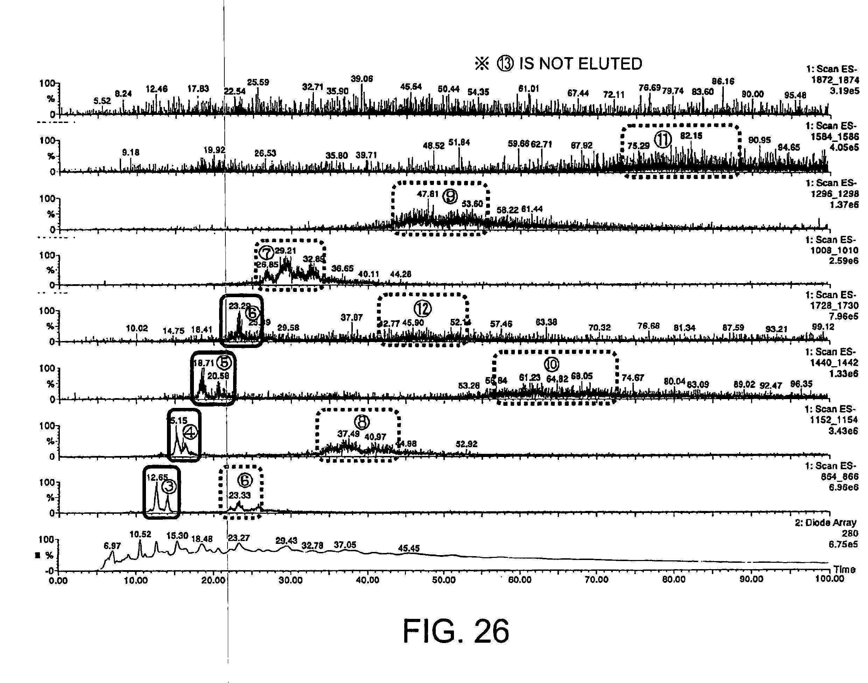

(1) LC-MS
   A chromatogram moni tored at 280 nm and an MS chromatogram are shown in Fig. 26. Plural peaks were detected for individual oligomers probably because isomers constructed with 4→8 linkage and 4→6 linkage were present. From Fr. 4, oligomers up to 12-mers were detected. Since the upper detection limit of the MS apparatus is 2000, oligomers up to hexamers with a molecular weight of 1730 were detected with monovalent ions and heptamers or larger oligomers were detected with divalent ions.
   The PEG column revealed that individual oligomers were eluted successively by the polymerization degree.
(2) Large scale purification
   Since the result of (1) showed that the amount of oligomers greater than decamers was small, purification of the hexamers to nonamers was carried out based on the assigned elution peak pattern. From 2.66 g of Fr.4, 156.2 mg of hexamers , 222.9 mg of heptamers, 236.1 mg of octamers, and 176.4 mg of nonamers were obtained. A portion of each of the purified products was subjected to HPLC as above to examine their purity. The result showed that the amount of cross-contamination between adjacent oligomers was very small and the individual purity was 95.9% for hexamers, 95.8% for heptamers, 90.3% for octamers, and 97.0% for nonamers.

### Example 18: Thiolysis analysis of procyanidin trimers to nonamers

### 1. Experimental methods

In order to calculate the procyanidin content (%, w/w) in the fractions obtained in Example 16 and Example 17, thiolysis analysis was performed by the method of Example 8 and the results were analyzed by the method of Example 11. In the thiolysis analysis, in addition to the procyanidin content (%, w/w), the average degree of polymerization can be calculated. Here, the hexamers used were those obtained in Example 17.

### 2. Experimental results

The procyanidin contents (%, w/w) of the trimer to nonamer fractions were shown in Table 9. The procyanidin (PC) content was 71.3% (hexamers) at the lowest and 83.0% (trimers) at the highest. It was also shown that the average degrees of polymerization calculated for individual fractions all agreed with the target degrees shown in Table 8 and thus the method of thiolysis analysis itself was extremely precise.

Table 9: Procyanidin trimers to nonamers were submitted to thiolysis analysis by the method of Example 8 and their average degree of polymerization and procyanidin (PC) content (%, w/w) were analyzed by the method of Example 11. The figures for the purity (%, w/w) of oligomers were the results of the quantification by HPLC in Example 16 and Example 17 and the total purity was calculated by multiplying the purity of oligomers by PC content.

| | 3mers | 4mers | 5mers | 6mers | 7mers | 8mers | 9mers |
|---|---|---|---|---|---|---|---|
| Average degree of polymerization | 2.9 | 3.8 | 4.9 | 6.0 | 6.6 | 7.7 | 9.2 |
| PC content (%) | 83.0 | 81.8 | 73.7 | 71.3 | 73.5 | 75.2 | 79.6 |
| Purity of oligomer (%) | 95.7 | 92.1 | 97.4 | 95.9 | 95.8 | 90.3 | 97.0 |
| Total purity (%) | 79.4 | 75.3 | 71.8 | 68.4 | 70.4 | 67.9 | 77.2 |

### Example 19: HPLC analysis of procyanidin oligomers

Procyanidins can be analyzed for their average degree of polymerization and content (%, w/w) by the thiolysis analysis as in Example 8 and Example 11 and for the distribution of oligomers by mass spectrometry as in Example 7, Example 16, and Example 17; however, the distribution of the procyanidin oligomers can be detected up to higher degrees of polymerization by setting the conditions of HPLC in Example 16 and Example 17 as follows.

### 1. Experimental methods

The conditions for LC-MS were set as follows. Column: Discovery (registered trademark), HS PEG φ10 x 250 mm, 5 µm (Sigma-Aldrich, Sperco section)
Pump: Alliance (trademark) 2690 by Waters
Mobile phase: 0.1% (v/v) formic acid-methanol:0.1% (v/v) aqueous formic acid (95:5, v/v)
Mobile phase flow rate: 2 ml/min
Detection wavelength: 280 nm (Waters 2996 photo-diode array detector)
MS apparatus: Waters Micromass ZQ2000

Fr. 5, Fr. 4, and Fr. 3 prepared in the same manner as in Example 9 were each made into a 100 µg/µl solution in methanol and an 80 µl portion of the solution was diluted to make the total volume of 120 µl with water. A 100 µl portion of thus diluted solution was injected. The exit channel of the UV detector was split into 1/6 to introduce to MS. The ionization mode used was the ESI negative ion mode and procyanidins with a molecular weight of more than 2000 were detected with divalent ions. The cone voltage was set to 40 V.

### 2. Experimental results

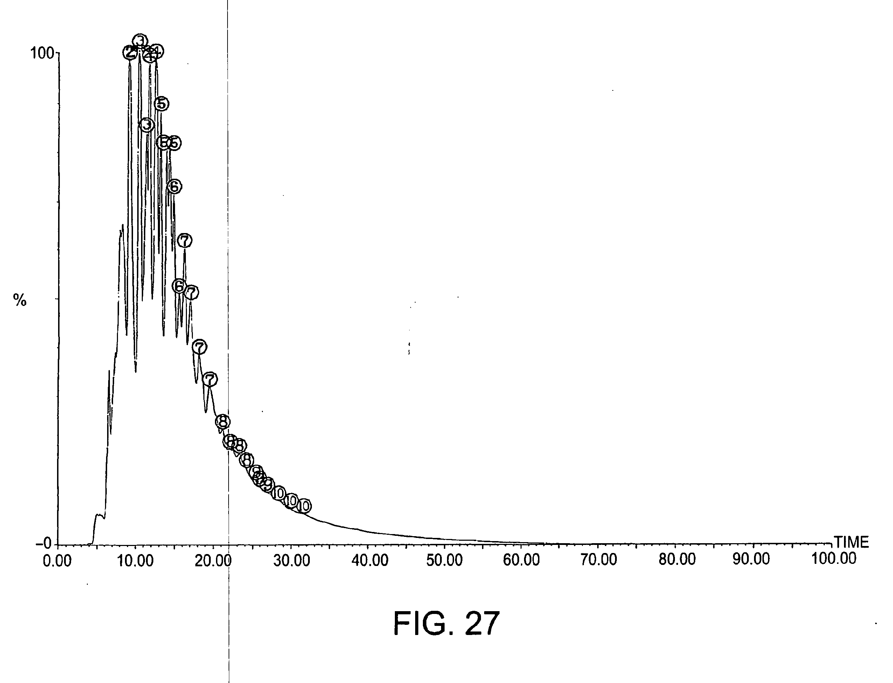

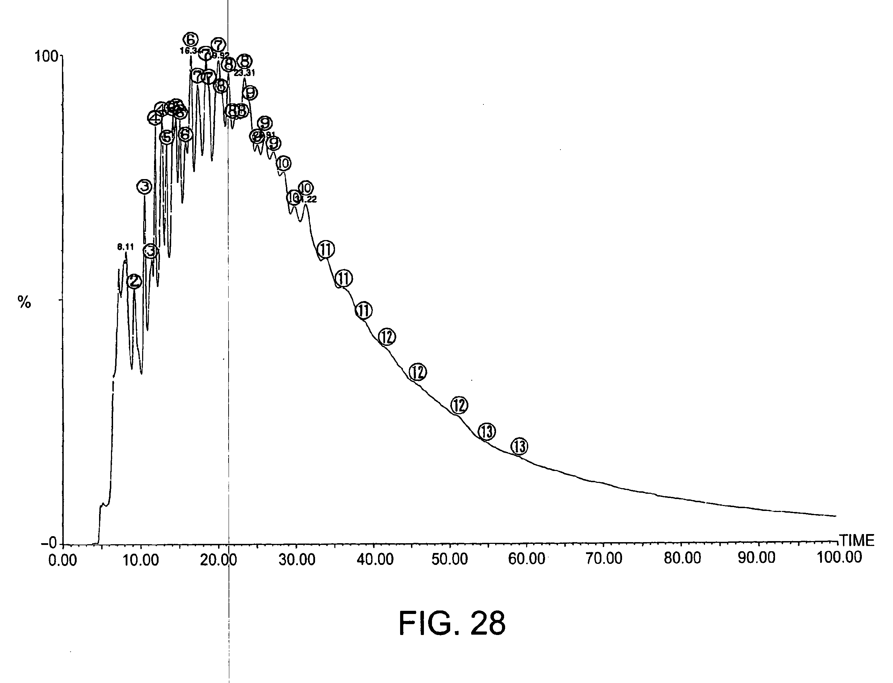

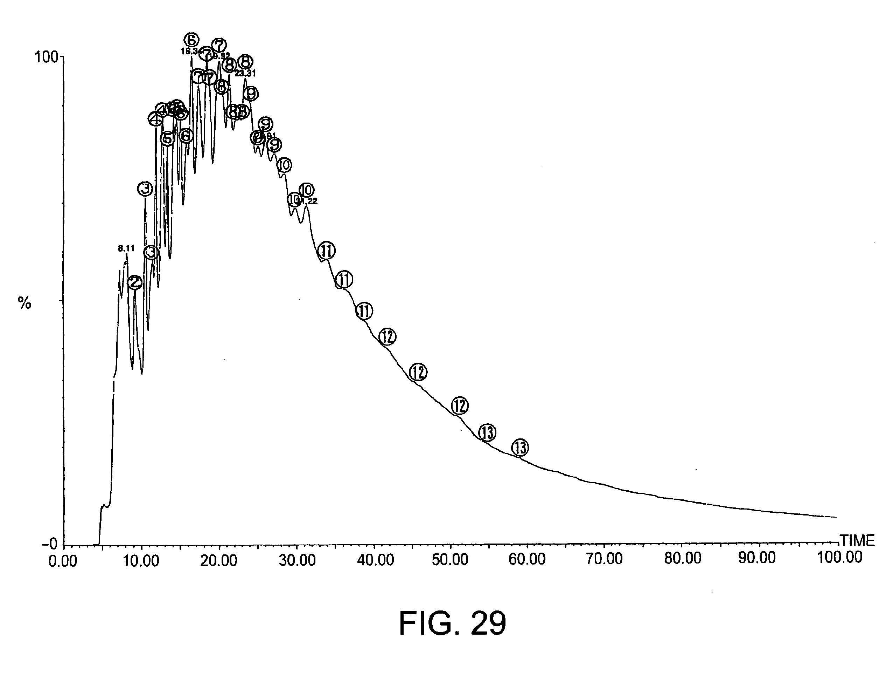

Chromatograms of Fr. 5, Fr. 4, and Fr. 3 monitored at 280 nm were shown in Fig. 27, Fig. 28, and Fig. 29, respectively. Peaks of up to 13mers were confirmed in Fr. 4 and Fr. 3 by MS. Since the upper detection limit of the MS apparatus is 2000, oligomers up to hexamers with a molecular weight of 1730 were detected with monovalent ions and heptamers or larger oligomers were detected with divalent ions. The detectable upper limit with dimer ions is 13 mers (ion peak 1873) and thus it is possible that Fr. 4 and Fr. 3 contain procyanidins with higher polymerization degrees.

### Example 20: In vivo effect of samples derived from various plant using EAE model

In order to study effects of proanthocyanidins in ameliorating autoimmune diseases, the samples derived from various plantmaterials in Example 8 were tested with EAE model mice.

### 1. Experimental methods

(1) Samples for administration
   The Jatoba ethanol extract of Example 2 and the individual samples of Example 12 were used. Each sample was prepared in a 1% ethanol solution so that its procyanidin content is the same as that of the Jatoba ethanol extract.
(2) Experimental conditions
   Six-week-old male C57BL/6 mice were divided into groups of 10 animals, i.e., a control group and groups to be administered with individual samples. In the control group, after the administration of MOG antigen on day 0, 1% ethanol was administered altogether 10 times on days 0, 2, 4, 7, 9, 11, 14, 16, 18, and 21. In the sample administration groups, the samples were administered in an amount of 50 mg/kg equivalent to that of Jatoba on the basis of an amount of procyanidin in the same schedule. On day 11, 5 animals were dissected, the splenocytes obtained were cocultured with MOG, and IFN-γ production in the culture supernatant was measured by ELISA. Further, clinical scores were observed up to day 25 with the remaining 5 animals in each group.

### 2. Experimental results

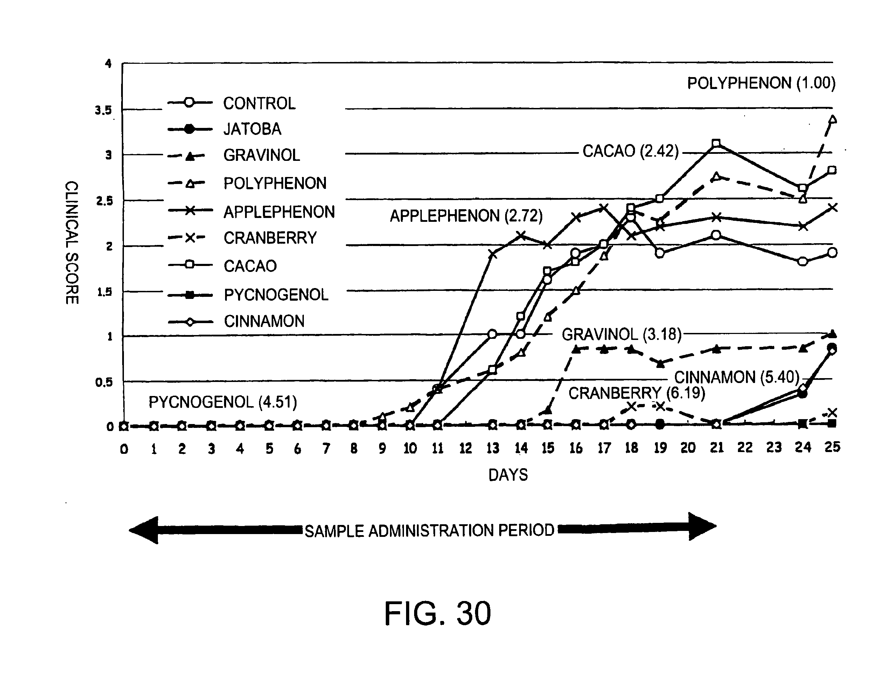

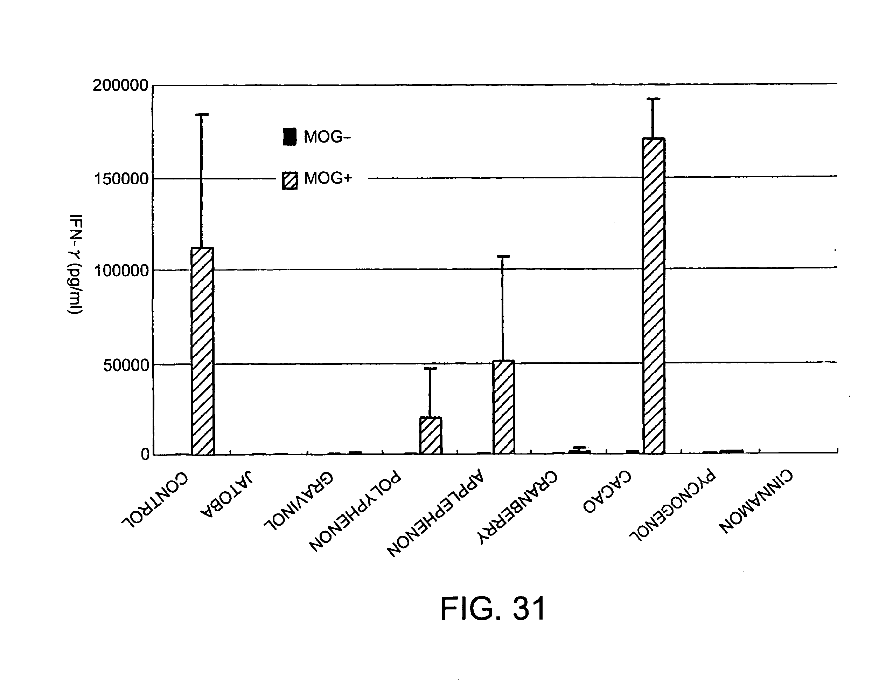

Fig. 30 shows the resultant clinical scores. In the control group, the onset started on day 11 and the maximum clinical score was about 2.5. Suppression of EAE onset similar with Jatoba was observed with the pine bark extract, the cranberry highly polymerized fraction, and the cinnamon extract and distinct onset was not observed with these materials up to day 25. Gravinol SL delayed the onset for 4 days and its clinical score showed suppressive effect at about a half the level of the control. As to other samples, EAE suppression was about the same as the control and thus no effect was recognized. The IFN-γ productivity by the splenocytes was completely suppressed with Gravinol, cranberry, pine bark extract, cinnamon, and Jatoba but it was not clearly recognized with other samples (Fig. 31).

The results above suggested that procyanidins with a polymerization degree of at least less than 3 has no suppressive effect on autoimmune diseases since Applephenon having trimers as its major component exhibited no effect as also shown in Example 12.

Here Gravinol exhibited a partial suppressive activity in spite of its polymerization degree (3.18) notmuch different from that of Applephenon (2.72). This difference is caused probably because Gravinol has a wider polymerization degree distribution including a sufficient amount of oligomers with a polymerization degree of 4 or more as confirmed in a preliminary test, whereas Applephenon mainly consists of trimers and is poor in oligomers with a higher degree of polymerization. These facts are considered to make the difference.

The kind of procyanidins contained in the samples which were effective cannot be simply specified; for example, linkage types, A or B, constitutive units, the presence or absence of gallate adducts were revealed not to correlate with the activity. From these results, it can be easily deduced that the number of hydroxyl group on the flavan backbone structure is also not related to the activity. Namely, it was revealed that solely the polymerization degree is involved in the activity and proanthocyanidins with a polymerization degree of 4 or more exhibit an ameliorating action on autoimmune diseases.

### Example 21: Generality of activity in materials containing highly polymerized procyanidins

For comparison of the activity of other materials containing highly polymerized procyanidins, fractions equivalent to Jatoba Fr3 (with an average polymerization degree of 10) were extracted from pine bark (Pycnogenol), cinnamon, and grape seeds (Gravinol) and studied for their ameliorating activity on autoimmune diseases.

### 1. Experimental methods

(1) Samples for administration
   Procyanidins used were Jatoba Fr3 (15.7) (Example 9) for highly polymerized procyanidins derived from Jatoba, Gravinol Fr2 (8.8) (Example 13) for highly polymerized procyanidins derived from Gravinol, Pycnogenol Fr2 (8.6) (Example 13) for highly polymerized procyanidins derived from pine bark, and cinnamon Fr1 (10.2) (Example 13) for highly polymerized procyanidins derived from cinnamon (figures in parentheses are average degrees of polymerization) . They were each dissolved in 1% ethanol at a concentration of 5 mg/ml. For control, a 1% ethanol solution was used.
(2) Experimental animals
   Animals used were 7 week-old male C57BL/6 mice divided into groups of 16 animals. To each mouse, an emulsion of CFA containing 200 µg of MOG and 800 µg of H37Ra was subcutaneously administered on day 0, and 200 µl of 1 µg/ml PTX (essentially 200 ng) was intraperitoneally administered on day 1 and on day 3 to induce symptoms.
(3) Measurement of splenocyte IFN-γ
   IFN-γ was measured using an OptEIA ELISA Set (Becton Dickinson).
(4) Experimental conditions
   Each sample was administered intraperitoneally 3 times a week at a dose of 1 mg/mouse. On day 11, animals were dissected to prepare splenocytes. The splenocytes were cultured in an RPMI1640 (Sigma) + 10% FCS (Roche) ± 2 µM MOG medium at 37°C in an atmosphere of 5% CO₂ for 7 days, after which the culture supernatant was recovered to measure the IFN-γ concentration.

### 2. Experimental results

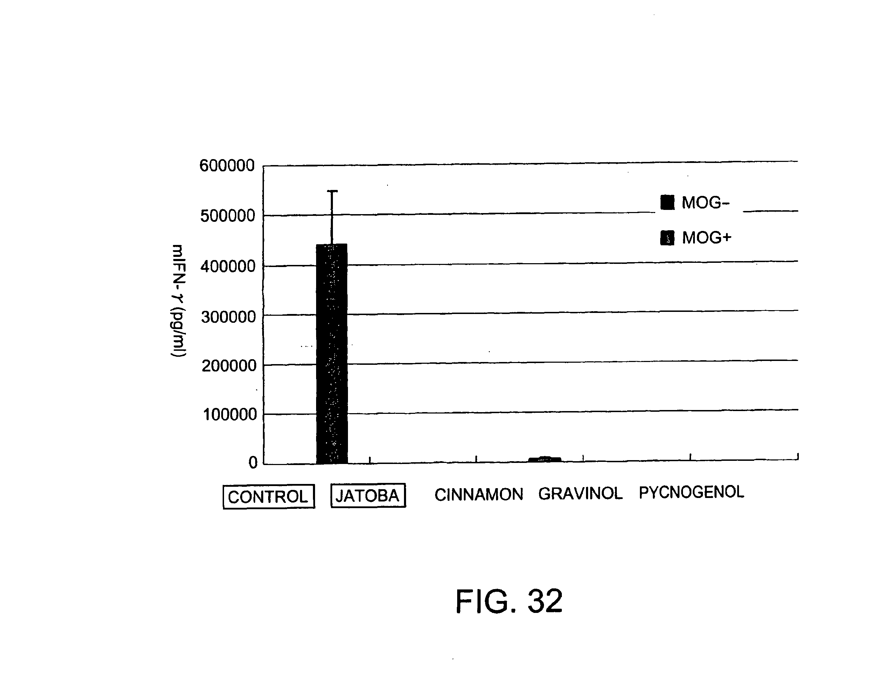

It was revealed that a strong anti-autoimmune disease activity was similarly found in other procyanidin-containing materials by concentrating highly polymerized procyanidins (Fig. 32). Further, as shown in Example 31 below, proanthocyanidins with a polymerization degree of 5 or more are responsible for the anti-autoimmune disease activity. Accordingly, it was revealed that this activity is not limited to Jatoba but also attributable to other materials which contain highly polymerized procyanidins.

### Example 22 : In vivo effect of Jatoba in type II collagen-induced arthritis model

In order to study efficacy of Jatoba in chronic rheumatoid arthritis, a type II collagen-induced arthritis model in mice was used.

### 1. Experimental methods

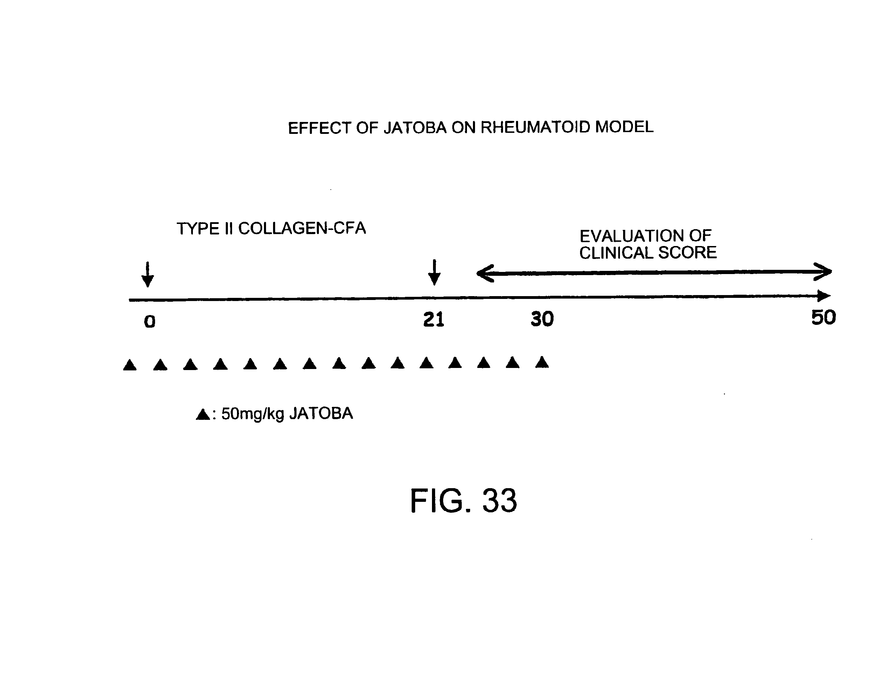

(1) Samples for administration
   The Jatoba ethanol extract of Example 2 was used.
(2) Experimental animals
   To 8-week-old male DBA/1J mice (purchased from Charles River Laboratories), an emulsion of 150 µg of type II collagen (Cosmo Bio) emulsified with 400 µg of CFA was subcutaneously administered on day 0 and on day 21 for induction.
(4) Experimental conditions
   The animals were divided into groups of 10, i.e., a control group and a Jatoba administration group. In the control group, 1% ethanol was intraperitoneally administered 3 times a week from day 0 to day 30. In the Jatoba administration group, the Jatoba ethanol extract was intraperitoneally administered at a dose of 50 mg/kg in the same schedule.
   The experimental schedule is shown in Fig. 33.
(4) How to record clinical scores
   Score 0: normal; score 1: swelling and/or redness of one small joint such as in toes and fingers; score 2: swelling and/or redness of two or more small joints or large joints such as in wrists and ankles; score 3: swelling and/or redness of one entire arm or leg; and score 4: maximum swelling in one arm or leg.
   Clinical observation was made on all four limbs according to the abovementioned criteria and the sums of the figures for the four were recorded as scores.

### 2. Experimental results

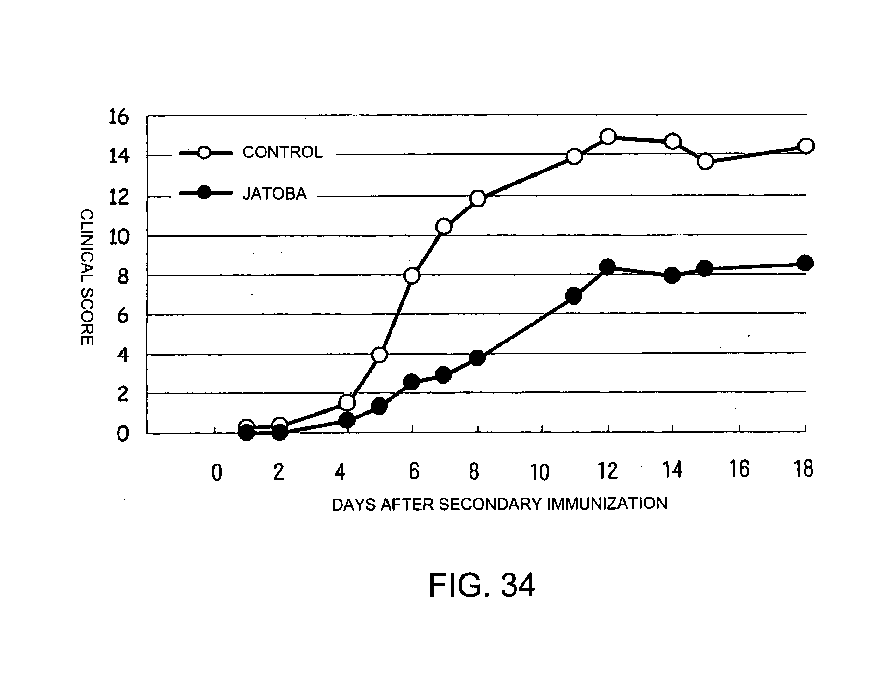

The change in the clinical scores with time is shown in Fig. 34. In the control group, a secondary immunization was given on day 21 (referred to as day 0) and the onset started on day 2 that was two days after the secondary immunization and nearly completed in 2 weeks (score 16). In the Jatoba administration group, the onset started on day 6 that was a week after the secondary immunization, but the symptoms progressed only up to score 8. These results showed that the Jatoba ethanol extract is effective also for chronic rheumatoid arthritis.

### Example 23: In vivo effect of Jatoba in type I diabetes model

In order to study efficacy of Jatoba in type I diabetes, type I diabetes-induced NOD/Ltj mice were used.

### 1. Experimental methods

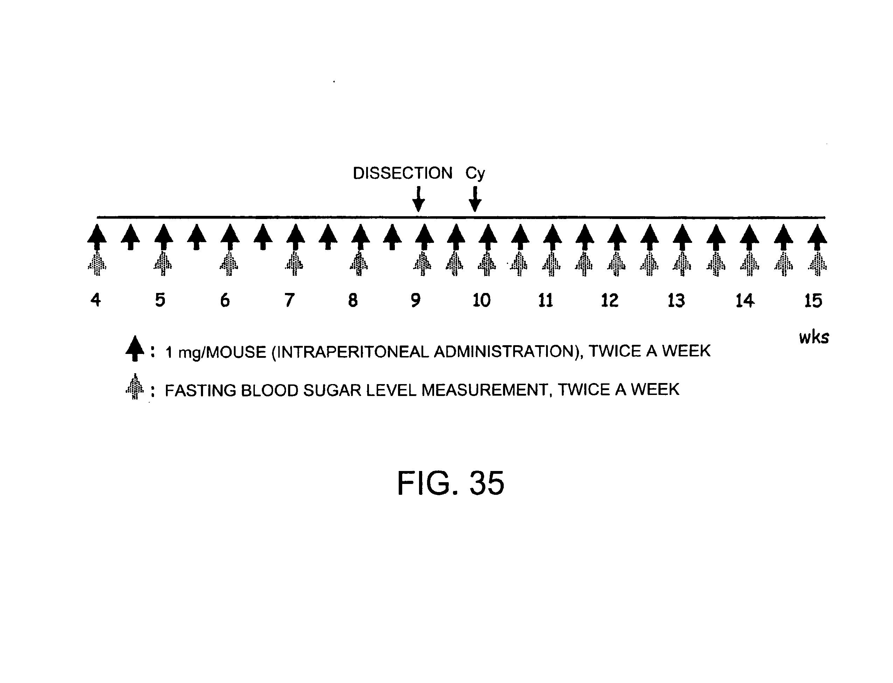

(1) Samples for administration
   The Jatoba ethanol extract of Example 2 was used.
(2) Experimental animals
   To 4-week-old female NOD/Ltj mice (purchased from Charles River Laboratories), cyclophosphamide (Cy) at a dose of 300 mg/kg was intraperitoneally administered at 10 weeks of age.
(4) Experimental conditions
   The animals were divided into groups of 20, i.e., a control group and a Jatoba administration group. In the control group, 1% ethanol was intraperitoneally administered two times a week from 4 weeks of age. In the Jatoba administration group, the Jatoba ethanol extract was intraperitoneally administered at a dose of 50 mg/kg in the same schedule. At 9 weeks of age, 5 animals were dissected and the obtained splenocytes were cocultured with insulin, one of antigens, after which the amount of IFN-yproducedin the culture supernatant was measured by ELISA. Cy was administered to the remaining 15 mice at 10 weeks of age and observation for clinical score was continued up to 35 days after Cy administration. The experimental schedule is shown in Fig. 35.
(4) Onset judgment
   The onset of diabetes was assessed by the measurement of a blood sample taken in a very small amount from the tail using Glutest Ace (Sanwa Kagaku). The values exceeding 250 mg/dl were assessed positive.

### 2. Experimental results

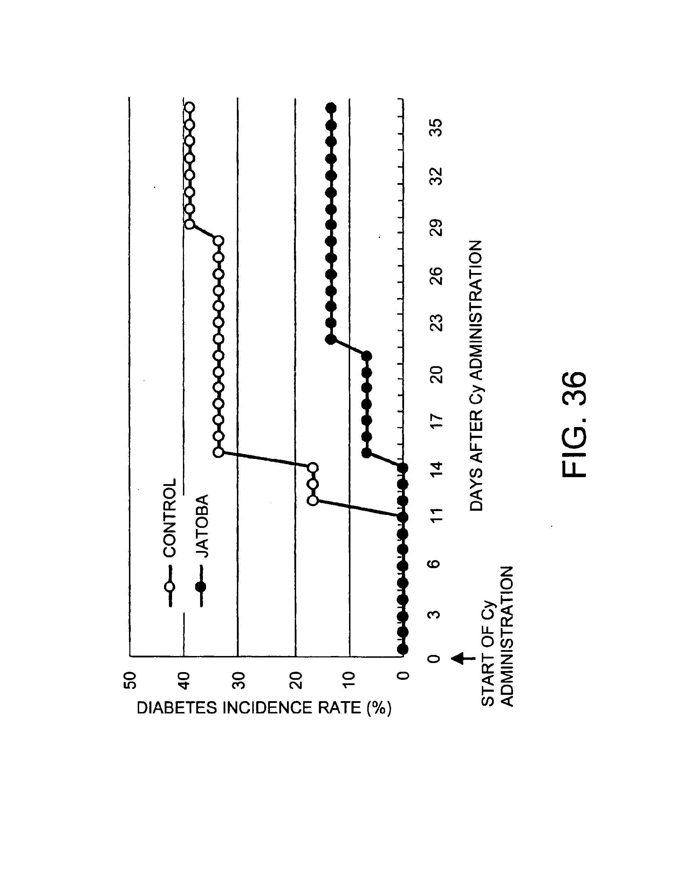

The diabetes incidence rate is shown in Fig. 36. On day 28 after Cy administration, the rate was 39% in the control group whereas it was only 13% in the Jatoba administration group.

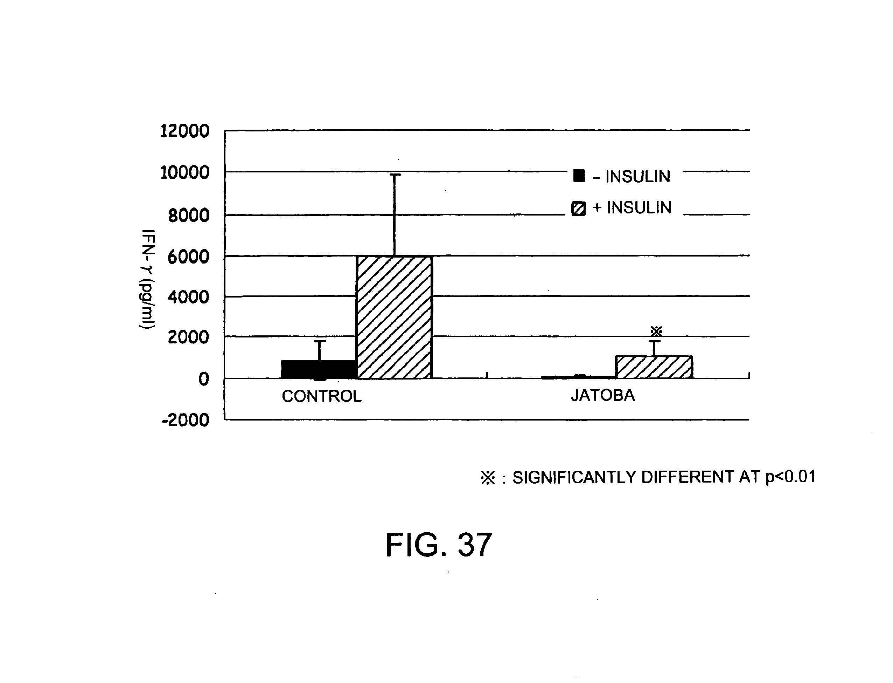

Further, the measurement of production of IFN-γ against insulin in the splenocytes at 9 weeks of age showed that the production was suppressed to about 1000 pg/ml in the Jatoba administration group whereas it was 6000 pg/ml in the control group. These results showed that the Jatoba extract was effective also for type I diabetes (Fig. 37).

### Example 24: Study on efficacy in type I diabetes

In Example 23, mice were administered with cyclophosphamide to induce type I diabetes and used for the experiment. In this example, a spontaneous model of long-term type I diabetes in mice was used to more precisely study the effect of the active ingredient according to the present invention.

### 1. Experimental methods

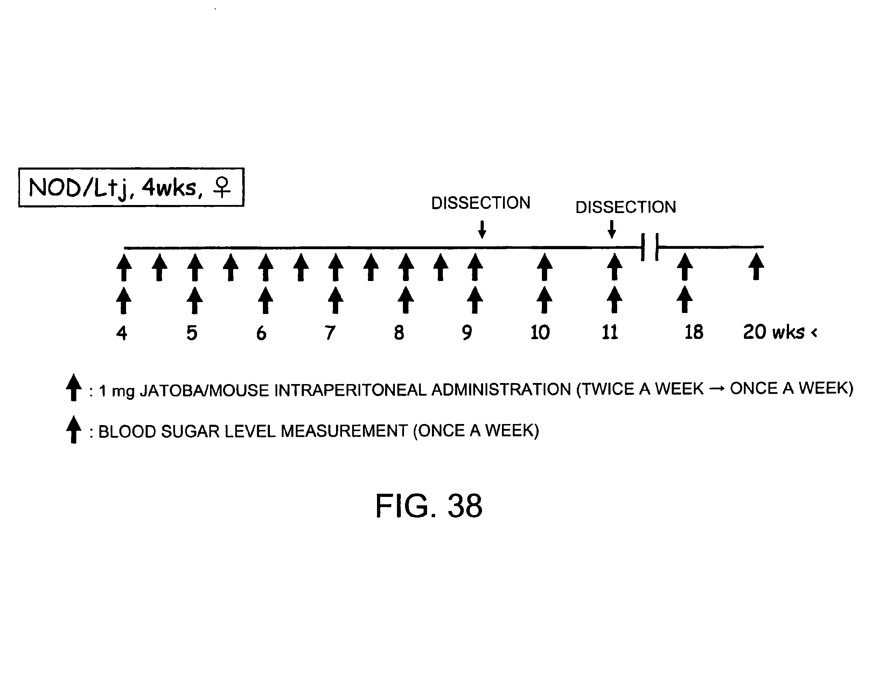

(1) Samples for administration
   The Jatoba ethanol extract (Example 2) dissolved in 1% ethanol at a concentration of 5 mg/ml was used for a Jatoba administration group and a 1% ethanol solution was used for a control group.
(2) Experimental animals
   Animals used were 4-week-old female NOD/Ltj mice (purchased from Charles River Laboratories).
(3) Experimental conditions
   The animals were divided into groups of 20. Upon the purchase at 4 weeks of age, intraperitoneal administration was started twice a week each with a dose of 1 mg/mouse of the Jatoba ethanol extract in the Jatoba administration group and with 200 µl of 1% ethanol in the control group. After week 9, the administration was changed to once a week and continued up to week 33 to complete the study on the incidence rate. Further, a portion of the animals (n = 3) were dissected on week 9 and antibody-specific reactions of the splenocytes were studied. The splenocytes were cultured in an RPMI1640 (Sigma) + 10% FCS (Roche) ± 300 µg/ml insulin (Sigma) medium for 3 days, after which the culture supernatant was measured for the IFN-γ concentration. Further on week 11, a portion of the animals (n=5) were dissected and the splenocytes were examined for the proportion of various immunocompetent cells. The blood sugar level of the remaining animals were measured once a week up to week 33 to study the incidence rate (Fig. 38).
(4) Measurement of splenocyte IFN-γ
   IFN-γ was measured using an OptEIA ELISA Set (Becton Dickinson).
(5) Ex vivo effect on the proportion of various immunocompetent cells
   Splenocytes were prepared from 5 mice of each group dissected on week 11. The expression of surface markers of immunocompetent cells were examined using flow cytometry under the same conditions as in Example 25.
(6) Onset judgment
   The onset of diabetes was assessed by the measurement of a blood sample taken in a very small amount from the tail using GlutestAce (Sanwa Kagaku) . The value exceeding 250 mg/dl were assessed positive.

### 2. Experimental results

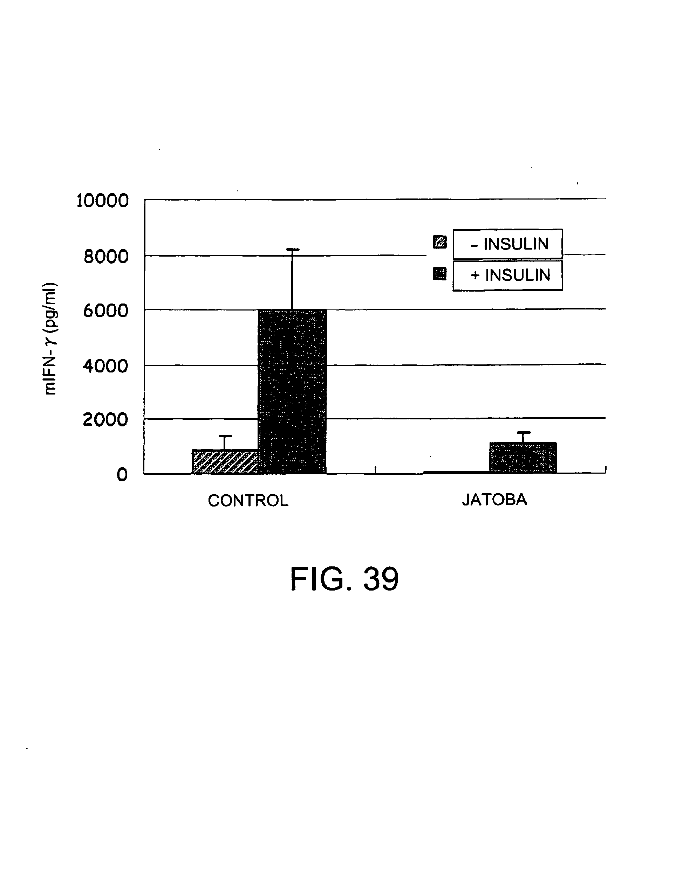

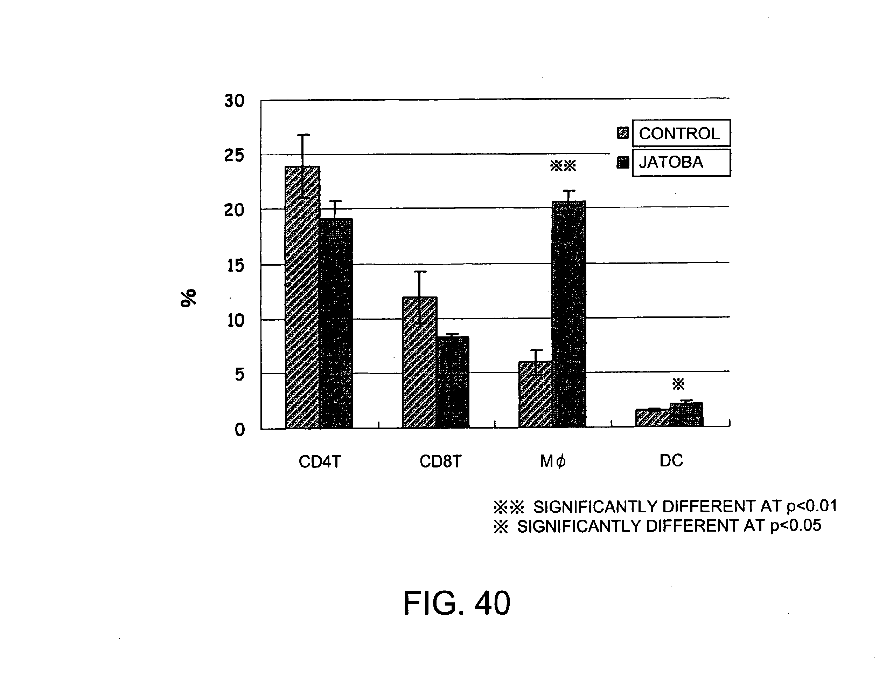

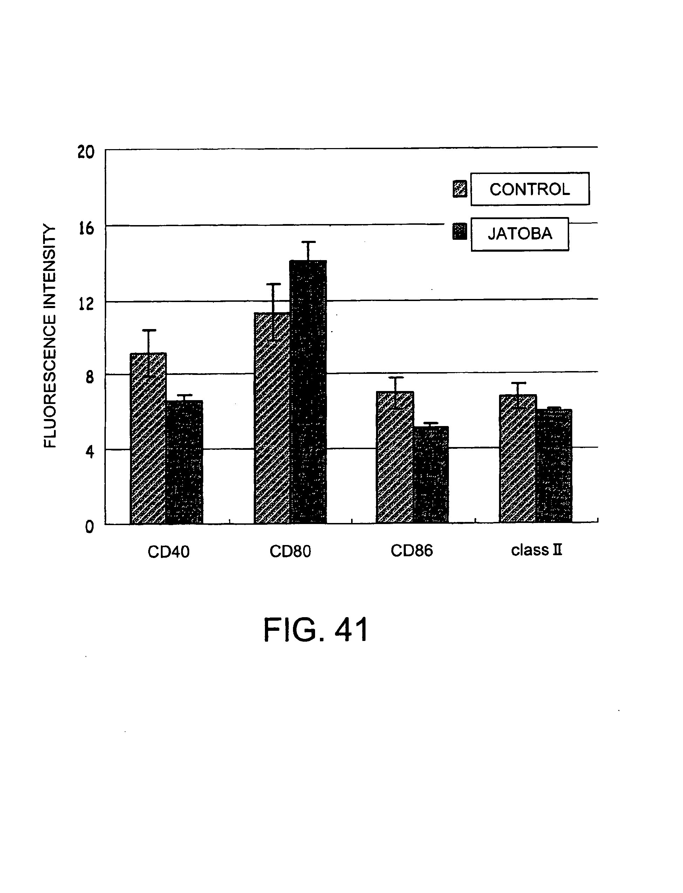

As a result of examining antigen-dependent reactions on week 9, IFN-γ production dependent on insulin, one of antigens, was detected. Further this IFN-γ production was suppressed by Jatoba administration (Fig. 39). As to the proportion of various immunocompetent cells of splenocytes on week 11, a marked increase in macrophages (about 6% ―> 20%) was observed whereas a decreasing tendency in the number of CD4⁺ T cells and CD8⁺ T cells was observed in the Jatoba administration group (Fig. 40). Further, when surface antigens of the increased macrophages were examined, decreasing tendencies in the expression ofMHC class II and co-stimulation molecules CD40 and CD86 were shown (Fig. 41) and thus immature macrophages with lower T-cell activating activity were considered to be increased in the Jatoba administration group. These are the same tendencies as observed in the effect of Jatoba administration in the EAE model in Example 25.

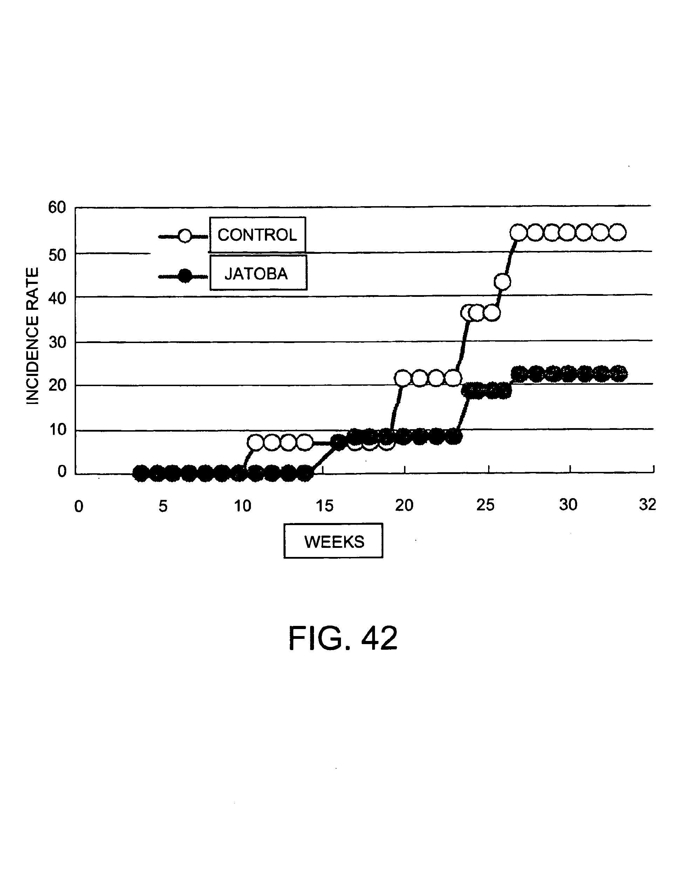

Further, the final incidence rate on week 33 was 54% in the control group whereas it was suppressed to 22% in the Jatoba administration group (Fig. 42) . These results revealed that the Jatoba extract rich in highlypolymerizedprocyanidins was effective in suppressing type I diabetes.

### Example 25: Study on the effect of Jatoba ethanol extract on the proportion of various immunocompetent cells in splenocytes

In order to elucidate the characteristics of the Jatoba extract, the proportion of immunocompetent cells in ex vivo splenocytes was studied in an experimental autoimmune encephalitis (EAE) model.

### 1. Experimental methods

(1) Samples for administration
   The Jatoba ethanol extract (Example 2) was dissolved in 1% ethanol at a concentration of 5 mg/ml. For a control group, a 1% ethanol solution was used.
(2) Experimental animals
   Animals (C57BL/6 mice) were immunized with MOG antigen to induce EAE and divided into groups of 3 animals. They were dissected with time on days 0, 2, 4, 8, and 15.
(3) Experimental conditions
   Intraperitoneal administration was carried out 3 times a week with 200 µl of 1% ethanol in a control group and with 200 µl of a 5 mg/ml solution in 1% ethanol in a Jatoba administration group.
(4) Ex vivo splenocyte population analysis
   On days 0, 2, 4, 8, and 15, 3 animals in each group were dissected and the splenocytes and lymph node cells were prepared. Expression of cell surface markers of immunocompetent cells was analyzed by flow cytometry (Becton Dickinson). The immunocompetent cells to be analyzed were T cells (CD3) [CD4-positive (CD4) T cells, CD8-positive (CD8) T cells, suppressive (CD4/CD25) T cells], B cells (B220), NK cells (DX5), macrophages (CD11b), and dendritic cells (CD11c) and cell surface markers in parentheses were used as indices. CD40, CD80, CD86, and MHC class II antibodies were used as cell surface markers to indicate macrophage maturity.
   Cells werepreincubatedwith CD16/CD32 (rat IgG2b, clone 93) to block Fc receptors and washed, after which 5 × 10⁵ cells were incubated at 4°C for 30 minutes with a mouse antibody specific to an antigen of interest in PBS containing 5% FBS and 0.1% sodium azide. Monoclonal antibodies labeled with fluoresceinisothiocyanate (FITC) orphycoerythrin (PE) were used for staining. The antibodies used were CD3e (Armenian hamster IgG, 145-2C11), CD4 (rat IgG2b, GK1.5), CD8a (rat IgG2a, 53-6.7), CD11b (rat IgG2b, M1/70), CD11c (Armenian hamster IgG, N418), CD40 (rat IgG2a, 1C10), CD80 (B7-1) (Armenian hamster IgG, 16-10A1), CD86 (B7-2) (rat IgG2b, GL1), and MHC class II (rat IgG2b, M5/114 15.2). As a negative control, FITC- or PE-labeled mouse IgG, rat IgG, or Armenian hamster IgG were used. These antibodies were all purchased from eBioscience Inc. (San Diego, CA).

### 2. Experimental results

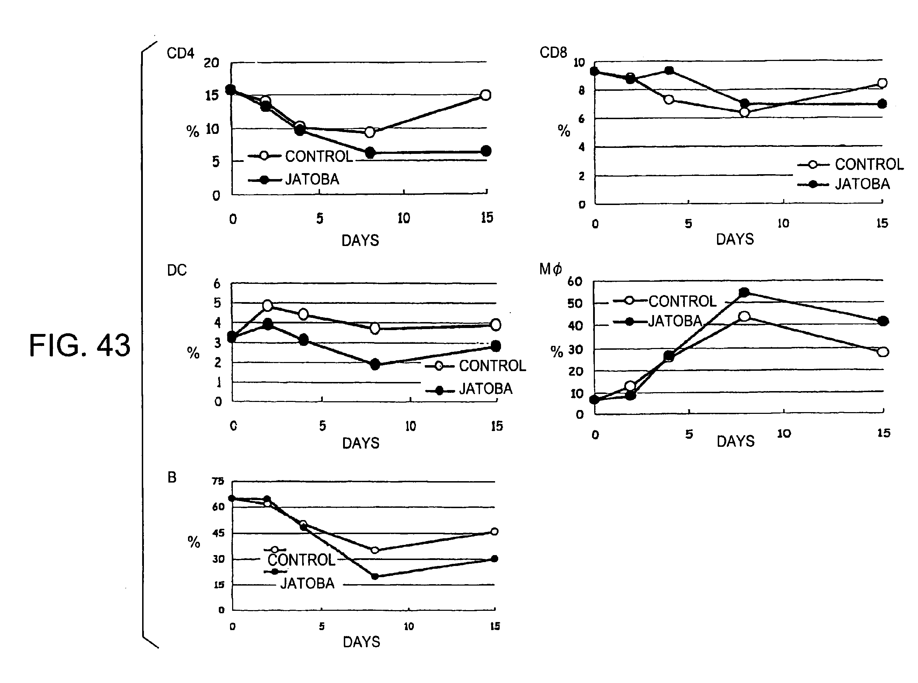

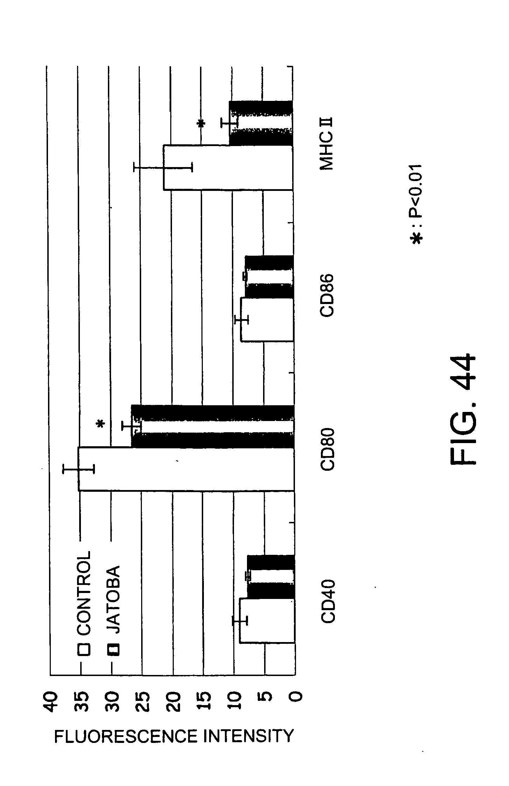

In the splenocytes in the period of day 8 to day 15, an increase in CD4⁺ T cells, which are helper T cells, was observed in the control group whereas they was suppressed in the Jatoba administration group. Further, DC and B cells, which are antigen presenting cells, tended to decrease through from the beginning of the experiment whereas macrophages tended to increase (Fig. 43). As to CD8⁺ T cells, no particular difference was observed between the two groups. In Th1 hyper-immune diseases, a decrease in CD4⁺ T cell, and a decrease in antigen presenting cells (DC) are considered to help suppress the symptoms, which was well explained by the decrease in the proportion of these cells by the Jatoba administration. On the other hand, in order to study the maturity of macrophages which contrarily increased in the Jatoba administration group, splenocytes on day 10 were further analyzed. Costimulation molecules CD40, CD80, CD86, and MHC class II, which are believed to reflect the antigen presenting ability, were used as surface markers. The result showed that the macrophages which were increased by the Jatoba administration tended to suppress expression of all the costimulation molecules tested and, in particular, significantly suppressed CD80 which is involved in inflammation amplification by Th1 reaction and MHC class II which is mainly involved in antigen presentation. These results suggested that the macrophages increased in splenocytes in the Jatoba administration group have a poorer antigen presenting ability than those in the control group (Fig. 44).

### Example 26: Improvement in clinical scores by oral Jatoba administration in type II collagen-induced arthritis model

In order to study the effect of oral Jatoba administration, a type II collagen-induced arthritis model was used.

### 1. Experimental methods

(1) Samples for administration
   The Jatoba ethanol extract (Example 2), the Jatoba Fr3 fraction (Example 9), and green tea polyphenol, a catechin monomer (Polyphenon, Mitsui Norin) were each dissolved in a 10% ethanol solution at a concentration of 50 mg/ml. A 10% ethanol solution was used for the control group.
(2) Experimental animals
   To 8-week-old male DBA/1J mice, an emulsion of 150 µg of bovine type II collagen (Cosmo Bio) emulsified with CFA containing 400 µg of dead tubercle bacilli (H37Ra; Difco) was subcutaneously administered on day 0 (primary immunization) and intraperitoneally 3 weeks after on day 21 (secondary immunization) to induce disease. The animals were divided into groups of 13.
(3) Experimental conditions
   From the day of starting the primary immunization to day 40, compulsory oral administration was carried out 5 times a week with 250 µl of 10% ethanol in the control group, and with 250 µl of each sample solution at 50 mg/ml in 10% ethanol in groups to be administered with the Jatoba ethanol extract, the Jatoba Fr3 fraction, and the green tea polyphenol (Polyphenon).
(4) How to record clinical scores
   The day of secondary immunization was set to be day 0 and the clinical conditions were observed up to day 31. Scoring was the same as in Example 22.

### 2. Experimental results

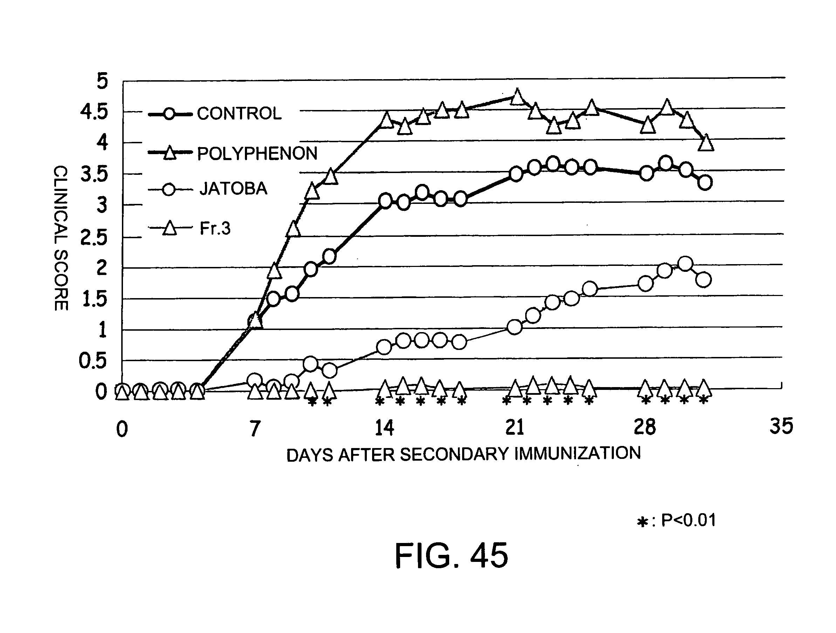

The change with time in clinical scores is shown in Fig. 45. The clinical score was suppressed by the administration of Jatoba ethanol extract. Further, the Fr3, which is a Jatoba fraction obtained by a rapid fractionation, exhibited a marked suppressive effect and the suppression was significant from day 10 after the secondary immunization. On the other hand, Polyphenon, a monomer polyphenol, showed a worsening tendency in the clinical score.

### Example 27: Suppression of Th1 cytokine productivity by Jatoba oral administration in type II collagen-induced arthritis model

In order to evaluate an ex vivo effect of Jatoba oral administration, a type II collagen-induced arthritis model was used.

### 1. Experimental methods

(1) Samples for administration
   The Jatoba ethanol extract (Example 2) dissolved in 10% ethanol at a concentration of 50 mg/ml was used for a Jatoba administration group. A 10% ethanol solution was used for a control group.
(2) Experimental animals
   To 8-week-old male DBA/1J mice, an emulsion of 150 µg of bovine type II collagen (Cosmo Bio) emulsified with CFA containing 400 µg of H37Ra was administered subcutaneously. The animals were divided into groups of 20.
(3) Measurement of splenocyte IFN-γ
   IFN-γ was measured using an OptEIA ELISA Set (Becton Dickinson) .
(4) Experimental conditions
   For 2 weeks after the start of the immunization, compulsory oral administration was carried 5 times a week with 250 µl of 10% ethanol in the control group and with 250 µl of a 50 mg/ml 10% ethanol solution in the Jatoba administration group. On day 14, animals were dissected to prepare splenocytes. The splenocytes were cultured in an RPMI1640 (Sigma) + 10% FCS (Roche) ± 25 µg/ml collagen medium at 37°C in an atmosphere of 5% CO₂ for 3 days, after which the culture supernatant was recovered. The IFN-γ concentration of the supernatant was measured.

### 2. Experimental results

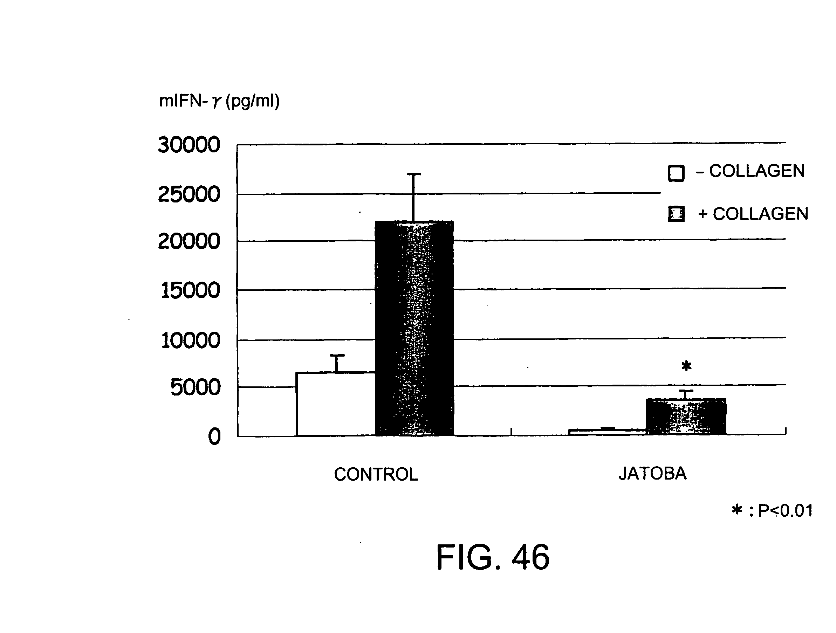

An antigen-dependent increase in IFN-γ in the control group and significant suppression in the Jatoba administration group were confirmed (Fig. 46) . This shows that the clinical score is improved through suppressing the production of Th1 cytokine IFN-γ by oral administration as well as by intraperitoneal administration.

### Example 28: Does dependence of the improving effect of Jatoba oral administration on clinical scores in type II collagen-induced arthritis model

In order to study does dependence of the effect of Jatoba oral administration, a type II collagen-induced arthritis model was used.

### 1. Experimental methods

(1) Samples for administration
   A rapid fractionation fraction Fr3 of the Jatoba extract (Example 9) was dissolved in 10% ethanol at concentrations of 50 mg/ml, 37.5 mg/ml, and 25 mg/ml. A 10% ethanol solution was used as a control.
(2) Experimental animals
   To 8-week-old male DBA/1J mice, an emulsion of 150 µg of bovine type II collagen (Cosmo Bio) emulsified with CFA containing 400 µg of dead tubercle bacilli (H37Ra; Difco) was administered subcutaneously on day 0 (primary immunization) . After 3 weeks, on day 21, intraperitoneal administration (secondary immunization) was carried out to induce symptoms. The animals were divided into groups of 7.
(3) Experimental conditions
   From the day of starting the primary immunization to day 40, compulsory oral administration was carried out 5 times a week with 250 µl of 10% ethanol in the control group and with 250 µl of the Fr3 solutions at concentrations of 50 mg/ml, 37.5 mg/ml, and 25 mg/ml in the administration groups. The amounts of the administration were 12.5, 9.375, and 6.25 mg/mouse on the basis of an amount of Fr3.
(4) How to record clinical scores
   Clinical conditions were observed from reset day 0 that was the day of the secondary immunization up to day 17. Scoring was the same as in Example 22.

### 2. Experimental results

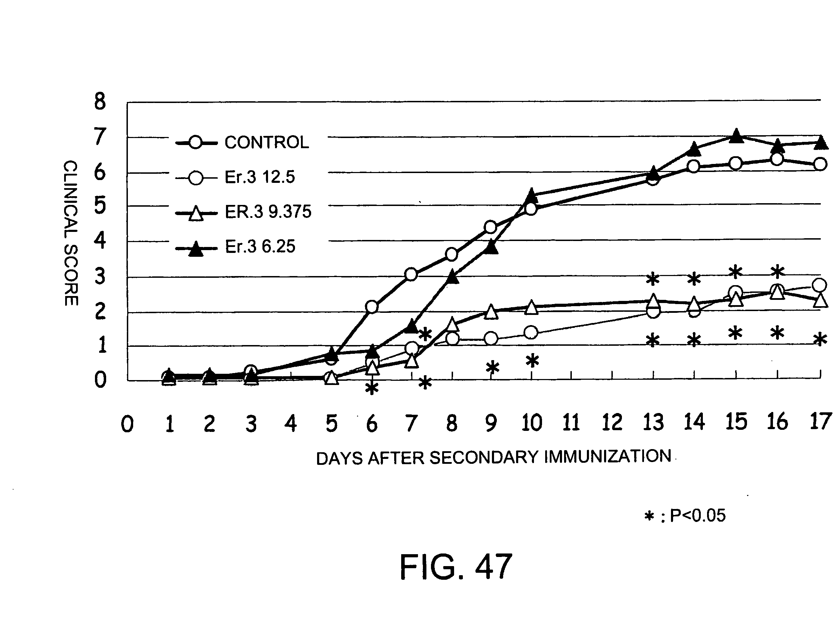

The change in the score is shown in Fig. 47. Conditions were significantly improved by Fr3 administration at a dose of about 9.375 mg/mouse or larger but no significant improvement in the score was observed at a dose smaller than that. Accordingly, the inhibitory effect on the disease of the Jatoba extract could be expected at a dose of about 9.375 mg/mouse or larger.

### Example 29: Study on timing of administration to obtain the effect of highly polymerized procyanidins

Oral administration of highly polymerized procyanidins was started after the onset, acute, or chronic periods to study effective timing of administration.

### 1. Experimental methods

(1) Samples for administration
   A 10% ethanol solution was used as a control and Jatoba Fr3 (Example 9) dissolved in 10% ethanol at a concentration of 50 mg/ml was used as a highlypolymerizedprocyanidin sample.
(2) Experimental animals
   To 8-week-old male DBA/1J mice, an emulsion of 150 µg of bovine type II collagen (Cosmo Bio) emulsified with CFA containing 400 µg of dead tubercle bacilli (H37Ra; Difco) was administered subcutaneously on day 0 (primary immunization) . After 3 weeks, on day 21, intraperitoneal administration (secondary immunization) was performed to induce symptoms. The animals were divided into groups of 12.
(3) Experimental conditions
   From day 0, the day of starting the primary immunization, to day 46, oral administration was carried out 5 times a week under the following conditions. (Administration starting on day 21, on day 28, and on 35 were designated to be onset, acute, and chronic period administrations, respectively.) A single dose of Jatoba Fr3 was 12.5 mg/mouse.
   I. Control group: Oral administration of 250 µl of 10% ethanol solution from the start of primary immunization to the end of the experiment;
   II. Jatoba administration group: Oral administration of Jatoba Fr3 from the start of primary immunization to the end of the experiment;
   III. Group starting on day 21: Oral administration of 250 µl of 10% ethanol solution from the start of primary immunization to day 20 and oral administration of Jatoba Fr3 from day 21 to the end of the experiment;
   IV. Group starting on day 28: Oral administration of 250 µl of 10% ethanol solution from the start of primary immunization to day 27 and oral administration of Jatoba Fr3 from day 28 to the end of the experiment; and
   V. Group starting on day 35: Oral administration of 250 µl of 10% ethanol solution from the start of primary immunization to day 34 and oral administration of Jatoba Fr3 from day 35, 2 weeks after secondary immunization, to the end of the experiment.
(4) How to record clinical scores
   Clinical conditions were observed from reset day 0 that was the day of secondary immunization to day 25. Scoring was the same as in Example 22.
2. Experimental results
   As to the incidence rate, the onsetperiod administration starting on day 21 and the acute period administration starting on day 28 were almost the same as the control (Table 10) . However, the maximum score was suppressed to about half of that of the control in the group starting administration on day 21 and to about 68% of that of the control in the group starting administration on day 28 (Table 10).

**Table 10**

| | incidence rate(%) | Maximum score |
|---|---|---|
| Jatoba Fr3 | 50.00 | 2.2 |
| Starting on day 21 | 92.86 | 4.1 |
| Starting on day 28 | 93.33 | 5.2 |
| Starting on day 35 | 100.00 | 6.8 |
| Control | 92.86 | 7.6 |

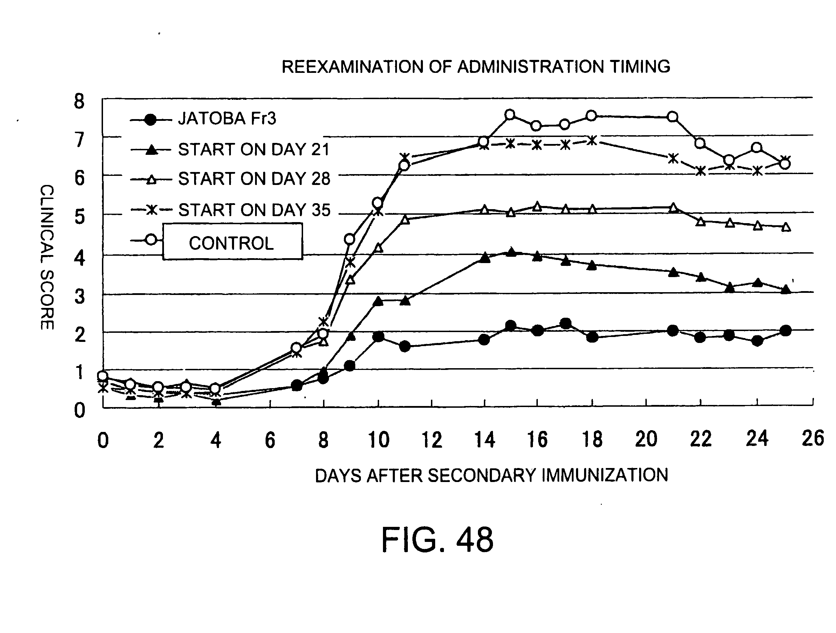

The change in average scores is shown in Fig. 48. It shows that worsening of the symptoms was suppressed in the groups starting the administration on day 21 and on day 28. It was thus demonstrated that effects of highly polymerized procyanidins in suppressing autoimmune diseases were efficacious even after the establishment of primary immunity.

### Example 30: Activity of highly polymerized procyanidins in reducing serum level of antibody

This experiment was to confirm the reduction of serum level of antibody in addition to the suppression of clinical conditions in a chronic rheumatoid arthritis model.

### 1. Experimental methods

(1) Samples for administration
   A 10% ethanol solution was used in a control group and Jatoba Fr3 (Example 9) dissolved in 10% ethanol at a concentration of 50 mg/ml was used in a Jatoba administration group.
(2) Experimental animals
   To 8-week-old male DBA/1J mice, an emulsion of 150 µg of bovine type II collagen (Cosmo Bio) emulsified with CFA containing 400 µg of dead tubercle bacilli (H37Ra; Difco) was administered subcutaneously on day 0 (primary immunization) . After 3 weeks, on day 21, intraperitoneal administration (secondary immunization) was performed to induce disease. The animals were divided into groups of 12.
(3) Measurement of serum level of antibody
   Measurement was made by adding a serum sample diluted 500 to 5000 times onto a plate which was coated at 4°C overnight with a 3 µg/ml bovine type II collagen (Cosmo Bio) solution in phosphate buffer. Primary antibodies used were 500-fold-diluted AP-conjugated anti-total IgG antibody, anti-IgG1 antibody, and anti-IgG2 antibody (Cosmo Bio). Thereafter, the detection was made at 410 nm after coloring with pNPP (Funakoshi).
(4) Experimental conditions
   From the day of starting primary immunization to day 40, oral administration was carried out 5 times a week with 250 µl of 10% ethanol in the control group and with 250 µl of Jatoba Fr3 in the Jatoba administration group. On day 42, blood was taken for serum preparation.

### 2. Experimental results

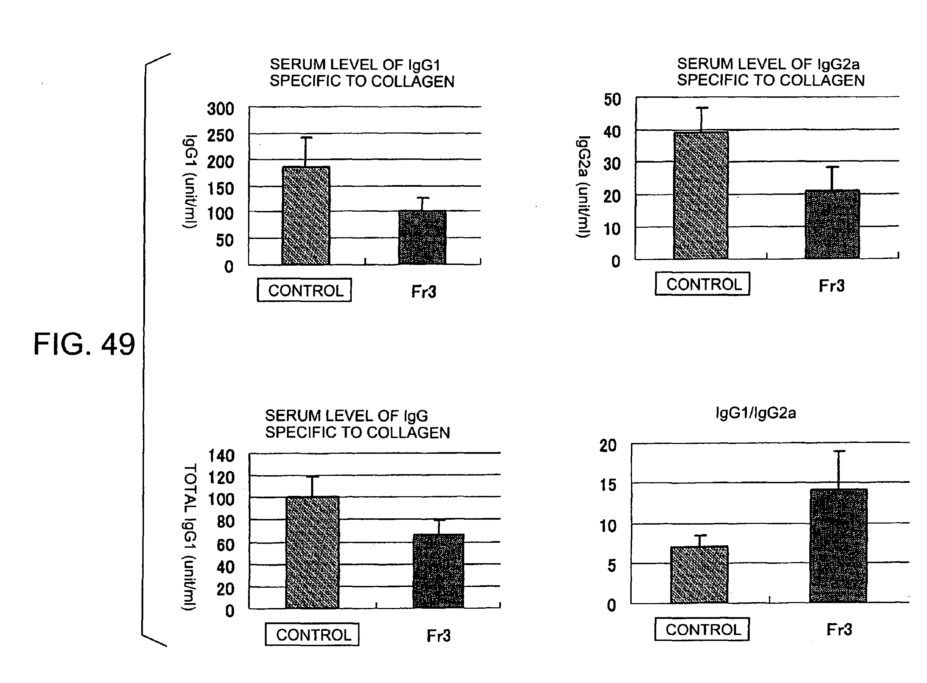

serum level of antibody specific to the antigen, i.e. , collagen was studied. The amounts of IgG1, IgG2a, and total IgG were all decreased by the Jatoba Fr3 administration. Further, the IgG1/IgG2a ratio tended to increase, showing a tendency of Th1 immune suppression (Fig. 49). These results revealed that auto-antibody production was suppressed by the administration of highly polymerized procyanidins and that the effect of the highly polymerized procyanidins in suppressing chronic rheumatoid arthritis was through the effect in suppressing the antigen-specific antibody production.

### Example 31: Study on procyanidin activity by polymerization degree (I)

Procyanidin trimer to hexamer fractions prepared by the polymerization degree from Jatoba extract were studied using an ex vivo EAE system.

### 1. Experimental methods

(1) Samples for administration
   The procyanidin trimer to hexamer fractions (Example 16) were each dissolved in a 1% ethanol solution at a concentration of 5 mg/ml on the basis of an amount of procyanidins. (-) Epicatechin (Sigma) as a monomer and procyanidin B2 (Funakoshi) as a dimer were purchased and dissolved in the same manner. A 1% ethanol solution was used for a control group.
(2) Experimental animals
   EAE was induced by immunizing C57 BL/6 mice with MOG antigen as described in Example 3. The animals were divided into groups of 12 and dissected on day 11.
(3) Measurement of splenocyte IFN-γ
   IFN-γ was measured using an OptEIA ELISA Set (Becton Dickinson).
(4) Experimental conditions
   From day 0 to day 10, intraperitoneal administration was performed 3 times a week with 200 µl of 1% ethanol in the control group and with 200 µl of a solution of (-) epicatechin, procyanidin B2 or each of the prepared oligomers in 1% ethanol at a concentration of 5 mg/ml in the experimental groups. On day 11, animals were dissected to prepare splenocytes. The splenocytes were cultured in an RPMI1640 (Sigma) + 10% FCS (Roche) + 2 µM MOG medium at 37°C in an atmosphere of 5% CO₂ for 7 days, after which the culture supernatant was recovered to measure the IFN-γ concentration.

### 2. Experimental results

The result of IFN-γ measurement is shown in Fig. 50. In comparing the activity of monomer (-) epicatechin to hexamers, IFN-γ suppression by pentamer or larger oligomers was observed. This result suggested that procyanidins with a polymerization degree of 5 or more were responsible for the Th1 cytokine suppression by administration of the Jatoba extraction.

### Example 32: Study on procyanidin activity by polymerization degree (II)

Procyanidin trimer to nonamer fractions prepared by the polymerization degree from Jatoba extract (Example 16 and Example 17) were studied using an ex vivo EAE system. In this experiment, the amount of dose used was a half of that used in Example 31 since the study included effects of heptamers to nonamers.

### 1. Experimental methods

(1) Samples for administration
   The purified procyanidin trimer to nonamer fractions (Example 16 and Example 17) were each dissolved in 1% ethanol at a concentration of 2.5 mg/ml on the basis of an amount of procyanidins. (-) Epicatechin (Sigma) as a monomer and Procyanidin B2 (Funakoshi) as a dimer were purchased and dissolved in the same manner. A 1% ethanol solution was used for a control group.
(2) Experimental animals
   EAE was induced by immunizing C57 BL/6 mice with MOG antigen as described in Example 31. The animals were divided into groups of 12 and dissected on day 11.
(3) Measurement of splenocyte IFN-γ
   IFN-γ was measured using an OptEIA ELISA Set (Becton Dickinson).
(4) Experimental conditions
   From day 0 to day 10, intraperitoneal administration was performed 3 times a week with 200 µl of 1% ethanol in the control group and with (-) epicatechin, Procyanidin B2 or each of the prepared oligomers at 500 µg/mouse in experimental groups. On day 11, animals were dissected to prepare splenocytes. The splenocytes were cultured in an RPMI1640 (Sigma) + 10% FCS (Roche) + 2 µM MOG medium at 37°C in an atmosphere of 5% CO₂ for 7 days, after which the culture supernatant was recovered to measure the IFN-γ concentration.

### 2. Experimental results

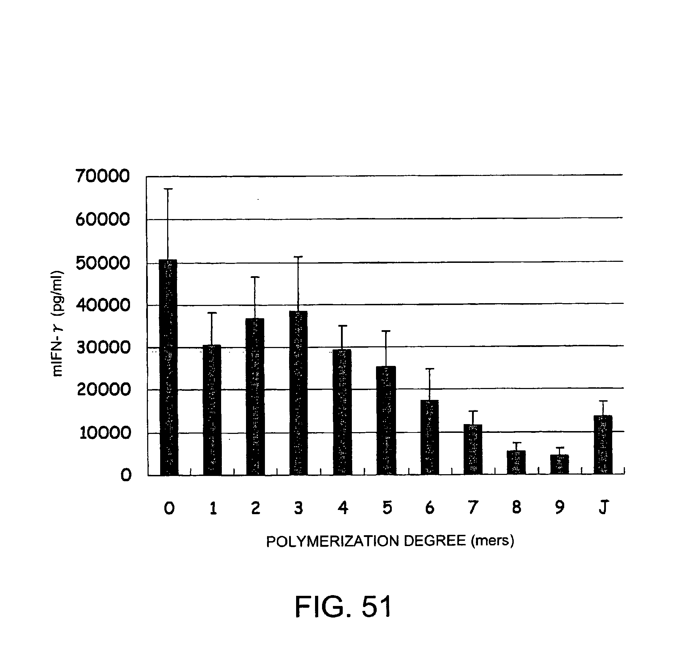

The result of IFN-γ measurement is shown in Fig. 51. Example 31 showed that procyanidins with a polymerization degree of 5 or more exhibited the activity. In this example, the amount of dose was reduced to compare the activity of up to procyanidin nonamers. The activity with pentamers was decreased because of the reduced dose; however, the activity was increased with an increase in the polymerization degree for pentameric or larger procyanidins, confirming a correlation between the activity and the polymerization degree for up to nonameric procyanidins.

### Example 33: Safety test for Jatoba extract (acute toxicity test in rats by single administration of Jatoba extract)

### 1. Experimental methods

(1) Samples for administration
   The Jatoba ethanol extract (Example 2) and Polyphenon (Mitsui Norin) as a green tea polyphenol (monomer) were dissolved in 10% ethanol at a concentration of 200 mg/ml. A 10% ethanol solution was used for a control group.
(2) Experimental animals
   Six-week-old male SD rats were used.
(3) Experimental conditions
   On day 0, compulsory oral administration was performed with 200 mg/ml of the Jatoba ethanol extract or 2 g/kg of green tea polyphenol. Up to day 6, body weight and feed intake were measured everyday at the same time.

### 2. Experimental results

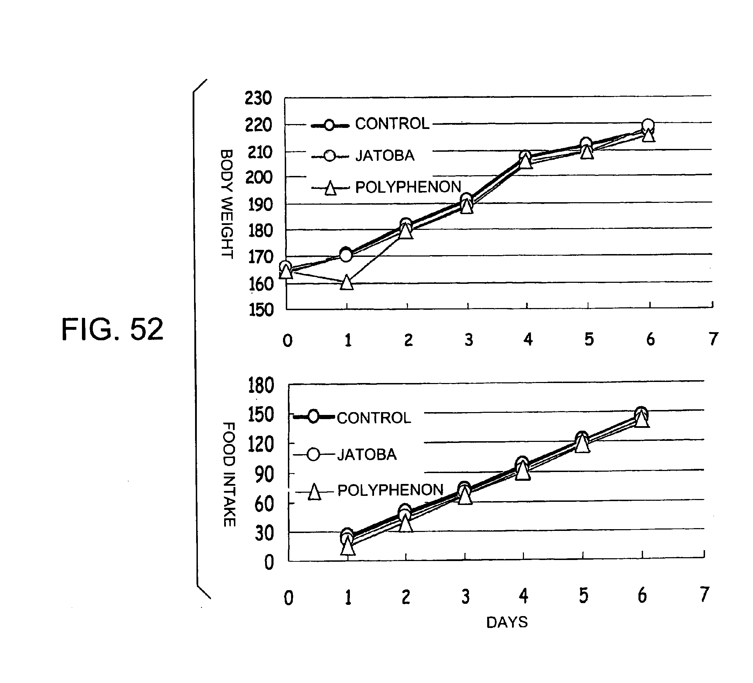

In the Jatoba administration group, no toxicity was observed and change in body weight (Fig. 52, top) and feed intake (Fig. 52, bottom) were comparative to those in the control group. On the other hand, in the green tea polyphenol administration group, a decrease in body weight by 10 g or more was noted on day 1 in 3 animals but it was recovered from the next day and no death was observed. These results revealed that no acute toxicity was recognized in the Jatoba extract.

## Claims

1. A therapeutic agent for the treatment of a disease in which the suppression of Th1 cytokine production is therapeutically effective and/or a disease in which the suppression of autoantibody production is therapeutically effective, a therapeutic agent for the eradicative treatment of these diseases, and an agent for inhibiting the progress of these diseases, comprising (i) a proanthocyanidin with a polymerization degree of at least 4 or (ii) a plant material extract containing a proanthocyanidin as an active ingredient.

2. The therapeutic agent and the inhibitory agent according to claim 1, wherein the polymerization degree of the proanthocyanidin in (i) and/or (ii) is at least 5.

3. The therapeutic agent and the inhibitory agent according to claim 1, wherein the polymerization degree of the proanthocyanidin in (i) and/or (ii) ranges from 4 to 30.

4. The therapeutic agent and the inhibitory agent according to claim 1, wherein the polymerization degree of the proanthocyanidin in (i) and/or (ii) ranges from 5 to 30.

5. The therapeutic agent and the inhibitory agent according to any one of claims 1 to 4, wherein the proanthocyanidin in (i) and/or (ii) at least includes constitutive units selected from catechin, epicatechin, epicatechin gallate, epigallocatechin gallate, and combinations thereof.

6. The therapeutic agent and the inhibitory agent according to any one of claims 1 to 5, wherein the proanthocyanidin in (i) and/or (ii) is selected from the group consisting of propelargonidin, procyanidin, prodelphinidin, proguibourtinidin, profisetinidin, prorobinetinidin, proteracacidin, promelacacidin, proapigeninidin, and proluteolinidin.

7. The therapeutic agent and the inhibitory agent according to any one of claims 1 to 6, wherein the proanthocyanidin in (i) is derived from Jatoba, grape seeds, cranberry, cinnamon, pine tree bark, or cacao.

8. The therapeutic agent and the inhibitory agent according to any one of claims 1 to 6, wherein the plant material extract in (ii) is selected from the group consisting of a Jatoba extract, a grape seed extract, a cranberry extract, a cinnamon extract, a pine tree bark extract, and a cacao extract.

9. The therapeutic agent and the inhibitory agent according to claim 1, wherein the proanthocyanidin in (i) and/or (ii) is represented by formula (I): 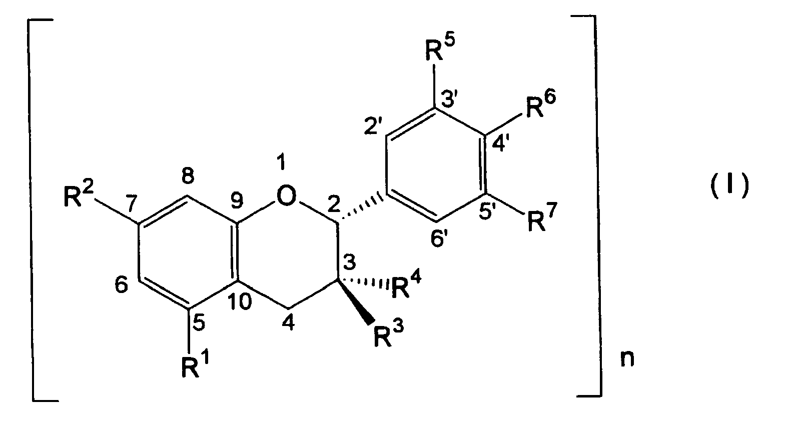 wherein R¹, R², R⁵, R⁶, and R⁷, which may be the same or different, represent a hydrogen atom, a hydroxyl group, or -O-R¹¹ (R¹¹ represents an alkyl group having 1 to 4 carbons or a sugar residue), and R³ and R⁴, which may be the same or different, represent a hydrogen atom, a hydroxyl group, or a group (II) : 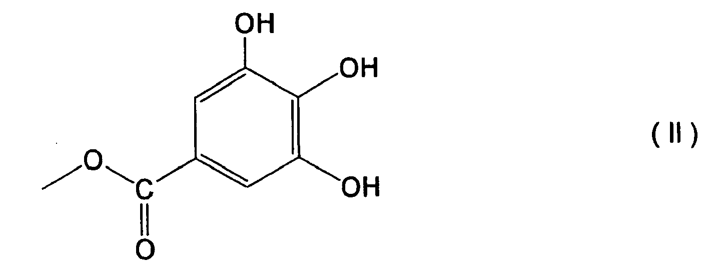 provided that R³ and R⁴ do not simultaneously represent a hydroxyl group; R³ and R⁴ do not simultaneously represent the group (II), n is an integer of 4 to 30; and individual constitutive units are linked each other on one site either between position 4 and position 6 or between position 4 and position 8 or on two sites between position 4 and position 8 and between position 2 and position 7.

10. The therapeutic agent and the inhibitory agent according to claim 9, wherein R¹ and R² represent a hydroxyl group or -O-R¹¹, R⁶ represents a hydroxyl group or -O-R¹¹, and R⁵ and R⁷ represent a hydrogen atom in the individual constitutive units.

11. The therapeutic agent and the inhibitory agent according to claim 10, wherein R¹ and R² represent a hydroxyl group, R⁶ represents a hydroxyl group, and R⁵ and R⁷ represent a hydrogen atom in the individual constitutive units.

12. The therapeutic agent and the inhibitory agent according to claim 10 or 11, wherein any one of R³ and R⁴ represents a hydroxyl group and the other represents a hydrogen atom in the individual constitutive units.

13. The therapeutic agent and the inhibitory agent according to claim 10 or 11, wherein any one of R³ and R⁴ represents group (II) and the other represents a hydrogen atom in the individual constitutive units.

14. The therapeutic agent and the inhibitory agent according to claim 9, wherein R¹ and R² represent a hydroxyl group or -O-R¹¹, R⁵ and R⁶ represent a hydroxyl group or -O-R¹¹, and R⁷ represents a hydrogen atomin the individual constitutive units.

15. The therapeutic agent and the inhibitory agent according to claim 14 , wherein R¹ and R² represent a hydroxyl group, R⁵ and R⁶ represent a hydroxyl group, and R⁷ represents a hydrogen atom in the individual constitutive units.

16. The therapeutic agent and the inhibitory agent according to claim 14 or 15, wherein any one of R³ and R⁹ represents a hydroxyl group and the other represents a hydrogen atom in the individual constitutive units.

17. The therapeutic agent and the inhibitory agent according to claim 14 or 15, wherein any one of R³ and R⁴ represents group (II) and the other represents a hydrogen atom in the individual constitutive units.

18. The therapeutic agent and the inhibitory agent according to claim 9, wherein R¹ and R² represent a hydroxyl group or -O-R¹¹ and R⁵, R⁶ and R⁷ represent a hydroxyl group or -O-R¹¹ in the individual constitutive units.

19. The therapeutic agent and the inhibitory agent according to claim 18, wherein R¹ and R² represent a hydroxyl group and R⁵, R⁶ and R⁷ represent a hydroxyl group in the individual constitutive units.

20. The therapeutic agent and the inhibitory agent according to claim 18 or 19, wherein any one of R³ and R⁴ represents a hydroxyl group and the other represents a hydrogen atom in the individual constitutive units.

21. The therapeutic agent and the inhibitory agent according to claim 18 or 19, wherein any one of R³ and R⁴ represents group (II) and the other represents a hydrogen atom in the individual constitutive units.

22. The therapeutic agent and the inhibitory agent according to any one of claims 9 to 21, wherein n is an integer of 5 to 30.

23. The therapeutic agent and the inhibitory agent according to any one of claims 9 to 22, wherein the individual constitutive units are linked each other between position 4 and position 6 or between position 4 and position 8.

24. The therapeutic agent and the inhibitory agent according to any one of claims 9 to 22, wherein the individual constitutive units are linked each other between position 4 and position 8 and between position 2 and position 7.

25. The therapeutic agent and the inhibitory agent according to any one of claims 1 to 24, wherein the disease in which the suppression of Th1 cytokine production is therapeutically effective is a Th1 cytokine-dependent autoimmune disease.

26. The therapeutic agent and the inhibitory agent according to claim 25, wherein the Th1 cytokine-dependent autoimmune disease is selected from multiple sclerosis, chronic rheumatoid arthritis, insulin-dependent diabetes mellitus, autoimmune thyroiditis (AT), autoimmune uveoretinitis (AUR), Hashimoto' s disease, psoriasis, Crohn' s disease, and alopecia areata.

27. The therapeutic agent and the inhibitory agent according to any one of claims 1 to 24, wherein the disease in which the suppression of autoantibody production is therapeutically effective is a autoantibody-mediated autoimmune disease.

28. The therapeutic agent and the inhibitory agent according to claim 27, wherein the autoantibody-mediated autoimmune disease is selected from systemic lupus erythematodes, Good Pasture' s syndrome, autoimmune hemolytic anemia, Sjogren's syndrome, acute rheumatic fever, pemphigus vulgaris, autoimmune thrombopenic purpura, and mixed type essential cryoglobulinemia.

29. The therapeutic agent and the inhibitory agent according to any one of claims 1 to 26, wherein the Th1 cytokine is IFN-γ.

30. The therapeutic agent and the inhibitory agent according to any one of claims 1 to 29, which is provided in the form of a medicine for oral administration or a medicine for external use.

31. Use of (i) a proanthocyanidin with a polymerization degree of at least 4 or (ii) a plant material extract containing a proanthocyanidin for the manufacture of a therapeutic agent for the treatment of a disease in which the suppression of Th1 cytokine production is therapeutically effective and/or a disease in which the suppression of autoantibody production is therapeutically effective, a therapeutic agent for the eradicative treatment of these diseases, and an agent for inhibiting the progress of these diseases.

32. The use according to claim 31, wherein the therapeutic agent for the treatment of a disease in which the suppression of Th1 cytokineproduction is therapeutically effective and/or a disease in which the suppression of autoantibody production is therapeutically effective, a therapeutic agent for the eradicative treatment of these diseases, and an agent for inhibiting the progress of these diseases are provided in the form of a food.

33. Amethod of treating a disease in which the suppression of Th1 cytokineproduction is therapeutically effective and/or a disease in which the suppression of autoantibody production is therapeutically effective, a method of fundamentally treating these diseases, and a method of inhibiting the progress of these diseases, which comprise administering to a mammal a therapeutically effective amount of (i) a proanthocyanidin with a polymerization degree of 4 or more or (ii) a plant material extract containing a proanthocyanidin together with a pharmaceutically acceptable carrier, if necessary.

34. An agent for suppressing Th1 cytokine production, comprising (i) a proanthocyanidin with a polymerization degree of at least 4 or (ii) a plant material extract containing a proanthocyanidin as an active ingredient.

35. Use of (i) a proanthocyanidin with a polymerization degree of at least 4 or (ii) a plant material extract containing a proanthocyanidin for the manufacture of an agent for suppressing Th1 cytokine production.

36. The use according to claim 35, wherein the agent for suppressing Th1 cytokine production is provided in the form of a food.

37. A method of suppressing Th1 cytokine production, comprising administering to a mammal an effective amount of (i) a proanthocyanidin wi th a polymerization degree of at least 4 or (ii) a plant material extract containing a proanthocyanidin.

38. An agent for suppressing autoantibody production, comprising (i) a pro anthocyanidin with a polymerization degree of at least 4 or (ii) a plant material extract containing a proanthocyanidin as an active ingredient.

39. Use of (i) a proanthocyanidin with a polymerization degree of at least 4 or (ii) aplantmaterial extract containing a proanthocyanidin for the manufacture of an agent for suppressing autoantibody production.

40. The use according to claim 39, wherein the agent for suppressing autoantibody production is provided in the form of a food.

41. A method of suppressing autoantibody production, comprising administering to a mammal an effective amount of (i) a proanthocyanidin with a polymerization degree of at least 4 or (ii) a plant material extract containing a proanthocyanidin.

42. A food comprising an effective amount of (i) a proanthocyanidin with a polymerization degree of at least 4 or (ii) aplantmaterialextractcontainingaproanthocyanidin, wherein said food is used for treating a disease in which the suppression of Th1 cytokine production is therapeutically effective and/or a disease in which the suppression of autoantibody production is therapeutically effective, fundamentally treating these diseases, and inhibiting the progress of these diseases.

43. A food comprising an effective amount of (i) a proanthocyanidin with a polymerization degree of at least 4 or (ii) aplantmaterialextractcontainingaproanthocyanidin, wherein said food has a function of suppressing Th1 cytokine production, a function of ameliorating or alleviating conditions associated with the enhancement of Th1 cytokine production, a function of suppressing autoantibody production, or a function of ameliorating or alleviating conditions associated with autoantibody production.

44. A food comprising (ii) a plant material extract containing a proanthocyanidin, wherein said extract has a function of suppressing Th1 cytokine production, a function of ameliorating or alleviating conditions associated with the enhancement of Th1 cytokine production, a function of suppressing autoantibody production, or a function of ameliorating or alleviating conditions associated with autoantibody production.

45. A food comprising an effective amount of (i) a proanthocyanidin with a polymerization degree of at least 4 or (ii) a plant material extract containing a proanthocyanidin, wherein said food has an indication for a function of suppressing Th1 cytokine production, a function of ameliorating or alleviating conditions associated with the enhancement of Th1 cytokine production, a function of suppressing autoantibody production, or a function of ameliorating or alleviating conditions associated with autoantibody production.

46. The food according to claim 45, wherein a function of suppressing Th1 cytokine production, a function of ameliorating or alleviating conditions associated with the enhancement of Th1 cytokine production, a function of suppressing autoantibody production, or a function of ameliorating or alleviating conditions associated with autoantibody production is indicated on food itself, a container, a package, an instruction, an attached document, or an advertisement.

47. The food according to any one of claims 42 to 46, wherein the polymerization degree of the proanthocyanidin in (i) and/or (ii) is at least 5.

48. The food according to any one of claims 42 to 46, wherein the polymerization degree of the proanthocyanidin in (i) and/or (ii) ranges from 4 to 30.

49. The food according to any one of claims 42 to 46, wherein the polymerization degree of the proanthocyanidin in (i) and/or (ii) ranges from 5 to 30.

50. The food according to any one of claims 42 to 49, wherein the proanthocyanidin in (i) and/or (ii) at least comprises constitutive units selected from catechin, epicatechin, epicatechin gallate, epigallocatechin gallate, and combinations thereof.

51. The food according to any one of claims 42 to 49, wherein the proanthocyanidin in (i) and/or (ii) is selected from the group consisting of propelargonidin, procyanidin, prodelphinidin, proguibourtinidin, profisetinidin, prorobinetinidin, proteracacidin, promelacacidin, proapigeninidin, and proluteolinidin.

52. The food according to any one of claims 42 to 51, wherein the proanthocyanidin in (i) is derived from Jatoba, grape seeds, cranberry, cinnamon, or pine tree bark.

53. The food according to any one of claims 42 to 46, wherein the plant material extract in (ii) is selected from the group consisting of a Jatoba extract, a grape seed extract, a cranberry extract, a cinnamon extract, a pine tree bark extract, and a cacao extract.

54. The food according to any one of claims 42 to 53, wherein the proanthocyanidin in (i) and/or (ii) is represented by formula (I): 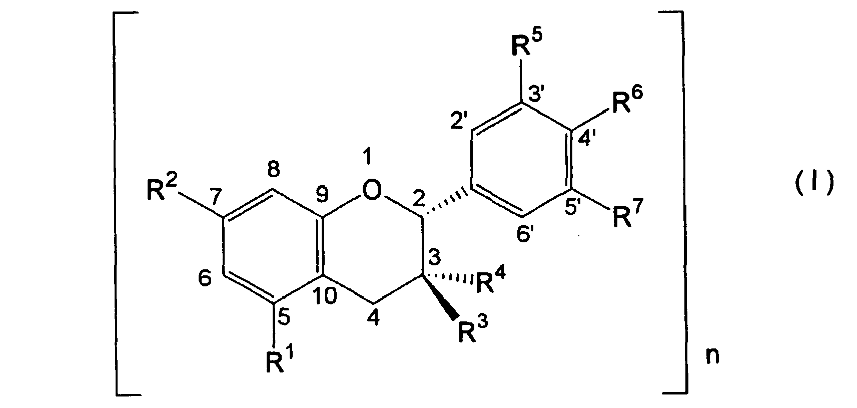 wherein R¹, R², R⁵, R⁶, and R⁷, which may be the same or different, represent a hydrogen atom, a hydroxyl group, or -O-R¹¹ (R¹¹ represents an alkyl group having 1 to 4 carbons or a sugar residue); and R³ and R⁴, which may be the same or different, represent a hydrogen atom, a hydroxyl group, or a group (II) : 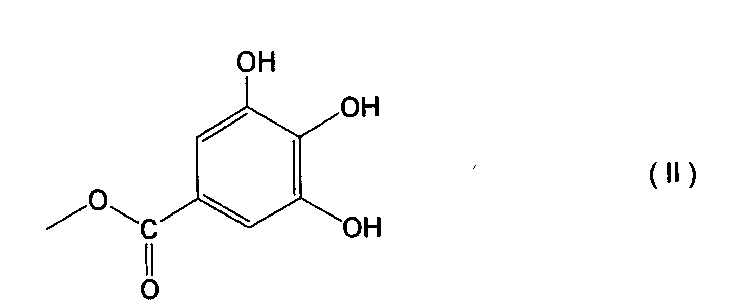 provided that R³ and R⁴ do not simultaneously represent a hydroxyl group; R³ and R⁴ do not simultaneously represent the group (II); n is an integer of 4 to 30; and individual constitutive units are linked each other on one site either between position 4 and position 6 or between position 4 and position 8 or on two sites between position 4 and position 8 and between position 2 and position 7.

55. The food according to claim 54, wherein R¹ and R² represent a hydroxyl group or -O-R¹¹, R⁶ represents a hydroxyl group or -O-R¹¹, and R⁵ and R⁷ represent a hydrogen atom in the individual constitutive units.

56. The food according to claim 55, wherein R¹ and R² represent a hydroxyl group, R⁶ represents a hydroxyl group, and R⁵ and R⁷ represent a hydrogen atom in the individual constitutive units.

57. The food according to claim 55 or 56, wherein any one of R³ and R⁴ represents a hydroxyl group and the other represents a hydrogen atom in the individual constitutive units.

58. The food according to claim 55 or 56, wherein any one of R³ and R⁴ represents group (II) and the other represents a hydrogen atom in the individual constitutive units.

59. The food according to claim 54, wherein R¹ and R² represent a hydroxyl group or -O-R¹¹, R⁵ and R⁶ represent a hydroxyl group or -O-R¹¹, and R⁷ represents a hydrogen atom in the individual constitutive units.

60. The food according to claim 59, wherein R¹ and R² represent a hydroxyl group, R⁵ and R⁶ represent a hydroxyl group, and R⁷ represents a hydrogen atom in the individual constitutive units.

61. The food according to claim 59 or 60, wherein any one of R³ and R⁴ represents a hydroxyl group and the other represents a hydrogen atom in the individual constitutive units.

62. The food according to claim 59 or 60, wherein any one of R³ and R⁴ represents group (II) and the other represents a hydrogen atom in the individual constitutive units.

63. The food according to claim 54, wherein R¹ and R² represent a hydroxyl group or -O-R¹¹ and R⁵, R⁶ and R⁷ represent a hydroxyl group or -O-R¹¹ in the individual constitutive units.

64. The food according to claim 63, wherein R¹ and R² represent a hydroxyl group and R⁵, R⁶ and R⁷ represent a hydroxyl group in the individual constitutive units.

65. The food according to claim 63 or 64, wherein any one of R³ and R⁴ represents a hydroxyl group and the other represents a hydrogen atom in the individual constitutive units.

66. The food according to claim 63 or 64, wherein any one of R³ and R⁴ represents group (II) and the other represents a hydrogen atom in the individual constitutive units.

67. The food according to any one of claims 54 to 66, wherein n is an integer of 5 to 30.

68. The food according to any one of claims 54 to 67, wherein the individual constitutive units are linked each other between position 4 and position 6 or between position 4 and position 8.

69. The food according to any one of claims 54 to 67, wherein the individual constitutive units are linked each other between position 4 and position 8 and between position 2 and position 7.

70. The food according to any one of claims 43 to 46, wherein the conditions associated with the enhancement of Th1 cytokine production are characteristic to a Th1 cytokine-dependent autoimmune disease.

71. The food according to claim 70, wherein the Th1 cytokine-dependent autoimmune disease is selected from multiple sclerosis, chronic rheumatoid arthritis, insulin-dependent diabetes mellitus, autoimmune thyroiditis (AT), autoimmune uveoretinitis (AUR), Hashimoto's disease, psoriasis, Crohn's disease, and alopecia areata.

72. The food according to any one of claims 43 to 46, wherein the conditions associated with autoantibody production are characteristic to a autoantibody-mediated autoimmune disease.

73. The food according to claim 72, wherein the autoantibody-mediated autoimmune disease is selected from systemic lupus erythematodes, Good Pasture's syndrome, autoimmune hemolytic anemia, Sjogren's syndrome, acute rheumatic fever, pemphigus vulgaris, autoimmune thrombopenic purpura, and mixed type essential cryoglobulinemia.

74. The food according to any one of claims 42 to 46, wherein the Th1 cytokine is IFN-γ.

75. The food according to any one of claims 43 to 46, wherein the conditions associated with the enhancement of Th1 cytokine production and/or the conditions associated with autoantibody production are systemic stiffness or strained feeling in joints.

76. A health food comprising a proanthocyanidin with a polymerization degree of at least 4 so that the amount of its daily ingestion for an adult ranges from 50 to 2000 mg.

77. The health food according to claim 76, wherein the proanthocyanidin is provided in the form of an extract which is selected from the group consisting of a Jatoba extract, a grape seed extract, a cranberry extract, a cinnamon extract, a pine bark extract, and a cacao extract and contains a proanthocyanidin with a polymerization degree of at least 4.

78. The health food according to claim 76 or 77, wherein the polymerization degree of the proanthocyanidin ranges from 4 to 30.

79. The health food according to claim 76 or 77, wherein the polymerization degree of the proanthocyanidin is at least 5.

80. The health food according to claim 76 or 77, wherein the polymerization degree of the proanthocyanidin ranges from 5 to 30.

81. The health food according to any one of claims 76 to 80, which is a food for specified health use, a nutritionally functional food, or a nutrient supplement food.

82. The health food according to any one of claims 76 to 81, wherein said food is in the form of an ordinary food or in the form of a nutrient supplement food.

83. A method of manufacturing a fraction having an activity for suppressing Th1 cytokine production, comprising the steps of
(a) subjecting a plant material containing a proanthocyanidin to an extraction process, and
(b) obtaining a cake by subjecting the extracted fraction obtained in step (a) to an ultrafiltration process.

84. The method according to claim 83, further comprising the step of (c) measuring the polymerization degree of the proanthocyanidin contained in the cake obtained in step (b), after step (b).

85. A method of testing an activity for suppressing Th1 cytokine production, comprising the step of measuring the polymerization degree of proanthocyanidins in a sample, **characterized in that** detection of proanthocyanidins with a polymerization degree of 4 or more indicates the activity for suppressing Th1 cytokine production.

86. The method according to claim 85, wherein the detection of proanthocyanidins with a polymerization degree of 5 or more is an index of the activity for suppressing Th1 cytokine production.

87. Amethod of testing an activity for treating a disease in which the suppression of Th1 cytokine production is therapeutically effective, comprising the step of measuring the polymerization degree of proanthocyanidins in a sample, **characterized in that** detection of proanthocyanidins with a polymerization degree of 4 or more indicates the therapeutic activity for treating the disease in which the suppression of Th1 cytokine production is therapeutically effective.

88. The method according to claim 87, wherein the detection of proanthocyanidins with a polymerization degree of 5 or more is an indication for the therapeutic activity for a disease in which the suppression of Th1 cytokine production is therapeutically effective.
